# EUROPEAN PATENT SPECIFICATION

(11) **EP 1 613 594 B1**
(45) Date of publication and mention of the grant of the patent: **26.03.2008**
(21) Application number: 04725299.4
(22) Date of filing: 02.04.2004
(51) Int. Cl.: C07D 211/70, C07D 405/12, A61K 31/4409, A61P 25/00

(54) **4-(2-PHENYLSULFANYL-PHENYL)-1,2,3,6-TETRAHYDROPYRIDINE DERIVATIVES AS SEROTONIN REUPTAKE INHIBITORS**
4-(2-PHENYLSULFANYL-PHENYL)-1,2,3,6-TETRAHYDROPYRIDINDERIVATEN ZUR VERWENDUNG ALS SEROTONIN-WIEDERAUFNAHMEHEMMER
DERIVES DE 4-(2-PHENYLSULFANYL-PHENYL)-1,2,3,6-TETRAHYDROPYRIDINE EN TANT QU'INHIBITEURS DE RECAPTAGE DE LA SEROTONINE

(30) Priority: 04.04.2003 DK 200300518; 04.04.2003 US 460266 P
(43) Date of publication of application: 11.01.2006
(73) Proprietor: H. LUNDBECK A/S, 2500 Kobenhavn-Valby (DK)
(72) Inventor: PÜSCHL, Ask, DK-2000 Frederiksberg (DK); BANG-ANDERSEN, Benny, Copenhagen S, 2300 (DK); JORGENSEN, Morten, Bagsvaerd, 2880 (DK); JUHL, Karsten, K benhavn S, 2300 (DK); RUHLAND, Thomas, DK-4000 Roskilde (DK); ANDERSEN, Kim, Ridgewood, New Jersey 07450 (US); KEHLER, Jan, Kgs. Lyngby, 2800 (DK)
(86) International application number: PCT/DK2004/000243
(87) International publication number: WO 2004/087662

(56) References cited:
- WO-A-01/49678
- WO-A-03/029232

## Description

The present invention relates to novel compounds which are serotonin reuptake inhibitors and as such effective in the treatment of for example depression and anxiety.

### Background of the invention

Selective serotonin reuptake inhibitors (hereinafter referred to as SSRIs) have become first choice therapeutics in the treatment of depression, certain forms of anxiety and social phobias, because they are effective, well tolerated and have a favourable safety profile compared to the classic tricyclic antidepressants.

However, clinical studies on depression indicate that non-response to SSRIs is substantial, up to 30%. Another, often neglected, factor in antidepressant treatment is compliance, which has a rather profound effect on the patient's motivation to continue pharmacotherapy.

First of all, there is the delay in therapeutic effect of SSRIs. Sometimes symptoms even worsen during the first weeks of treatment. Secondly, sexual dysfunction is a side effect common to all SSRIs. Without addressing these problems, real progress in the pharmacotherapy of depression and anxiety disorders is not likely to happen.

In order to cope with non-response, psychiatrists sometimes make use of augmentation strategies. Augmentation of antidepressant therapy may be accomplished through the co-administration of mood stabilizers such as lithium carbonate or triiodothyronin or by the use of electroshock.

The effect of combined administration of a compound that inhibits serotonin reuptake and a 5-HT_{1A} receptor antagonist has been evaluated in several studies (Innis et al. Eur. J. Pharmacol. 1987, 143, 1095-204 and Gartside Br. J. Pharmacol. 1995, 115, 1064-1070, Blier et al. Trends in Pharmacol. Science 1994, 15, 220). In these studies, it was found that 5-HT_{1A} receptor antagonists would abolish the initial brake on 5-HT neurotransmission induced by the serotonin reuptake inhibitors and thus produce an immediate boost of 5-HT transmission and a rapid onset of therapeutic action.

Several patent applications have been filed, which cover the use of a combination of a 5-HT_{1A} antagonist and a serotonin reuptake inhibitor for the treatment of depression (see e.g. EP-A2-687472 and EP-A2-714663).

WO 01/49678 describes compounds that potently bind to the 5-HT1A receptor, for the treatment of certain psychiatric and neurological disorders, and which can also have potent serotonin reuptake inhibitors and/or D3/D4 ligands, thus considered to be particularly useful for the treatment of depression and psychosis.

Another approach to increase terminal 5-HT would be through blockade of the 5-HT_{1B} autoreceptor. Microdialysis experiments in rats have indeed shown that increase of hippocampal 5-HT by citalopram is potentiated by GMC 2-29, an experimental 5-HT_{1B} receptor antagonist.

Several patent applications covering the combination of an SSRI and a 5-HT_{1B} antagonist or partial agonist have also been filed (WO 97/28141, WO 96/03400, EP-A-701819 and WO 99/13877).

It has previously been found that the combination of a serotonin reuptake inhibitor with a compound having 5-HT_{2C} antagonistic or inverse agonistic effect (compounds having a negative efficacy at the 5-HT_{2C} receptor) provides a considerable increase in the level of 5-HT in terminal areas, as measured in microdialysis experiments (WO 01/41701). This would imply a shorter onset of antidepressant effect in the clinic and an augmentation or potentiation of the therapeutic effect of the serotonin reuptake inhibitor (SRI).

The combined effect of serotonin reuptake inhibition and norepinephrine uptake inhibition on depression is explored in clinical studies of compounds such as Duloxetine (Wong, Duloxetine (LY-248686): an inhibitor of serotonin and noradrenaline uptake and an antidepressant drug candidate Expert Opinion on Investigational Drugs, 1998, 7, 10, 1691-1699) and Venlafaxine (Khan-A et al, Venlafaxine in depressed outpatients Psychopharmacology Bulletin, 1991, 27, 141-144).

The present invention provides compounds which are serotonin reuptake inhibitors. Some of the compounds also have a combined effect of serotonin reuptake inhibition and 5-HT_{2C} receptor modulation, which according to WO01/41701 would imply a faster onset of antidepressant activity. Moreover, some of the compounds posses the combined effect of serotonin reuptake inhibition and norepinephrine uptake inhibition. Basically, the present compounds are suitable for the treatment of affective disorders, such as depression, anxiety disorders including general anxiety disorder, social anxiety disorder, post traumatic stress disorder, obsessive compulsive disorder, panic disorder, panic attacks, specific phobias, social phobia and agoraphobia.

### Summary of the invention

The present invention provides compounds of the general formula I 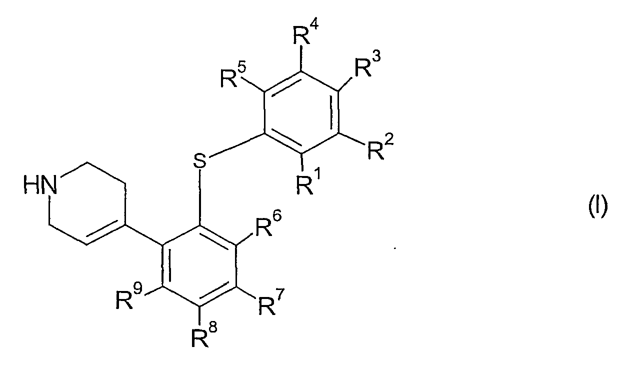 wherein R¹, R², R³, R⁴, R⁵, R⁶, R⁷, R⁸, and R⁹ are as defined below.

The invention provides a compound according to the above for use as a medicament.

The invention provides a pharmaceutical composition comprising a compound according to the above or a pharmaceutically acceptable acid addition salt thereof and at least one pharmaceutically acceptable carrier or diluent.

The invention provides the use of a compound according to the above or a pharmaceutically acceptable acid addition salt thereof for the preparation of a medicament for the treatment of affective disorders, such as depression, anxiety disorders including general anxiety disorder, social anxiety disorder, post traumatic stress disorder, obsessive compulsive disorder, panic disorder, panic attacks, specific phobias, social phobia and agoraphobia.

### Definition of substituents

Halogen means fluoro, chloro, bromo or iodo,

The expression C₁₋₆-alk(en/yn)yl means a C₁₋₆-alkyl, C₂₋₆-alkenyl or a C₂₋₆-alkynyl group.

The term C₁₋₆ alkyl refers to a branched or unbranched alkyl group having from one to six carbon atoms inclusive, including but not limited to methyl, ethyl, 1-propyl, 2-propyl,1-butyl, 2-butyl, 2-methyl-2-propyl and 2-methyl-1-propyl.

Similarly, C₂₋₆ alkenyl and C₂₋₆ alkynyl, respectively, designate such groups having from two to six carbon atoms, including one double bond and one triple bond respectively, including but not limited to ethenyl, propenyl, butenyl, ethynyl, propynyl and butynyl.

The terms C₁₋₆-alk(en/yn)yloxy, C₁₋₆ alk(en/yn)ylsulfanyl, hydroxy-C₁₋₆-alk(en/yn)yl, halo-C₁₋₆-alk(en/yn)yl, cyano-C₁₋₆-alk(en/yn)yl, NR^{z}R^{w}-C₁₋₆-alk(en/yn)yl, C₁₋₆-alk(en/yn)yloxy-C₁₋₆-alk(en/yn)yl and halo-C₁₋₆-alk(en/yn)yloxy designate such groups in which the C₁₋₆-alk(en/yn)yl are as defined above. Halo means halogen. NR^{z}R^{w}-C₁₋₆-alk(en/yn)yl designate the group

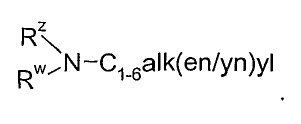

The term C₃₋₈ cycloalkyl designates a monocyclic or bicyclic carbocycle having three to eight C-atoms, including but not limited to cyclopropyl, cyclopentyl, cyclohexyl, etc.

The term C₃₋₈ cycloalkenyl designates a monocyclic or bicyclic carbocycle having three to eight C-atoms and including one double bond.

In the term C₃₋₈-cycloalk(en)yl-C₁₋₆-alk(en/yn)yl, C₃₋₈-cycloalk(en)yl and C₁₋₆-alk(en/yn)yl are as defined above.

The term 3-7-membered ring optionally containing one further heteroatom, such as N, O, or S, as used herein refers to ring systems such as 1-morpholinyl, 1-piperidinyl, 1-azepinyl, 1-piperazinyl, 1-homopiperazinyl, 1-imidazolyl, 1-pyrrolidinyl, 1-azetidinyl, 1-pyrrolyl or pyrazolyl, all of which may be further substituted with a group selected from a C₁₋₆-alk(en/yn)yl, hydroxy, hydroxy-C₁₋₆-alk(en/yn)yl, C₁₋₆-alk(en/yn)yloxy-C₁₋₆-alk(en/yn)yl.

### Description of the invention

The present invention relates to 4-(2-phenylsulfanyl-phenyl)-1,2,3,6-tetrahydropyridine derivatives which are serotonin reuptake inhibitors and as such effective in the treatment of for example depression and anxiety. Most of the tested compounds posses the combined effect of serotonin reuptake inhibition and norepinephrine uptake inhibition as measured in the tests described in the examples section.

Accordingly the present invention relates to a compound represented by the general formula I 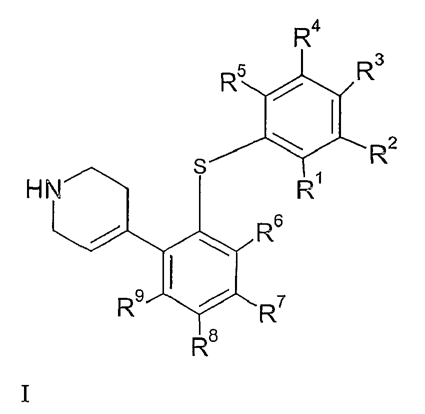 wherein
R¹, R², R³, R⁴, R⁵ are independently selected from hydrogen, halogen, cyano, C₁₋₆-alk(en/yn)yl, C₁₋₆-alk(en/yn)yloxy, C₁₋₆-alk(en/yn)ylsulfanyl, hydroxy, hydroxy-C₁₋₆-alk(en/yn)yl, halo-C₁₋₆-alk(en/yn)yl, halo-C₁₋₆-alk(en/yn)yloxy, or NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃₋₈-cycloalk(en)yl-C₁₋₆-alk(en/yn)yl, or NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl; or R^{x} and R^{y} together with the nitrogen to which they are attached form a 3-7-membered ring which optionally contains one further heteroatom; or
R² and R³ together form a heterocycle fused to the phenyl ring selected from 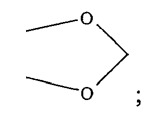 and R¹, R⁴, R⁵ are as defined above;
R⁶, R⁷, R⁸, R⁹ are independently selected from hydrogen, halogen, C₁₋₆-alk(en/yn)yl, C₁₋₆-alk(en/yn)yloxy, C₁₋₆-alk(en/yn)ylsulfanyl, hydroxy, hydroxy-C₁₋₆-alk(en/yn)yl, halo-C₁₋₆-alk(en/yn)yl, halo-C₁₋₆-alk(en/yn)yloxy, or NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃₋₈-cycloalk(en)yl-C₁₋₆-alk(en/yn)yl, or NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl; or R^{x} and R^{y} together with the nitrogen to which they are attached form a 3-7-membered ring which optionally contains one further heteroatom;
provided that at least one of R¹, R², R³, R⁴, R⁵, R⁶, R⁷, R⁸, and R⁹ is different from hydrogen; also provided that when R³ is methyl or methoxy, then at least one of R¹, R², R⁴, R⁵, R⁶, R⁷, R⁸, R⁹ is different from hydrogen;
or a salt thereof.

In one embodiment of the compound of formula I, R¹ is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl, or NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃₋₈-cycloalk(en)yl-C₁₋₆-alk(en/yn)yl, or NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl, provided that if one of R^{x} and R^{y} is NR^{z}R^{w}-C₁₋₆-alk(en/yn)yl then the other is selected from hydrogen, C₁₋₆-alk(en/yn)yl, cyano-C₁₋₆-alk(en/yn)yl, C₃₋₈-cycloalk(en)yl, or C₃₋₈-cycloalk(en)yl-C₁₋₆-alk(en/yn)yl; or R^{x} and R^{y} together with the nitrogen to which they are attached form a 3-7-membered ring which optionally contains one further heteroatom. In a further embodiment of the compound of formula I R¹ is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl. In a further embodiment R¹ is NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃₋₈-cycloalk(en)yl-C₁₋₆-alk(en/yn)yl, such as hydrogen, cyanomethyl, C₁₋₆-alk(en/yn)yl. In a further embodiment R¹ is NR^{x}R^{y} wherein R^{x} is NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl, and R^{y} is selected from hydrogen, C₁₋₆-alk(en/yn)yl, cyano-C₁₋₆-alk(en/yn)yl, C₃₋₈-cycloalk(en)yl, or C₃₋₈-cycloalk(en)yl-C₁₋₆-alk(en/yn)yl. In a further embodiment R¹ is NR^{x}R^{y} wherein R^{x} and R^{y} together with the nitrogen to which they are attached form a 3-7-membered ring which optionally contains one further heteroatom, such as 1-morpholinyl, 1-piperidinyl, 1-azepinyl, 1-piperazinyl, 1-homopiperazinyl, 1-imidazolyl, 1-pyrrolidinyl, 1-azetidinyl, 1-pyrrolyl or pyrazolyl, optionally substituted with one or more selected from a C₁₋₆-alk(en/yn)yl, hydroxy, hydroxy-C₁₋₆-alk(en/yn)yl, C₁₋₆-alk(en/yn)yloxy-C₁₋₆-alk(en/yn)yl, e.g. one or two selected from hydroxy, hydroxy-C₁₋₆-alkyl, C₁₋₆-alkyloxy-C₁₋₆-alkyl, C₁₋₆-alkyl, in particular one or two selected from hydroxy, methoxy-methyl, methyl. Typically, R¹ is selected from hydrogen; halogen; cyano; C₁₋₆-alkyl; C₁₋₆-alkyloxy; C₁₋₆-alkylsulfanyl; halo-C₁₋₆-alkyl; NR^{x}R^{y} wherein R^{x} and R^{y} are independently selected from hydrogen, C₁₋₆-alkyl, cyanomethyl; NR^{x}R^{y} wherein R^{y} is selected from hydrogen, or C₁₋₆-alkyl, and R^{x} is NR^{z}R^{w}-C₁₋₆-alk(en/yn)yl wherein R^{z} and R^{w} are independently selected from hydrogen, or C₁₋₆-alkyl; 1-morpholinyl, 1-piperidinyl, 1-azepinyl, 1-piperazinyl, 1-homopiperazinyl, 1-imidazolyl, 1-pyrrolidinyl, 1-azetidinyl, 1-pyrrolyl or pyrazolyl, optionally substituted with one or two selected from hydroxy, hydroxy-C₁₋₆-alkyl, C₁₋₆-alkyloxy-C₁₋₆-alkyl, C₁₋₆-alkyl, in particular one or two selected from hydroxy, methoxy-methyl, methyl. To further illustrate without limiting the invention an embodiment of R¹ is hydrogen; another embodiment of R¹ is C₁₋₆-alkyl, such as methyl, ethyl, tert-butyl; a further embodiment of R¹ is halogen, such as fluoro, bromo, or chloro; a further embodiment of R¹ is C₁₋₆-alkyloxy, such as methoxy; a further embodiment of R¹ is halo-C₁₋₆-alkyl, such as CF₃.

In a further embodiment of the compound of formula I, R² is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl. Typically, R² is selected from hydrogen, halogen, cyano, C₁₋₆-alkyl, C₁₋₆-alkyloxy, C₁₋₆-alkylsulfanyl, halo-C₁₋₆-alkyl. To further illustrate without limiting the invention an embodiment of R² is hydrogen; another embodiment of R² is C₁₋₆-alkoxy, such as methoxy; another embodiment of R² is halogen, such as fluoro, bromo, or chloro; another embodiment of R² is C₁₋₆-alkyl, such as methyl; another embodiment of R² is halo-C₁₋₆-alkyl, such as CF₃.

In a further embodiment of the compound of formula I, R³ is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl. Typically, R³ is selected from hydrogen, halogen, cyano, C₁₋₆-alkyl, C₁₋₆-alkyloxy, C₁₋₆-alkylsulfanyl, halo-C₁₋₆-alkyl. To further illustrate without limiting the invention an embodiment of R³ is hydrogen; another embodiment of R³ is C₁₋₆-alkyl, such as methyl, ethyl, tert-butyl; a further embodiment of R³ is C₁₋₆-alkoxy, such as methoxy; a further embodiment of R³ is halogen, such as chloro, bromo, or fluoro; a further embodiment of R³ is C₁₋₆-alkylsulfanyl, such as methylsulfanyl; a further embodiment of R³ is halo-C₁₋₆-alkyl, such as CF₃; a further embodiment of R³ is halo-C₁₋₆-alkyloxy, such as trifluoromethyloxy.

In a further embodiment of the compound of formula I, R² and R³ together form a heterocycle fused to the phenyl ring selected from

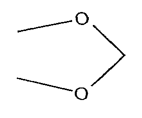

In a further embodiment of the compound of formula I, R⁴ is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl. Typically, R⁴ is selected from hydrogen, halogen, cyano, C₁₋₆-alkyl, C₁₋₆-alkyloxy, C₁₋₆-alkylsulfanyl, halo-C₁₋₆-alkyl. To further illustrate without limiting the invention an embodiment of R⁴ is hydrogen; another embodiment of R⁴ is C₁₋₆-alkoxy, such as methoxy; another embodiment of R⁴ is halogen, such as fluoro, bromo, or chloro; another embodiment of R⁴ is C₁₋₆-alkyl, such as methyl; another embodiment of R⁴ is halo-C₁₋₆-alkyl, such as CF₃.

In a further embodiment of the compound of formula I, R⁵ is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl, or NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃₋₈-cycloalk(en)yl-C₁₋₆-alk(en/yn)yl, or NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl, provided that if one of R^{x} and R^{y} is NR^{z}R^{w}-C₁₋₆-alk(en/yn)yl then the other is selected from hydrogen, C₁₋₆-alk(en/yn)yl, cyano-C₁₋₆-alk(en/yn)yl, C₃₋₈-cycloalk(en)yl, or C₃₋₈-cycloalk(en)yl-C₁₋₆-alk(en/yn)yl; or R^{x} and R^{y} together with the nitrogen to which they are attached form a 3-7-membered ring which optionally contains one further heteroatom. In a further embodiment of the compound of formula I R⁵ is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl.

In a further embodiment, R⁵ is NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃₋₈-cycloalk(en)yl-C₁₋₆-alk(en/yn)yl, such as hydrogen, cyanomethyl, C₁₋₆-alk(en/yn)yl. In a further embodiment R⁵ is NR^{x}R^{y} wherein R^{x} is NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl, and R^{y} is selected from hydrogen, C₁₋₆-alk(en/yn)yl, cyano-C₁₋₆-alk(en/yn)yl, C₃₋₈-cycloalk(en)yl, or C₃₋₈-cycloalk(en)yl-C₁₋₆-alk(en/yn)yl. In a further embodiment R⁵ is NR^{x}R^{y} wherein R^{x} and R^{y} together with the nitrogen to which they are attached form a 3-7-membered ring which optionally contains one further heteroatom, such as 1-morpholinyl, 1-piperidinyl, 1-azepinyl, 1-piperazinyl, 1-homopiperazinyl, 1-imidazolyl, 1-pyrrolidinyl, 1-azetidinyl, 1-pyrrolyl or pyrazolyl, optionally substituted with one or more selected from a C₁₋₆-alk(en/yn)yl, hydroxy, hydroxy-C₁₋₆-alk(en/yn)yl, C₁₋₆-alk(en/yn)yloxy-C₁₋₆-alk(en/yn)yl, e.g. one or two selected from hydroxy, hydroxy-C₁₋₆-alkyl, C₁₋₆-alkyloxy-C₁₋₆-alkyl, C₁₋₆-alkyl, in particular one or two selected from hydroxy, methoxy-methyl, methyl. Typically, R⁵ is selected from hydrogen; halogen; cyano; C₁₋₆-alkyl; C₁₋₆-alkyloxy; C₁₋₆-alkylsulfanyl; halo-C₁₋₆-alkyl; NR^{x}R^{y} wherein R^{x} and R^{y} are independently selected from hydrogen, C₁₋₆-alkyl, cyanomethyl; NR^{x}R^{y} wherein R^{y} is selected from hydrogen, or C₁₋₆-alkyl, and R^{x} is NR^{z}R^{w}-C₁₋₆-allc(en/yn)yl wherein R^{z} and R^{w} are independently selected from hydrogen, or C₁₋₆-alkyl; 1-morpholinyl, 1-piperidinyl, 1-azepinyl, 1-piperazinyl, 1-homopiperazinyl, 1-imidazolyl, 1-pyrrolidinyl, 1-azetidinyl, 1-pyrrolyl or pyrazolyl, optionally substituted with one or two selected from hydroxy, hydroxy-C₁₋₆-alkyl, C₁₋₆-alkyloxy-C₁₋₆-alkyl, C₁₋₆-alkyl, in particular one or two selected from hydroxy, methoxy-methyl, methyl. To further illustrate without limiting the invention an embodiment of R⁵ is hydrogen; another embodiment of R⁵ is C₁₋₆-alkyl, such as methyl, ethyl, tert-butyl; a further embodiment of R⁵ is halogen, such as fluoro, bromo, or chloro; a further embodiment of R⁵ is C₁₋₆-alkyloxy, such as methoxy; a further embodiment of R⁵ is halo-C₁₋₆-alkyl, such as CF₃.

In a further embodiment of the compound of formula I, R⁶ is selected from hydrogen, halogen, C₁₋₆-alk(en/yn)yl, halo-C₁₋₆-alk(en/yn)yl. Typically, R⁶ is selected from hydrogen, halogen, C₁₋₆-alkyl, halo-C₁₋₆-alkyl. To further illustrate without limiting the invention an embodiment of R⁶ is hydrogen; another embodiment of R⁶ is halogen, such as fluoro.

In a further embodiment of the compound of formula I, R⁷ is selected from hydrogen, halogen, C₁₋₆-alk(en/yn)yl, halo-C₁₋₆-alk(en/yn)yl. Typically, R⁷ is selected from hydrogen, halogen, C₁₋₆-alkyl, halo-C₁₋₆-alkyl. To further illustrate without limiting the invention an embodiment of R⁷ is hydrogen; another embodiment of R⁷ is halogen, such as fluoro.

In a further embodiment of the compound of formula I, R⁸ is selected from hydrogen, halogen, C₁₋₆-alk(en/yn)yl, halo-C₁₋₆-alk(en/yn)yl, or NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃₋₈-cycloalk(en)yl-C₁₋₆-alk(en/yn)yl, or NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl, provided that if one of R^{x} and R^{y} is NR^{z}R^{w}-C₁₋₆-alk(en/yn)yl then the other is selected from hydrogen, C₁₋₆-alk(en/yn)yl, cyano-C₁₋₆-alk(en/yn)yl, C₃₋₈-cycloalk(en)yl, or C₃₋₈-cycloalk(en)yl-C₁₋₆-alk(en/yn)yl; or R^{x} and R^{y} together with the nitrogen to which they are attached form a 3-7-membered ring which optionally contains one further heteroatom. In a further embodiment of the compound of formula I R⁸ is selected from hydrogen, halogen, C₁₋₆-alk(en/yn)yl, halo-C₁₋₆-alk(en/yn)yl, or NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃₋₈-cycloalk(en)yl-C₁₋₆-alk(en/yn)yl. In a further embodiment R⁸ is NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃-₈-cycloalk(en)yl-C₁₋₆-alk(en/yn)yl, such as hydrogen, cyanomethyl, C₁₋₆-alk(en/yn)yl. In a further embodiment R⁸ is NR^{x}R^{y} wherein R^{x} is NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl, and R^{y} is selected from hydrogen, C₁₋₆-alk(en/yn)yl, cyano-C₁₋₆-alk(en/yn)yl, C₃₋₈-cycloalk(en)yl, or C₃₋₈-cycloalk(en)yl-C₁₋₆-alk(en/yn)yl. In a further embodiment R⁸ is NR^{x}R^{y} wherein R^{x} and R^{y} together with the nitrogen to which they are attached form a 3-7-membered ring which optionally contains one further heteroatom, such as 1-morpholinyl, 1-piperidinyl, 1-azepinyl, 1-piperazinyl, 1-homopiperazinyl, 1-imidazolyl, 1-pyrrolidinyl, 1-azetidinyl, 1-pyrrolyl or pyrazolyl, optionally substituted with one or more selected from a C₁₋₆-alk(en/yn)yl, hydroxy, hydroxy-C₁₋₆-alk(en/yn)yl, C₁₋₆-alk(en/yn)yloxy-C₁₋₆-alk(en/yn)yl, e.g. one or two selected from hydroxy, hydroxy-C₁₋₆-alkyl, C₁₋₆-alkyloxy-C₁₋₆-alkyl, C₁₋₆-alkyl, in particular one or two selected from hydroxy, methoxy-methyl, methyl. Typically, R⁸ is selected from hydrogen; halogen; cyano; C₁₋₆-alkyl; C₁₋₆-alkyloxy; C₁₋₆-alkylsulfanyl; halo-C₁₋₆-alkyl; NR^{x}R^{y} wherein R^{x} and R^{y} are independently selected from hydrogen, C₁₋₆-alkyl, cyanomethyl; NR^{x}R^{y} wherein R^{y} is selected from hydrogen, or C₁₋₆-alkyl, and R^{x} is NR^{z}R^{w}-C₁₋₆-alk(en/yn)yl wherein R^{z} and R^{w} are independently selected from hydrogen, or C₁₋₆-alkyl; 1-morpholinyl, 1-piperidinyl, 1-azepinyl, 1-piperazinyl, 1-homopiperazinyl, 1-imidazolyl, 1-pyrrolidinyl, 1-azetidinyl, 1-pyrrolyl or pyrazolyl, optionally substituted with one or two selected from hydroxy, hydroxy-C₁₋₆-alkyl, C₁₋₆-alkyloxy-C₁₋₆-alkyl, C₁₋₆-alkyl, in particular one or two selected from hydroxy, methoxy-methyl, methyl. To further illustrate without limiting the invention an embodiment of R⁸ is hydrogen; another embodiment of R⁸ is halogen, such as fluoro, or bromo; a further embodiment of R⁸ is C₁₋₆-alkyl, such as methyl; a further embodiment of R⁸ is halo-C₁₋₆-alkyl, such as CF₃; another embodiment of R⁸ is NR^{x}R^{y} wherein R^{x} is hydrogen and R^{y} is C₁₋₆-alkyl, such as methyl.

In a further embodiment of the compound of formula I, R⁹ is selected from hydrogen, halogen, C₁₋₆-alk(en/yn)yl, halo-C₁₋₆-alk(en/yn)yl. Typically, R⁹ is selected from hydrogen, halogen, C₁₋₆-alkyl, halo-C₁₋₆-alkyl. To further illustrate without limiting the invention an embodiment of R⁹ is hydrogen.

Typically, the compound of formula I has at least one substituent in the phenyl ring(s), selected from any one of R¹-R⁹, which is different from hydrogen, such as 1, 2, 3, or 4 substituents in the phenyl ring(s), selected from any one of R¹-R⁹, which is/are different from hydrogen, and the remaining substituents are hydrogen. Thus, in a further embodiment 1 substituent selected from any one of R¹-R⁹, which is different from hydrogen, is present in either of the two phenyl rings, such as 1 substituent selected from R¹-R⁵, or the substituent is selected from R⁶-R⁹. In a further embodiment 2 substituents selected from R¹-R⁹, which are different from hydrogen, are present in either of the two phenyl rings, such as 1 substituent selected from R¹-R⁵, and the other selected from R⁶-R⁹, or both substituents are selected from R¹- R⁵; in this respect R² and R³ may be taken together to form the heterocycle as defined above. In a further embodiment 3 substituents selected from R¹-R⁹, which are different from hydrogen, are present in either of the two phenyl rings, such as 2 substituents selected from R¹-R⁵, and the last substituent is selected from R⁶-R⁹. In each embodiment, as mentioned the remaining substituents are hydrogen. To illustrate this further without limiting the invention, some typical embodiments are outlined hereafter.

Thus, in a further embodiment of the compound of formula I one substituent is present which is R² as defined above, except hydrogen. In a further embodiment of the compound of formula I one substituent is present which is R³ as defined above, except hydrogen. In a further embodiment of the compound of formula I two substituents are present being R³ and R⁸, wherein R³ and R⁸ are as defined above, except hydrogen. In a further embodiment of the compound of formula I two substituents are present being R³ and R⁶, wherein R³ and R⁶ are as defined above, except hydrogen. In a further embodiment of the compound of formula I two substituents are present being R³ and R⁷, wherein R³ and R⁷ are as defined above, except hydrogen. In a further embodiment of the compound of formula I two substituents are present being R¹ and R³, wherein R¹ and R³ are as defined above, except hydrogen. In a further embodiment of the compound of formula I two substituents are present being R² and R³, wherein R² and R³ are as defined above, except hydrogen, in this respect R² and R³ may be taken together to form the heterocycle as defined above. In a further embodiment of the compound of formula I three substituents are present being R¹, R³ and R⁸, wherein R¹, R³ and R⁸ are as defined above, except hydrogen. In each embodiment, as mentioned above the remaining substituents are hydrogen.

In a further embodiment of the compound of formula I, said compound is selected from
4-[2-(4-Fluorophenylsulfanyl)-phenyl]-1,2,3,6-tetrahydropyridine,
4-[2-(4-Chlorophenylsulfanyl)-phenyl]-1,2,3,6-tetrahydropyridine,
4-[2-(4-Methoxyphenylsulfanyl)-5-methyl-phenyl]-1,2,3,6-tetrahydropyridine,
4-(5-Methyl-2-p-tolylsulfanylphenyl)-1,2,3,6-tetrahydropyridine,
4-[2-(4-Fluorophenylsulfanyl)-5-methyl-phenyl]-1,2,3,6-tetrahydropyridine,
4-[2-(4-Chlorophenylsulfanyl)-5-methyl-phenyl]-1,2,3,6-tetrahydropyridine,
4-[2-(2,4-Dimethylphenylsulfanyl)-phenyl]-1,2,3,6-tetrahydropyridine,
4-[2-(4-Chlorophenylsulfanyl)-5 -trifluoromethyl-phenyl]-1,2,3,6-tetrahydropyridine,
4-[2-(4-Fluoro-2-methylphenylsulfanyl)-phenyl]-1,2,3,6-tetrahydropyridine,
4-[2-(4-Chlorophenylsulfanyl)-4-fluoro-phenyl]-1,2,3,6-tetrahydropyridine,
4-[4-Fluoro-2-(p-tolylsulfanyl)-phenyl]-1,2,3,6-tetrahydropyridine,
4-[4-Fluoro-2-(4-methoxyphenylsulfanyl)-phenyl]-1,2,3,6-tetrahydropyridine,
4-[2-(2,4-Dimethylphenylsulfanyl)-5-methyl-phenyl]-1,2,3,6-tetrahydropyridine,
4-[2-(2,4-Dimethylphenylsulfanyl)-5-trifluoromethyl-phenyl]-1,2,3,6-tetrahydropyridine,
4-(2-p-Tolylsulfanyl-5-trifluoromethylphenyl)-1,2,3,6-tetrahydropyridine,
4-[2-(4-Fluoro-2-methylphenylsulfanyl)-5-methyl-phenyl]-1,2, 3,6-tetrahydropyridine,
4-[5-Bromo-2-(2,4-dimethylphenylsulfanyl)-phenyl]-1,2,3,6-tetrahydropyridine,
4-[2-(4-Clilorophenylsulfaiiyl)-5-fluoro-phenyl]-1,2,3,6-tetrahydropyridine,
4-[5-Fluoro-2-(4-methoxyphenylsulfanyl)-phenyl]-1,2,3,6-tetrahydropyridine,
4-[2-(2,4-Dichlorophenylsulfanyl)-phenyl]-1,2,3,6-tetrahydropyridine,
4-[2-(4-Chloro-2-fluoro-phenylsulfanyl)-phenyl]-1,2,3,6-tetrahydropyridine,
4-[2-(3-Methoxyphenylsulfanyl)-phenyl]-1,2,3,6-tetrahydropyridine,
4-[3-Fluoro-2-(4-methoxyphenylsulfanyl)-phenyl]-1,2,3,6-tetrahydropyridine,
4-[2-(2-Chlorophenylsulfanyl)-phenyl]-1,2,3,6-tetrahydropyridine,
4-[2-(2-Chloro-4-methoxyphenylsulfanyl)-phenyl]-1,2,3,6-tetrahydropyridine,
4-[2-(2-Fluorophenylsulfanyl)-phenyl]-1,2,3,6-tetrahydropyridine,
4-[2-(2-Bromophenylsulfanyl)-phenyl]-1,2,3,6-tetrahydropyridine,
4-[2-(4-Bromophenylsulfanyl)-phenyl]-1,2,3,6-tetrahydropyridine,
4-(2-o-Tolylsulfanylphenyl)-1,2,3,6-tetrahydropyridine,
4-[2-(4-Chloro-2-methylphenylsulfanyl)-phenyl]-1,2,3,6-tetrahydropyridine,
4-[2-(4-Trifluoromethyl-phenylsulfanyl)-phenyl]-1,2,3,6-tetrahydro-pyridine,
4-[2-(2,3-Dichloro-phenylsulfanyl)-phenyl]-1,2,3,6-tetrahydro-pyridine,
4-[2-(2,3-Dimethyl-phenylsulfanyl)-phenyl]-1,2,3,6-tetrahydro-pyridine,
4-[2-(3,4-Dimethyl-phenylsulfanyl)-phenyl]-1,2,3,6-tetrahydro-pyridine,
4-[2-(2-Methoxy-5-methyl-phenylsulfanyl)-phenyl]-1,2,3,6-tetrahydro-pyridine,
4-[2-(2-Chloro-4-fluoro-phenylsulfanyl)-phenyl]-1,2,3,6-tetrahydro-pyridine,
4-[2-(4-Methoxy-2-methyl-phenylsulfanyl)-phenyl]-1,2,3,6-tetrahydro-pyridine,
4-[2-(2-Fluoro-4-methoxy-phenylsulfanyl)-phenyl]-1,2,3,6-tetrahydro-pyridine,
4-[2-(2,4-Dimethyl-phenylsulfanyl)-5-fluoro-phenyl]-1,2,3,6-tetrahydro-pyridine,
4-(5-Fluoro-2-phenylsulfanyl-phenyl)-1,2,3,6-tetrahydro-pyridine,
4-[5-Fluoro-2-(4-fluoro-phenylsulfanyl)-phenyl]-1,2,3,6-tetrahydro-pyridine,
4-(2-m-Tolylsulfanyl-phenyl)-1,2,3,6-tetrahydro-pyridine,
4-[2-(4-Fluoro-3-methoxy-phenylsulfanyl)-phenyl]-1,2,3,6-tetrahydro-pyridine,
4-[2-(2-Bromo-4-fluoro-phenylsulfanyl)-phenyl]-1,2,3,6-tetrahydro-pyridine,
4-[2-(2-Chloro-4-fluoro-phenylsulfanyl)-5-fluoro-phenyl]-1,2,3,6-tetrahydro-pyridine,
4-[2-(2,4-Dichloro-phenylsulfanyl)-5-fluoro-phenyl]-1,2,3,6-tetrahydro-pyridine,
4-[2-(3-Chloro-phenylsulfanyl)-phenyl]-1,2,3,6-tetrahydro-pyridine,
4-[2-(2-Methoxy-phenylsulfanyl)-phenyl]-1,2,3,6-tetrahydro-pyridine,
4-[2-(2,4-Dichloro-phenylsulfanyl)-5-methyl-phenyl]-1,2,3,6-tetrahydro-pyridine,
4-[2-(4-Chloro-2-fluoro-phenylsulfanyl)-5-methyl-phenyl]-1,2,3,6-tetrahydro-pyridine,
4-[2-(4-Chloro-2-methyl-phenylsulfanyl)-5-methyl-phenyl]-1,2,3,6-tetrahydro-pyridine,
4-[2-(2-Fluoro-4-methyl-phenylsulfanyl)-5-methyl-phenyl]-1,2,3,6-tetrahydro-pyridine,
4-[2-(2,4-Difluoro-phenylsulfanyl)-5-methyl-phenyl]-1,2,3,6-tetrahydro-pyridine,
4-[2-(2-Chloro-4-fluoro-phenylsulfanyl)-5-methyl-phenyl]-1,2,3,6-tetrahydro-pyridine,
4-[2-(2-Bromo-4-fluoro-phenylsulfanyl)-5-methyl-phenyl]-1,2,3,6-tetrahydro-pyridine,
4-[2-(2-Bromo-4-methyl-phenylsulfanyl)-5-methyl-phenyl]-1,2,3,6-tetrahydro-pyridine,
4-[2-(2-Bromo-phenylsulfanyl)-5-methyl-phenyl]-1,2,3,6-tetrahydro-pyridine,
4-[2-(4-Bromo-phenylsulfanyl)-5-methyl-phenyl]-1,2,3,6-tetrahydro-pyridine,
4-(5-Methyl-2-o-tolylsulfanyl-phenyl)-1,2,3,6-tetrahydro-pyridine,
4-[2-(4-Methoxy-2-methyl-phenylsulfanyl)-5-methyl-phenyl]-1,2,3,6-tetrahydro-pyridine,
4-[2-(2-Fluoro-4-methoxy-phenylsulfanyl)-5-methyl-phenyl]-1,2,3,6-tetrahydro-pyridine,
4-[2-(3,4-Dichloro-phenylsulfanyl)-5-methyl-phenyl]-1,2,3,6-tetrahydro-pyridine,
4-[2-(2-Chloro-4-methyl-phenylsulfanyl)-5-methyl-phenyl]-1,2,3,6-tetrahydro-pyridine,
4-[2-(2-Fluoro-4-methyl-phenylsulfanyl)-phenyl]-1,2,3,6-tetrahydro-pyridine,
4-[2-(3,4-Dichloro-phenylsulfanyl)-phenyl]-1,2,3,6-tetrahydro-pyridine,
4-[2-(4-Bromo-2-fluoro-phenylsulfanyl)-phenyl]-1,2,3,6-tetrahydro-pyridine,
4-[2-(4-Bromo-2-methyl-phenylsulfanyl)-phenyl]-1,2,3,6-tetrahydro-pyridine,
4-[2-(2,4-Difluoro-phenylsulfanyl)-phenyl]-1,2,3,6-tetrahydro-pyridine,
4-[2-(2-Bromo-4-methyl-phenylsulfanyl)-phenyl]-1,2,3,6-tetrahydro-pyridine,
4-[2-(4-Chloro-2-methoxy-phenylsulfanyl)-phenyl]-1,2,3,6-tetrahydro-pyridine,
4-[5-Fluoro-2-(2-fluoro-4-methoxy-phenylsulfanyl)-phenyl]-1,2,3,6-tetrahydro-pyridine,
4-[2-(2-Chloro-4-methoxy-phenylsulfanyl)-5-fluoro-phenyl]-1,2,3,6-tetrahydro-pyridine,
4-[5-Fluoro-2-(2-fluoro-4-methyl-phenylsulfanyl)-phenyl]-1,2,3,6-tetrahydro-pyridine,
4-[2-(2-Chloro-4-methyl-phenylsulfanyl)-5-fluoro-phenyl]-1,2,3,6-tetrahydro-pyridine,
4-[2-(4-Chloro-2-fluoro-phenylsulfanyl)-5-fluoro-phenyl]-1,2,3,6-tetrahydro-pyridine,
4-[5-Fluoro-2-(4-methoxy-2-methyl-phenylsulfanyl)-phenyl]-1,2,3,6-tetrahydro-pyridine,
4-[2-(2,3-Dimethyl-phenylsulfanyl)-5-methyl-phenyl]-1,2,3,6-tetrahydro-pyridine,
4-[5-Methyl-2-(3-methyl-phenylsulfanyl)-phenyl]-1,2,3,6-tetrahydro-pyridine,
4-[2-(2-Fluoro-phenylsulfanyl)-5-methyl-phenyl]-1,2,3,6-tetrahydro-pyridine,
4-[2-(2-Chloro-phenylsulfanyl)-5-methyl-phenyl]-1,2,3,6-tetrahydro-pyridine,
4-[2-(3,4-Dimethyl-phenylsulfanyl)-5-methyl-phenyl]-1,2,3,6-tetrahydro-pyridine,
4-[2-(4-Chloro-2-methoxy-phenylsulfanyl)-5-methyl-phenyl]-1,2,3,6-tetrahdro-pyridine,
4-[2-(2,3-Difluoro-phenylsulfanyl)-phenyl]-1,2,3,6-tetrahydro-pyridine,
4-[2-(2-Chloro-4-methyl-phenylsulfanyl)-phenyl]-1,2,3,6-tetrahydro-pyridine,
4-[2-(2,3-Difluoro-phenylsulfanyl)-5-methyl-phenyl]-1,2,3,6-tetrahydro-pyridine,
4-[2-(3-Chloro-2-fluoro-phenylsulfanyl)-5-methyl-phenyl]-1,2,3,6-tetrahydro-pyridine,
4-[2-(3-Fluoro-2-methyl-phenylsulfanyl)-phenyl]-1,2,3,6-tetrahydro-pyridine,
4-[2-(3-Chloro-2-methyl-phenylsulfanyl)-phenyl]-1,2,3,6-tetrahydro-pyridine,
4-[2-(3-Fluoro-2-methyl-phenylsulfanyl)-5-methyl-phenyl]-1,2,3,6-tetrahydro-pyridine,
4-[2-(3-Fluoro-phenylsulianyl)-5-methyl-phenyl]-1,2,3,6-tetrahydro-pyridine,
4-[2-(3-Chloro-phenylsulfanyl)-5-methyl-phenyl]-1,2,3,6-tetrahydro-pyridine,
4-[2-(3-Bromo-phenylsulfanyl)-5-methyl-phenyl]-1,2,3,6-tetrahydro-pyridine,
4-[2-(3-Methoxy-phenylsulfanyl)-5-methyl-phenyl]-1,2,3,6-tetrahydro-pyridine,
4-[5-Methyl-2-(3-trifluoromethyl-phenylsulfanyl)-phenyl]-1,2,3,6-tetrahydro-pyridine,
4-[2-(2-Methoxy-phenylsulfanyl)-5-methyl-phenyl]-1,2,3,6-tetrahydro-pyridine,
4-[2-(2-Ethyl-phenylsulfanyl)-phenyl]-1,2,3,6-tetrahydro-pyridine,
4-[2-(4-Ethyl-phenylsulfanyl)-phenyl]-1,2,3,6-tetrahydro-pyridine,
4-[2-(2-tert-Butyl-phenylsulfanyl)-phenyl]-1,2,3,6-tetrahydro-pyridine,
4-[2-(4-tert-Butyl-phenylsulfanyl)-phenyl]-1,2,3,6-tetrahydro-pyridine,
4-[2-(3 -Fluoro-phenylsulfanyl)-phenyl]-1,2, 3, 6-tetrahydro-pyridine,
4-[2-(2-Trifluoromethyl-phenylsulfanyl)-phenyl]-1,2,3,6-tetrahydro-pyridine,
4-[2-(3-Trifluoromethyl-phenylsulfanyl)-phenyl]-1,2,3,6-tetrahydro-pyridine,
4-[2-(4-Trifluoromethoxy-phenylsulfanyl)-phenyl]-1,2,3,6-tetrahydro-pyridine,
4-[2-(4-Methylsulfanyl-phenylsulfanyl)-phenyl]-1,2,3,6-tetrahydro-pyridine,
4-[2-(3,5-Dimethyl-phenylsulfanyl)-phenyl]-1,2,3,6-tetrahydro-pyridine,
4-[2-(2,5-Dimethyl-phenylsulfanyl)-phenyl]-1,2,3,6-tetrahydro-pyridine,
4-[2-(2,5-Dichloro-phenylsulfanyl)-phenyl]-1,2,3,6-tetrahydro-pyridine,
4-[2-(3,5-Dichloro-phenylsulfanyl)-phenyl]-1,2,3,6-tetrahydro-pyridine,
4-[2-(3-Chloro-4-fluoro-phenylsulfanyl)-phenyl]-1,2,3,6-tetrahydro-pyridine,
4-[2-(2,4,6-Trimethyl-phenylsulfanyl)-phenyl]-1,2,3,6-tetrahydro-pyridine,
4-[5-Methylamino-2-(4-methyl-pheylsulfanyl)-phenyl]-1,2,3,6-tetrahydro-pyridine,
4-[5-Fluoro-2-(2-methoxy-phenylsulfanyl)-phenyl]-1,2,3,6-tetrahydro-pyridine,
4-(5-Fluoro-2-o-tolylsulfanyl-phenyl)-1,2,3,6-tetrahydro-pyridine,
4-(5-Fluoro-2-p-tolylsulfanyl-phenyl)-1,2,3,6-tetrahydro-pyridine,
4-[2-(Benzo[1,3]dioxol-5-ylsulfanyl)-5-methyl-phenyl]-1,2,3,6-tetrahydro-pyridine,
4-[2-(2-Chloro-4-methoxy-phenylsulfanyl)-5-methyl-phenyl]-1,2,3,6-tetrahydro-pyridine,
4-[2-(2,3-Dichloro-phenylsulfanyl)-5-methyl-phenyl]-1,2,3,6-tetrahydro-pyridine,
4-[2-(3-Chloro-2-methyl-phenylsulfanyl)-5-methyl-phenyl]-1,2,3,6-tetrahydro-pyridine,
4-[2-(Benzo[1,3]dioxol-S-ylsulfanyl)-phenyl]-1,2,3,6-tetrahydro-pyridine,
4-[5-Fluoro-2-(3-fluoro-phenylsulfanyl)-phenyl]-1,2,3,6-tetrahydro-pyridine,
4-(5-Fluoro-2-m-tolylsulfanyl-phenyl)-1,2,3,6-tetrahydro-pyridine,
4-[5-Fluoro-2-(3-methoxy-phenylsulfanyl)-phenyl]-1,2,3,6-tetrahydro-pyridine,
4-[2-(3-Chloro-phenylsulfanyl)-5-fluoro-phenyl]-1,2,3,6-tetrahydro-pyridine,
4-[2-(2-Chloro-phenylsulfanyl)-5-fluoro-phenyl]-1,2,3,6-tetrahydro-pyridine,
4-[4-Fluoro-2-(4-methyl-pheylsulfanyl)-phenyl]-1,2, 3, 6-tetrahydro-pyridine,
4-[2-(4-Bromo-2-fluoro-phenylsulfanyl)-5-fluoro-phenyl]-1,2,3,6-tetrahydro-pyridine,
4-[2-(4-Fluoro-3-methoxy-phenylsulfanyl)-5-methyl-phenyl]-1,2,3,6-tetrahydro-pyridine,
4-[2-(3-Fluoro-4-methyl-phenylsulfanyl)-5-methyl-phenyl]-1,2,3,6-tetrahydro-pyridine,
4-[5-Fluoro-2-(4-methoxy-phenylsulfanyl)-phenyl]-1,2,3,6-tetrahydro-pyridine,
or a pharmaceutically acceptable salt thereof. Each of these compounds is considered a specific embodiment and may be subject to individual claims.

As mentioned above, most of the tested compounds posses the combined effect of serotonin reuptake inhibition and norepinephrine uptake inhibition, however, a few compounds selected from
4-[2-(2,3-Dimethyl-phenylsulfanyl)-phenyl]-1,2,3,6-tetrahydro-pyridine,
4-[2-(4-Methoxy-2-methyl-phenylsulfanyl)-phenyl]-1,2,3,6-tetrahydro-pyridine,
4-[2-(2-Fluoro-4-methyl-phenylsulfanyl)-5-methyl-phenyl]-1,2,3,6-tetrahydro-pyridine,
4-[2-(2-Fluoro-4-methoxy-phenylsulfanyl)-5-methyl-phenyl]-1,2,3,6-tetrahydro-pyridine,
4-[2-(2,3-Difluoro-phenylsulfanyl)-phenyl]-1,2,3,6-tetrahydro-pyridine,
4-[2-(2-Chloro-4-methyl-phenylsulfanyl)-phenyl]-1,2,3,6-tetrahydro-pyridine,
4-[2-(3-Chloro-2-fluoro-phenylsulfanyl)-5-methyl-phenyl]-1,2,3,6-tetrahydro-pyridine,
4-[2-(3-Fluoro-2-methyl-phenylsulfanyl)-phenyl]-1,2,3,6-tetrahydro-pyridine, 4-[2-(3-Fluoro-2-methyl-phenylsulfanyl)-5-methyl-phenyl]-1,2,3,6-tetrahydro-pyridine, or
4-[2-(3-Fluoro-4-methyl-phenylsulfanyl)-5-methyl-phenyl]-1,2,3,6-tetrahydro-pyridine,
did show serotonin reuptake inhibition, but did not show norepinephrine uptake inhibition in the test herein.

The present invention also comprises salts of the present compounds, typically, pharmaceutically acceptable salts. Such salts include pharmaceutical acceptable acid addition salts, pharmaceutically acceptable metal salts, ammonium and alkylated ammonium salts. Acid addition salts include salts of inorganic acids as well as organic acids.

Representative examples of suitable inorganic acids include hydrochloric, hydrobromic, hydroiodic, phosphoric, sulfuric, sulfamic, nitric acids and the like. Representative examples of suitable organic acids include formic, acetic, trichloroacetic, trifluoroacetic, propionic, benzoic, cinnamic, citric, fumaric, glycolic, itaconic, lactic, methanesulfonic, maleic, malic, malonic, mandelic, oxalic, picric, pyruvic, salicylic, succinic, methane sulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, theophylline acetic acids, as well as the 8-halotheophyllines, for example 8-bromotheophylline and the like.

Examples of metal salts include lithium, sodium, potassium, magnesium salts and the like.

Examples of ammonium and alkylated ammonium salts include ammonium, methyl-, dimethyl-, trimethyl-, ethyl-, hydroxyethyl-, diethyl-, n-butyl-, sec-butyl-, tert-butyl-, tetramethylammonium salts and the like.

Further, the compounds of this invention may exist in unsolvated as well as in solvated forms with pharmaceutically acceptable solvents such as water, ethanol and the like. In general, the solvated forms are considered equivalent to the unsolvated forms for the purposes of this invention.

The compounds of the present invention may have one or more asymmetric centres and it is intended that any optical isomers (i.e. enantiomers or diastereomers), as separated, pure or partially purified optical isomers and any mixtures thereof including racemic mixtures are included within the scope of the invention.

Racemic forms can be resolved into the optical antipodes by known methods, for example, by separation of diastereomeric salts thereof with an optically active acid, and liberating the optically active amine compound by treatment with a base. Another method for resolving racemates into the optical antipodes is based upon chromatography on an optically active matrix. Racemic compounds of the present invention can also be resolved into their optical antipodes, e.g. by fractional crystallization of d- or 1- (tartrates, mandelates or camphorsulphonate) salts. The compounds of the present invention may also be resolved by the formation of diastereomeric derivatives.

Additional methods for the resolution of optical isomers, known to those skilled in the art, may be used. Such methods include those discussed by J. Jaques, A. Collet and S. Wilen in "Enantiomers, Racemates, and Resolutions", John Wiley and Sons, New York (1981).

Optically active compounds can also be prepared from optically active starting materials, or by stereoselective synthesis.

Furthermore, when a double bond or a fully or partially saturated ring system is present in the molecule geometric isomers may be formed. It is intended that any geometric isomers, as separated, pure or partially purified geometric isomers or mixtures thereof are included within the scope of the invention. Likewise, molecules having a bond with restricted rotation may form geometric isomers. These are also intended to be included within the scope of the present invention.

Furthermore, some of the compounds of the present invention may exist in different tautomeric forms and it is intended that any tautomeric forms that the compounds are able to form are included within the scope of the present invention.

As mentioned above, the compounds of formula I are serotonin reuptake inhibitors, and accordingly may be applicable for the treatment, including prevention, of affective disorders, such as depression, anxiety disorders including general anxiety disorder and panic disorder and obsessive compulsive disorder.

Accordingly, in a further aspect the invention relates to a compound of formula I for use as a medicament.

The present invention also relates to a pharmaceutical composition comprising a compound of formula I and a pharmaceutically acceptable carrier or diluent. The composition may comprise any one of the embodiments of formula I described above.

In an embodiment of the pharmaceutical composition, the compound of formula I is present in an amount of from about 0.001 to about 100 mg/kg body weight per day.

The present invention also relates to use of a compound of formula I for the preparation of a medicament for the treatment of a disease or disorder, wherein a serotonin reuptake inhibitor is beneficial. The medicament may comprise any one of the embodiments of formula I described above,

In particular, the present invention also relates to use of a compound of formula I for the preparation of a medicament for the treatment of affective disorders.

In a further embodiment, the present invention also relates to use of a compound of formula I for the preparation of a medicament for the treatment of depression.

In a further embodiment, the present invention also relates to use of a compound of formula I for the preparation of a medicament for the treatment of anxiety disorders.

In a further embodiment, the present invention also relates to use of a compound of formula I for the preparation of a medicament for the treatment of general anxiety disorder.

In a further embodiment, the present invention also relates to use of a compound of formula I for the preparation of a medicament for the treatment of social anxiety disorder.

In a further embodiment, the present invention also relates to use of a compound of formula I for the preparation of a medicament for the treatment of post traumatic stress disorder.

In a further embodiment, the present invention also relates to use of a compound of formula I for the preparation of a medicament for the treatment of obsessive compulsive disorder.

In a further embodiment, the present invention also relates to use of a compound of formula I for the preparation of a medicament for the treatment of panic disorder.

In a further embodiment, the present invention also relates to use of a compound of formula I for the preparation of a medicament for the treatment of panic attacks.

In a further embodiment, the present invention also relates to use of a compound of formula I for the preparation of a medicament for the treatment of specific phobias.

In a further embodiment, the present invention also relates to use of a compound of formula I for the preparation of a medicament for the treatment of social phobia.

In a further embodiment, the present invention also relates to use of a compound of formula I for the preparation of a medicament for the treatment of agoraphobia.

In a further aspect, the present invention relates to a method of preparing a compound of formula I, comprising
a) deprotection or cleavage from a polymer support of a compound with formula II 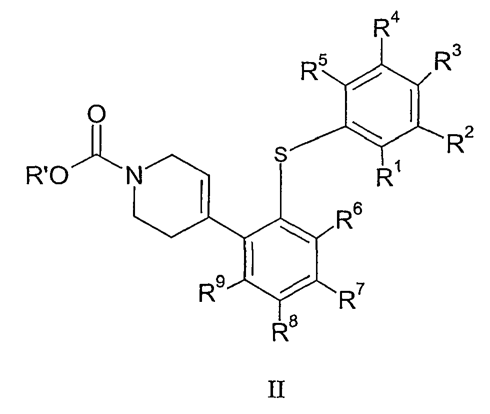 wherein R¹-R⁹ are as previously described, and R' is a *tert*-butyl, methyl, ethyl, allyl or benzyl group or R'OCO is a solid supported carbamate group, or
b) chemical transformation of a compound with formula III 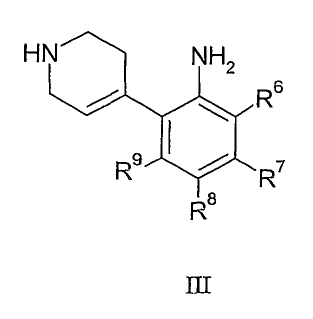 to the corresponding diazonium compound and subsequently reacting with a thiophenol of formula IV 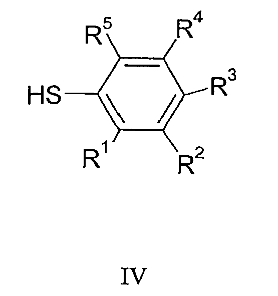 wherein R¹-R⁹ are as previously described, or
c) dehydrating and optionally simultaneously deprotecting a compound of formula V 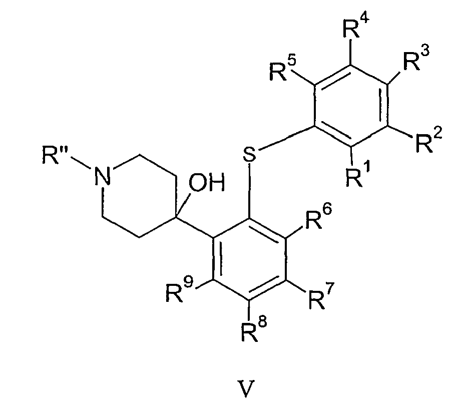 wherein R¹-R⁹ are as previously described, and R" is either a hydrogen atom or R" can be a carbamate R'OCO wherein R' is a *tert*-butyl, methyl, ethyl, allyl or benzyl group or R'OCO is a solid supported carbamate group.

### Pharmaceutical compositions

The compounds of the invention may be administered alone or in combination with pharmaceutically acceptable carriers or excipients, in either single or multiple doses. The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy, 19 Edition, Gennaro, Ed., Mack Publishing Co., Easton, PA, 1995.

The pharmaceutical compositions may be specifically formulated for administration by any suitable route such as the oral, rectal, nasal, pulmonary, topical (including buccal and sublingual), transdermal, intracisternal, intraperitoneal, vaginal and parenteral (including subcutaneous, intramuscular, intrathecal, intravenous and intradermal) route, the oral route being preferred. It will be appreciated that the preferred route will depend on the general condition and age of the subject to be treated, the nature of the condition to be treated and the active ingredient chosen.

Pharmaceutical compositions for oral administration include solid dosage forms such as capsules, tablets, dragees, pills, lozenges, powders and granules. Where appropriate, they can be prepared with coatings such as enteric coatings or they can be formulated so as to provide controlled release of the active ingredient such as sustained or prolonged release according to methods well known in the art.

Liquid dosage forms for oral administration include solutions, emulsions, suspensions, syrups and elixirs.

Pharmaceutical compositions for parenteral administration include sterile aqueous and nonaqueous injectable solutions, dispersions, suspensions or emulsions as well as sterile powders to be reconstituted in sterile injectable solutions or dispersions prior to use. Depot injectable formulations are also contemplated as being within the scope of the present invention.

Other suitable administration forms include suppositories, sprays, ointments, cremes, gels, inhalants, dermal patches, implants etc.

A typical oral dosage is in the range of from about 0.001 to about 100 mg/kg body weight per day, preferably from about 0.01 to about 50 mg/kg body weight per day, and more preferred from about 0.05 to about 10 mg/kg body weight per day administered in one or more dosages such as 1 to 3 dosages. The exact dosage will depend upon the frequency and mode of administration, the sex, age, weight and general condition of the subject treated, the nature and severity of the condition treated and any concomitant diseases to be treated and other factors evident to those skilled in the art.

The formulations may conveniently be presented in unit dosage form by methods known to those skilled in the art. A typical unit dosage form for oral administration one or more times per day such as 1 to 3 times per day may contain from 0.01 to about 1000 mg, preferably from about 0.05 to about 500 mg, and more preferred from about 0.5 mg to about 200 mg.

For parenteral routes such as intravenous, intrathecal, intramuscular and similar administration, typically doses are in the order of about half the dose employed for oral administration.

The compounds of this invention are generally utilized as the free substance or as a pharmaceutically acceptable salt thereof. One example is an acid addition salt of a compound having the utility of a free base. When a compound of the formula (I) contains a free base such salts are prepared in a conventional manner by treating a solution or suspension of a free base of the formula (I) with a chemical equivalent of a pharmaceutically acceptable acid. Representative examples are mentioned above.

For parenteral administration, solutions of the novel compounds of the formula (I) in sterile aqueous solution, aqueous propylene glycol, aqueous vitamin E or sesame or peanut oil may be employed. Such aqueous solutions should be suitably buffered if necessary and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art.

Suitable pharmaceutical carriers include inert solid diluents or fillers, sterile aqueous solution and various organic solvents. Examples of solid carriers are lactose, terra alba, sucrose, cyclodextrin, talc, gelatine, agar, pectin, acacia, magnesium stearate, stearic acid and lower alkyl ethers of cellulose. Examples of liquid carriers are syrup, peanut oil, olive oil, phospho lipids, fatty acids, fatty acid amines, polyoxyethylene and water. Similarly, the carrier or diluent may include any sustained release material known in the art, such as glyceryl monostearate or glyceryl distearate, alone or mixed with a wax. The pharmaceutical compositions formed by combining the novel compounds of the formula (I) and the pharmaceutical acceptable carriers are then readily administered in a variety of dosage forms suitable for the disclosed routes of administration. The formulations may conveniently be presented in unit dosage form by methods known in the art of pharmacy.

Formulations of the present invention suitable for oral administration may be presented as discrete units such as capsules or tablets, each containing a predetermined amount of the active ingredient, and which may include a suitable excipient. Furthermore, the orally available formulations may be in the form of a powder or granules, a solution or suspension in an aqueous or non-aqueous liquid, or an oil-in-water or water-in-oil liquid emulsion.

If a solid carrier is used for oral administration, the preparation may be a tablet, placed in a hard gelatine capsule in powder or pellet form or it can be in the form of a troche or lozenge.

The amount of solid carrier will vary widely but will usually be from about 25 mg to about 1 g.

If a liquid carrier is used, the preparation may be in the form of a syrup, emulsion, soft gelatine capsule or sterile injectable liquid such as an aqueous or non-aqueous liquid suspension or solution.

The compounds of the invention are prepared by the following general methods:
a) Deprotection or cleavage from a polymer support of a compound with formula II 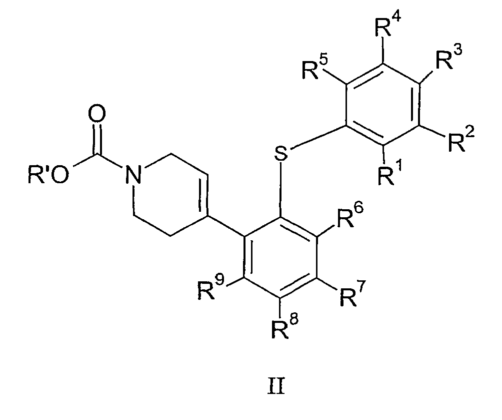 wherein R¹-R⁹ are as previously described, and R' is a *tert*-butyl, methyl, ethyl, allyl or benzyl group or R'OCO is a solid supported carbamate group, such as the Wang resin-based carbamate linker.
b) Chemical transformation of a compound with formula III to the corresponding diazonium compound and subsequently reacting with a thiophenol of formula IV 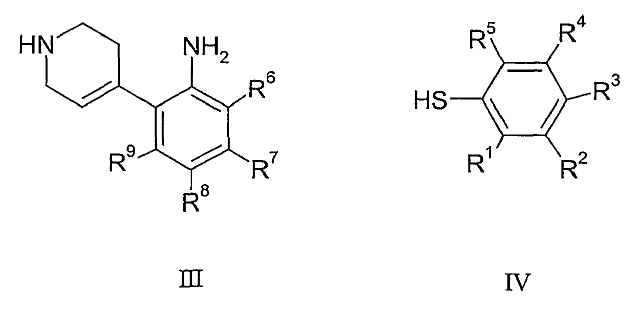 wherein R¹-R⁹ are as previously described,
c) Dehydrating and optionally simultaneously deprotecting a compound of formula V 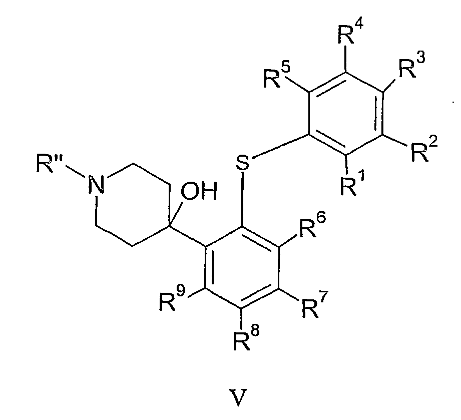 wherein R¹-R⁹ are as previously described, and R" is either a hydrogen atom or R" can be a carbamate R'OCO wherein R' is a *tert-*butyl, methyl, ethyl, allyl or benzyl group or R'OCO is a solid supported carbamate group, such as the Wang resin-based carbamate linker.

The deprotection according to method a) was performed by standard techniques, known to the persons skilled in the art and detailed in the textbook Protective Groups in Organic Synthesis T.W.Greene and P.G.M. Wuts, Wiley Interscience, (1991) ISBN 0471623016. The cleavage from a polymer support, such as from the Wang resin based carbamate linker, according to method a) was performed according to literature known procedures (Zaragoza Tetrahedron Lett. 1995, 36, 8677-8678 and Conti et al. Tetrahedron Lett. 1997, 38, 2915-2918).

Starting materials of formula II in method a) can be prepared by dehydrating a compound of formula V by treatment of V with an acid e.g. trifluoro acetic acid or concentrated HCl in glacial acetic acid (1:5) as illustrated in the experimental procedure below. Starting materials of formula II can also be prepared by reacting a compound of formula VI with a thiophenol of formula IV in the presence of a palladium catalyst as illustrated in the experimental procedure below. Compounds of formula VI and IV 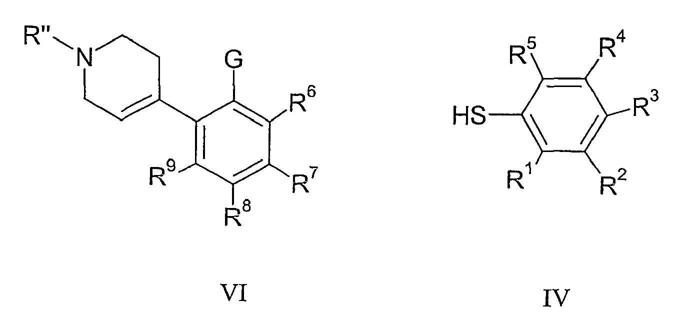 wherein R¹-R⁹ are as previously described, and G is a bromine or iodine atom.

The diazotation followed by reaction with a thiophenol IV according to the method b) can be performed by addition of the diazonium salt of the corresponding aniline to a solution of sodium salt of a thiophenol in an aqueous suspension of copper. The starting material of formula III and the corresponding diazonium salt can be prepared by methods analogues to those described in the literature (e.g. Berridge, M. S. et al. J. Med. Chem. 1993, 36,1284-1290). Thiophenols of the formula IV are either commercially available or can be prepared according to methods described in standard works such as Houben-Weyl, Methoden der organischen Chemie (Methods of Organic Chemistry), Georg-Thieme-Verlag, Stuttgart; Organic Reactions, John Wiley & Sons, Inc. New York, namely under reaction conditions such as those which are known and suitable for such reactions.

The dehydration reaction and optional simultaneous deprotection of a compound of formula V in method c) was performed in a similar manner as described in Palmer et al J. Med. Chem. 1997, 40, 1982-1989 or by acid treatment as illustrated in the experimental procedure below.

Starting materials of formula V in method c) can be prepared from the corresponding properly substituted 1-bromo-phenylsulfanylbenzenes of formula VII by metal-halogen exchange followed by addition of an appropriate electrophile of the formula VIII in a similar manner as described in Palmer et al. J. Med. Chem. 1997, 40, 1982-1989. Compounds of formula VIII and VII 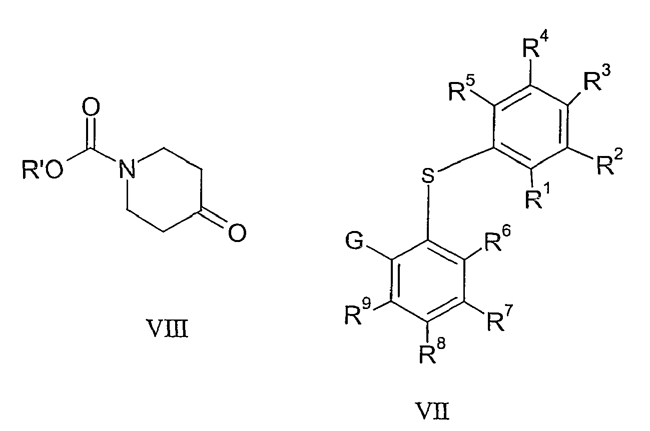 wherein R¹-R⁹ and R' are as previously described, and G is a bromine or iodine atom. The properly substituted 1-bromo-phenylsulfanylbenzenes VII were prepared in a similar manner as described in the literature by reaction of properly substituted thiophenols with properly substituted aryliodides according to Schopfer and Schlapbach Tetrahedron 2001, 57, 3069-3073; Bates et al., Org. Lett. 2002, 4, 2803-2806 and Kwong et al. Org. Lett. 2002, 4, 581-584.
Starting materials of formula V in method c) were also prepared by reaction of thiophenols of formula IX with the corresponding properly substituted aryliodides or arylbromides X in the presence of a palladium catalyst as illustrated in the experimental procedure below. Compounds of formula IX and X 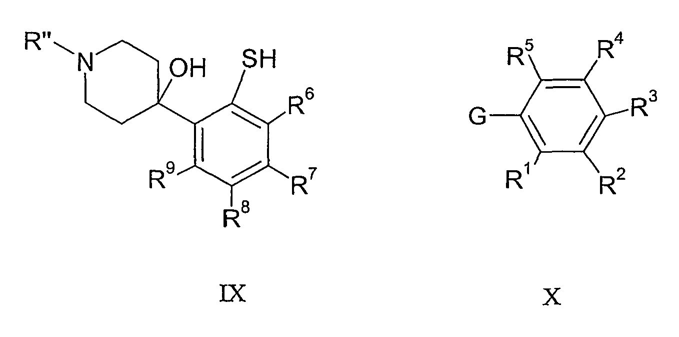 wherein R¹-R⁹ and R' are as previously described, and G is a bromine or iodine atom.

### Examples

Analytical LC-MS data were obtained on a PE Sciex API 150EX instrument equipped with IonSpray source and Shimadzu LC-8A/SLC-10A LC system. Column: 30 X 4.6 mm Waters Symmmetry C18 column with 3.5 µm particle size; Solventsystem: A = water/trifluoroacetic acid (100:0.05) and B = water/acetonitrile/trifluoroacetic acid (5:95:0.03); Method: Linear gradient elution with 90% A to 100% B in 4 min and with a flow rate of 2 mL/min. Purity was determined by integration of the UV (254 nm) and ELSD trace. The retention times (RT) are expressed in minutes.
Preparative LC-MS-purification was performed on the same instrument. Column: 50 X 20 mm YMC ODS-A with 5 µm particle size; Method: Linear gradient elution with 80% A to 100% B in 7 min and with a flow rate of 22.7 mL/min. Fraction collection was performed by split-flow MS detection.
For ion-exchange chromatography, the following material was used: SCX-columns (1 g) from Varian Mega Bond Elut®, Chrompack cat. No. 220776. Prior to use, the SCX-columns were pre-conditioned with 10% solution of acetic acid in methanol (3 mL). For de-complexation by irradiation, a ultaviolet light source (300 W) from Philipps was used. As starting polymer supports for solid phase synthesis, Wang-resin (1.03 mmol/g, Rapp-Polymere, Tuebingen, Germany) was used.

### Preparation of Intermediates

### 4-Hydroxy-4-[2-(4-chlorophenylsulfanyl)phenyl]-piperidine-1-carboxylic acid tert-butyl ester.

A solution of BuLi (2.5 M in hexane, 12.0 ml, 30 mmol) was slowly added to a stirred solution of 1-bromo-2-(4-chlorophenylsulfanyl)benzene (30 mmol) in dry THF (75 ml) under Argon at -78 °C. The solution was stirred for 10 min before 4-oxo-piperidine-1-carboxylic acid *tert*-butyl ester (5.98 g, 30 mmol) was added in one portion. The solution was allowed to warm up to room temperature and then stirred for 3 h. Saturated aqueous NH₄Cl (150 ml) was added and the solution was extracted with ethylacetate (150 mL). The organic phase was washed with brine, dried (MgSO₄) and the solvent was evaporated *in vacuo.* Crude product was purified by flash chromatography on silica gel (eluent: Ethylacetat/heptane 20:80) to produce the target compound as a white foam. HPLC: RT = 3.97; purity UV: 92%; ELSD: 99%; yield: 4.66 g (37%).
The following derivatives were prepared analogously:
4-[2-(4-Fluorophenylsulfanyl)-phenyl]piperidin-4-ol,
4-[2-(4-Chlorophenylsulfanyl)-phenyl]piperidin-4-ol,
4-[2-(4-Methoxyphenylsulfanyl)-5-methyl-phenyl]piperidin-4-ol,
4-(5-Methyl-2-p-tolylsulfanylphenyl)piperidin-4-ol,
4-[2-(4-Fluorophenylsulfanyl)-5-methyl-phenyl]piperidin-4-ol,
4-[2-(4-Chlorophenylsulfanyl)-5-methyl-phenyl]piperidin-4-ol,
4-[2-(2,4-Dimethylphenylsulfanyl)-phenyl]piperidin-4-ol,
4-[2-(4-Chlorophenylsulfanyl)-5-trifluoromethyl-phenyl]piperidin-4-ol,
4-[2-(4-Fluoro-2-methylphenylsulfanyl)-phenyl]piperidin-4-ol,
4-[2-(4-Chlorophenylsulfanyl)-4-fluoro-phenyl]piperidin-4-ol,
4-[4-Fluoro-2-(p-tolylsulfanyl)-phenyl]piperidin-4-ol,
4-[4-Fluoro-2-(4-methoxyphenylsulfanyl)-phenyl]piperidin-4-ol,
4-[2-(2,4-Dimethylphenylsulfanyl)-5-methyl-phenyl]piperidin-4-ol,
4-[2-(2,4-Dimethylphenylsulfanyl)-5-trifluoromethyl-phenyl]piperidin-4-ol,
4-(2-p-Tolylsulfanyl-5-trifluoromethylphenyl)piperidin-4-ol,
4-[2-(4-Fluoro-2-methylphenylsulfanyl)-5-methyl-phenyl]piperidin-4-ol,
4-[5-Bromo-2-(2,4-dimethylphenylsulfanyl)-phenyl]piperidin-4-ol,
4-[2-(4-Chlorophenylsulfanyl)-5-fluoro-phenyl]piperidin-4-ol,
4-[5-Fluoro-2-(4-methoxyphenylsulfanyl)-phenyl]piperidin-4-ol,
4-[2-(2,4-Dichlorophenylsulfanyl)-phenyl]-piperidin-4-ol,
4-[2-(4-Chloro-2-fluoro-phenylsulfanyl)-phenyl]piperidin-4-ol,
4-[2-(3-Methoxyphenylsulfanyl)-phenyl]piperidin-4-ol,
4-[3-Fluoro-2-(4-methoxyphenylsulfanyl)-phenyl]piperidin-4-ol,
4-[2-(2-Chlorophenylsulfanyl)-phenyl]piperidin-4-ol,
4-[2-(2-Chloro-4-methoxyphenylsulfanyl)-phenyl]piperidin-4-ol,
4-[2-(2-Fluorophenylsulfanyl)-phenyl]piperidin-4-ol,
4-[2-(2-Bromophenylsulfanyl)-phenyl]piperidin-4-ol,
4-[2-(4-Bromophenylsulfanyl)-phenyl]piperidin-4-ol,
4-(2-o-Tolylsulfanylphenyl)piperidin-4-ol,

### 4-Hydroxy-4-(2-mercaptophenyl)-piperidine-1-carboxylic acid tert-butyl ester.

4 mL 1.6 M *n*-butyllithium in hexane (6.4 mmol) was added dropwise to 590 mg 2-bromo-thiophenol (3.1 mmol) in 5 mL dry THF at -78 °C. The solution was stirred 30 min. 640 mg *N*-*tert*-butoxycarbonyl piperidone (3.2 mmol) in 5 mL dry THF was added dropwise and the reaction mixture was stirred 3 hours at -78 °C. The solution was poured into sat. NH₄Cl (aq) and extracted with ethyl acetate (2×50mL). The combined organic phases were washed with brine, dried with MgSO₄ and concentrated *in vacuo.* Flash chromatography (ethyl acetate/heptane) yielded 650 mg product (2.1 mmol, 66%).

### Compounds of the invention:

### Example 1

### Synthesis method A:

### Compound 2: 4-[2-(4-Chorophenylsulfanyl)-phenyl]-1,2,3,6-tetrahydropyridine

Concentrated aq hydrochloric acid (10 mL) was added to a stirred solution of 1-*tert-*butoxycarbonyl-4-[2-(4-chlorophenylsulfanyl)phenyl]piperidin-4-ol (0.84 g, 2 mmol) in acetic acid (30 mL). The solution was boiled under reflux overnight, cooled to room temperature and then stirred in an ice bath. An aqueous solution of NaOH (9.1 M, 40 mL) was slowly added and the unclear solution was extracted with ethyl acetate (2 x 40 ml). The combined organic phases were dried (MgSO₄) and the solvents evaporated *in vacuo.* The crude material (0.48 g) was dissolved in ethyl acetate (3.2 mL) at 50 °C and a solution of oxalic acid (0.11 g) in EtOH (3.2 mL) was slowly added. The target compound was collected as a white oxalic salt. ¹H (DMSO-*d₆*) δ 7.3-7.2 (m, 7H); 7.15 (m, 1H); 7.00 (m, 1H); 5.6 (d, 1H); 3.7 (d, 2H); 3.25 (t, 2H); 2.6 (m, 2H); LC/MS (m/z) 302.1 (MH⁺); RT = 2.29; purity (UV, ELSD): 100%, 100%; yield: 0.31 g (40%).

### Example 2

### Synthesis method B:

### Compound 24: 4-[2-(2-Chlorophenylsulfanyl)-phenyl]-1,2,3,6-tetrahydro-pyridine.

Bis[(2-diphenylphosphino) phenyl] ether (22mg; 0.04 mmol) and bis(dibenzylidene)palladium (23 mg; 0.04 mmol) were dissolved in 2.5 mL toluene and added to a solution of 4-hydroxy-4-(2-mercapto-phenyl)-piperidine-1-carboxylic acid *tert*-butyl ester (200 mg; 0.64 mmol) and 1-chloro-2-iodobenzene (200 mg; 0.84 mmol) in 4 mL toluene. Potassium *tert*-butoxide (80 mg; 0.68 mmol) was added and the reaction mixture was stirred under argon for 16 hours at 100°C. The reaction mixture was filtered through silica with ethyl acetate as eluent. The solvent was removed *in vacuo.* The residue was dissolved in 4 mL glacial acetic acid and 1 mL concentrated HCl. The mixture was stirred for 16 hours at 100 °C. The solution was poured on to ice and 8 mL 28% aquous NaOH was added. The alkaline suspension was extracted with ethyl acetate (2x50 mL). The combined organic phases were washed with brine, dried with MgSO₄ and concentrated *in vacuo* to give a brown oil. LC-MS: M+H: 302.1; retention time = 2.03 min; UV: 78%; ELSD 94%.

**The following compounds were made by the methods indicated in table 1 and accompanying analytical data are shown in table 1.**

### Examples

**1.** 4-[2-(4-Fluorophenylsulfanyl)-phenyl]-1,2,3,6-tetrahydropyridine,
**2.** 4-[2-(4-Chlorophenylsulfanyl)-phenyl]-1,2,3,6-tetrahydropyridine,
**3.** 4-[2-(4-Methoxyphenylsulfanyl)-5-methyl-phenyl]-1,2,3,6-tetrahydropyridine,
**4.** 4-(5-Methyl-2-p-tolylsulfanylphenyl)-1,2,3,6-tetrahydropyridine,
**5.** 4-[2-(4-Fluorophenylsulfanyl)-5-methyl-phenyl]-1,2,3,6-tetrahydropyridine,
**6.** 4-[2-(4-Chlorophenylsulfanyl)-5-methyl-phenyl]-1,2,3,6-tetrahydropyridine,
**7.** 4-[2-(2,4-Dimethylphenylsulfanyl)-phenyl]-1,2,3,6-tetrahydropyridine,
**8.** 4-[2-(4-Chlorophenylsulfanyl)-5-trifluoromethyl-phenyl]-1,2,3,6-tetrahydropyridine,
**9.** 4-[2-(4-Fluoro-2-methylphenylsulfanyl)-phenyl]-1,2,3,6-tetrahydropyridine,
**10.** 4-[2-(4-Chlorophenylsulfanyl)-4-fluoro-phenyl]-1,2,3,6-tetrahydropyridine,
**11.** 4-[4-Fluoro-2-(p-tolylsulfanyl)-phenyl]-1,2,3,6-tetrahydropyridine,
**12.** 4-[4-Fluoro-2-(4-methoxyphenylsulfanyl)-phenyl]-1,2,3,6-tetrahydropyridine,
**13.** 4-[2-(2,4-Dimethylphenylsulfanyl)-5-methyl-phenyl]-1,2,3,6-tetrahydropyridine,
**14.** 4-[2-(2,4-Dimethylphenylsulfanyl)-5-trifluoromethyl-phenyl]-1,2,3,6-tetrahydropyridine,
**15.** 4-(2-p-Tolylsulfanyl-5-trifluoromethylphenyl)-1,2,3,6-tetrahydropyridine,
**16.** 4-[2-(4-Fluoro-2-methylphenylsulfanyl)-5-methyl-phenyl]-1,2,3,6-tetrahydropyridine,
**17.** 4-[5-Bromo-2-(2,4-dimethylphenylsulfanyl)-phenyl]-1,2,3,6-tetrahydropyridine,
**18.** 4-[2-(4-Chlorophenylsulfanyl)-5-fluoro-phenyl]-1,2,3,6-tetrahydropyridine,
**19.** 4-[5-Fluoro-2-(4-methoxyphenylsulfanyl)-phenyl]-1,2,3,6-tetrahydropyridine,
**20.** 4-[2-(2,4-Dichlorophenylsulfanyl)-phenyl]-1,2,3,6-tetrahydropyridine,
**21.** 4-[2-(4-Chloro-2-fluoro-phenylsulfanyl)-phenyl]-1,2,3,6-tetrahydropyridine,
**22.** 4-[2-(3-Methoxyphenylsulfanyl)-phenyl]-1,2,3,6-tetrahydropyridine,
**23.** 4-[3-Fluoro-2-(4-methoxyphenylsulfamyl)-phenyl]-1,2,3,6-tetrahydropyridine,
**24.** 4-[2-(2-Chlorophenylsulfanyl)-phenyl]-1,2,3,6-tetrahydropyridine,
**25.** 4-[2-(2-Chloro-4-methoxyphenylsulfanyl)-phenyl]-1,2,3,6-tetrahydropyridine,
**26.** 4-[2-(2-Fluorophenylsulfanyl)-phenyl]-1,2,3,6-tetrahydropyridine,
**27.** 4-[2-(2-Bromophenylsulfanyl)-phenyl]-1,2,3,6-tetrahydropyridine,
**28.** 4-[2-(4-Bromophenylsulfanyl)-phenyl]-1,2,3,6-tetrahydropyridine,
**29.** 4-(2-o-Tolylsulfanylphenyl)-1,2,3,6-tetrahydropyridine,
**30.** 4-[2-(4-Chloro-2-methylphenylsulfanyl)-phenyl]-1,2,3,6-tetrahydropyridine,
**31.** 4-[2-(4-Trifluoromethyl-pheriylsulfanyl)-phenyl]-1,2,3,6-tetrahydro-pyridine,
**32.** 4-[2-(2,3-Dichloro-phenylsulfanyl)-phenyl]-1,2,3,6-tetrahydro-pyridine,
**33.** 4-[2-(2,3-Dimethyl-phenylsulfanyl)-phenyl]-1,2,3,6-tetrahydro-pyridine,
**34.** 4-[2-(3,4-Dimethyl-phenylsulfanyl)-phenyl]-1,2,3,6-tetrahydro-pyridine,
**35.** 4-[2-(2-Methoxy-5-methyl-phenylsulfanyl)-phenyl]-1,2,3,6-tetrahydro-pyridine,
**36.** 4-[2-(2-Chloro-4-fluoro-phenylsulfanyl)-phenyl]-1,2,3,6-tetrahydro-pyridine,
**37.** 4-[2-(4-Methoxy-2-methyl-phenylsulfanyl)-phenyl]-1,2,3,6-tetrahydro-pyridine,
**38.** 4-[2-(2-Fluoro-4-methoxy-phenylsulfanyl)-phenyl]-1,2,3,6-tetrahydro-pyridine,
**39.** 4-[2-(2,4-Dimethyl-phenylsulfanyl)-5-fluoro-phenyl]-1,2,3,6-tetrahydro-pyridine,
**40.** 4-(5-Fluoro-2-phenylsulfanyl-phenyl)-1,2,3,6-tetrahydro-pyridine,
**41.** 4-[5-Fluoro-2-(4-fluoro-phenylsulfanyl)-phenyl]-1,2,3,6-tetrahydro-pyridine,
**42.** 4-(2-m-Tolylsulfanyl-phenyl)-1,2,3,6-tetrahydro-pyridine,
**43.** 4-[2-(4-Fluoro-3-methoxy-phenylsulfanyl)-phenyl]-1,2,3,6-tetrahydro-pyridine,
**44.** 4-[2-(2-Bromo-4-fluoro-phenylsulfanyl)-phenyl]-1,2,3,6-tetrahydro-pyridine,
**45.** 4-[2-(2-Chloro-4-fluoro-phenylsulfanyl)-5-fluoro-phenyl]-1,2,3,6-tetrahydro-pyridine,
**46.** 4-[2-(2,4-Dichloro-phenylsulfanyl)-5-fluoro-phenyl]-1,2,3,6-tetrahydro-pyridine,
**47.** 4-[2-(3-Chloro-phenylsulfanyl)-phenyl]-1,2,3,6-tetrahydro-pyridine,
**48.** 4-[2-(2-Methoxy-phenylsulfanyl)-phenyl]-1,2,3,6-tetrahydro-pyridine,
**49.** 4-[2-(2,4-Dichloro-phenylsulfanyl)-5-methyl-phenyl]-1,2,3,6-tetrahydro-pyridine,
**50.** 4-[2-(4-Chloro-2-fluoro-phenylsulfanyl)-5-methyl-phenyl]-1,2,3,6-tetrahydro-pyridine,
**51.** 4-[2-(4-Chloro-2-methyl-phenylsulfanyl)-5-methyl-phenyl]-1,2,3,6-tetrahydro-pyridine,
**52.** 4-[2-(2-Fluoro-4-methyl-phenylsulfanyl)-5-methyl-phenyl]-1,2,3,6-tetrahydro-pyridine,
**53.** 4-[2-(2,4-Difluoro-phenylsulfanyl)-5-methyl-phenyl]-1,2,3,6-tetrahydro-pyridine,
**54.** 4-[2-(2-Chloro-4-fluoro-phenylsulfanyl)-5-methyl-phenyl]-1,2,3,6-tetrahydro-pyridine,
**55.** 4-[2-(2-Bromo-4-fluoro-phenylsulfanyl)-5-methyl-phenyl]-1,2,3,6-tetrahydro-pyridine,
**56.** 4-[2-(2-Bromo-4-methyl-phenylsulfanyl)-5-methyl-phenyl]-1,2,3,6-tetrahydro-pyridine,
**57.** 4-[2-(2-Bromo-phenylsulfanyl)-5-methyl-phenyl]-1,2,3, 6-tetrahydro-pyridine,
**58.** 4-[2-(4-Bromo-phenylsulfanyl)-5-methyl-phenyl]-1,2,3,6-tetrahydro-pyridine,
**59.** 4-(5-Methyl-2-o-tolylsulfanyl-phenyl)-1,2,3,6-tetrahydro-pyridine,
**60.** 4-[2-(4-Methoxy-2-methyl-phenylsulfanyl)-5-methyl-phenyl]-1,2,3,6-tetrahydro-pyridine,
**61.** 4-[2-(2-Fluoro-4-methoxy-phenylsulfanyl)-5-methyl-phenyl]-1,2,3,6-tetrahydro-pyridine,
**62.** 4-[2-(3,4-Dichloro-phenylsulfanyl)-5-methyl-phenyl]-1,2,3,6-tetrahydro-pyridine,
**63.** 4-[2-(2-Chloro-4-methyl-phenylsulfanyl)-5-methyl-phenyl]-1,2,3,6-tetrahydro-pyridine,
**64.** 4-[2-(2-Fluoro-4-methyl-phenylsulfanyl)-phenyl]-1,2,3,6-tetrahydro-pyridine,
**65.** 4-[2-(3,4-Dichloro-phenylsulfanyl)-phenyl]-1,2,3,6-tetrahydro-pyridine,
**66.** 4-[2-(4-Bromo-2-fluoro-phenylsulfanyl)-phenyl]-1,2,3,6-tetrahydro-pyridine,
**67.** 4-[2-(4-Bromo-2-methyl-phenylsulfanyl)-phenyl]-1,2,3,6-tetrahydro-pyridine,
**68.** 4-[2-(2,4-Difluoro-phenylsulfanyl)-phenyl]-1,2,3,6-tetrahydro-pyridine,
**69.** 4-[2-(2-Bromo-4-methyl-phenylsulfanyl)-phenyl]-1,2,3,6-tetrahydro-pyridine,
**70.** 4-[2-(4-Chloro-2-methoxy-phenylsulfanyl)-phenyl]-1,2,3,6-tetrahydro-pyridine,
**71.** 4-[5-Fluoro-2-(2-fluoro-4-methoxy-phenylsulfanyl)-phenyl]-1,2,3,6-tetrahydro-pyridine,
**72.** 4-[2-(2-Chloro-4-methoxy-phenylsulfanyl)-5-fluoro-phenyl]-1,2,3,6-tetrahydro-pyridine,
**73.** 4-[5-Fluoro-2-(2-fluoro-4-methyl-phenylsulfanyl)-phenyl]-1,2,3,6-tetrahydro-pyridine,
**74.** 4-[2-(2-Chloro-4-methyl-phenylsulfanyl)-5-fluoro-phenyl]-1,2,3,6-tetrahydro-pyridine,
**75.** 4-[2-(4-Chloro-2-fluoro-phenylsulfanyl)-5-fluoro-phenyl]-1,2,3,6-tetrahydro-pyridine,
**76.** 4-[5-Fluoro-2-(4-methoxy-2-methyl-phenylsulfanyl)-phenyl]-1,2,3,6-tetrahydro-pyridine,
**77.** 4-[2-(2,3-Dimethyl-phenylsulfanyl)-5-methyl-phenyl]-1,2,3,6-tetrahydro-pyridine,
**78.** 4-[5-Methyl-2-(3-methyl-phenylsulfanyl)-phenyl]-1,2,3,6-tetrahydro-pyridine,
**79.** 4-[2-(2-Fluoro-phenylsulfanyl)-5-methyl-phenyl]-1,2,3,6-tetrahydro-pyridine,
**80.** 4-[2-(2-Chloro-phenylsulfanyl)-5-methyl-phenyl]-1,2,3,6-tetrahydro-pyridine,
**81.** 4-[2-(3,4-Dimethyl-phenylsulfanyl)-5-methyl-phenyl]-1,2,3,6-tetrahydro-pyridine,
**82.** 4-[2-(4-Chloro-2-methoxy-phenylsulfanyl)-5-methyl-phenyl]-1,2,3,6-tetratrydro-pyridine,
**83.** 4-[2-(2,3-Difluoro-plienylsulfanyl)-phenyl]-1,2,3,6-tetrahydro-pyridine,
**84.** 4-[2-(2-Chloro-4-methyl-phenylsulfanyl)-phenyl]-1,2,3,6-tetrahydro-pyridine,
**85.** 4-[2-(2,3-Difluoro-phenylsulfanyl)-5-methyl-phenyl]-1,2,3,6-tetrahydro-pyridine,
**86.** 4-[2-(3-Chloro-2-fluoro-phenylsulfanyl)-5-methyl-phenyl]-1,2,3,6-tetrahydro-pyridine,
**87.** 4-[2-(3-Fluoro-2-methyl-phenylsulfanyl)-phenyl]-1,2,3,6-tetrahydro-pyridine,
**88.** 4-[2-(3-Chloro-2-methyl-phenylsulfanyl)-phenyl]-1,2,3,6-tetrahydro-pyridine,
**89.** 4-[2-(3-Fluoro-2-methyl-phenylsulfanyl)-5-methyl-phenyl]-1,2,3,6-tetrahydro-pyridine,
**90.** 4-[2-(3-Fluoro-phenylsulfanyl)-5-methyl-phenyl]-1,2,3,6-tetrahydro-pyridine,
**91.** 4-[2-(3-Chloro-phenylsulfanyl)-5-methyl-phenyl]-1,2,3,6-tetrahydro-pyridine,
**92.** 4-[2-(3-Bromo-phenylsulfanyl)-5-methyl-phenyl]-1,2,3,6-tetrahydro-pyridine,
**93.** 4-[2-(3-Methoxy-phenylsulfanyl)-5-methyl-phenyl]-1,2,3,6-tetrahydro-pyridine,
**94.** 4-[5-Methyl-2-(3-trifluoromethyl-phenylsulfanyl)-phenyl]-1,2,3,6-tetrahydro-pyridine,
**95.** 4-[2-(2-Methoxy-phenylsulfanyl)-5-methyl-phenyl]-1,2,3,6-tetrahydro-pyridine,
**96.** 4-[2-(2-Ethyl-phenylsulfanyl)-phenyl]-1,2,3,6-tetrahydro-pyridine,
**97.** 4-[2-(4-Ethyl-phenylsulfanyl)-phenyl]-1,2,3,6-tetrahydro-pyridine,
**98.** 4-[2-(2-tert-Butyl-phenylsulfanyl)-phenyl]-1,2,3,6-tetrahydro-pyridine,
**99.** 4-[2-(4-tert-Butyl-phenylsulfanyl)-phenyl]-1,2,3,6-tetrahydro-pyridine,
**100.** 4-[2-(3-Fluoro-phenylsulfanyl)-phenyl]-1,2,3,6-tetrahydro-pyridine,
**101.** 4-[2-(2-Trifluoromethyl-phenylsulfanyl)-phenyl]-1,2,3,6-tetrahydro-pyridine,
**102.** 4-[2-(3-Trifluoromethyl-phenylsulfanyl)-phenyl]-1,2,3,6-tetrahydro-pyridine,
**103.** 4-[2-(4-Trifluoromethoxy-phenylsulfanyl)-phenyl]-1,2,3,6-tetrahydro-pyridine,
**104.** 4-[2-(4-Methylsulfanyl-phenylsulfanyl)-phenyl]-1,2,3,6-tetrahydro-pyridine,
**105.** 4-[2-(3,5-Dimethyl-phenylsulfanyl)-phenyl]-1,2,3,6-tetrahydro-pyridine,
**106.** 4-[2-(2,5-Dimethyl-phenylsulfanyl)-phenyl]-1,2,3,6-tetrahydro-pyridine,
**107.** 4-[2-(2,5-Dichloro-phenylsulfanyl)-phenyl]-1,2,3,6-tetrahydro-pyridine,
**108.** 4-[2-(3, 5-Dichloro-phenylsulfanyl)-phenyl]-1,2, 3, 6-tetrahydro-pyridine,
**109.** 4-[2-(3-Chloro-4-fluoro-phenylsulfanyl)-phenyl]-1,2,3,6-tetrahydro-pyridine,
**110.** 4-[2-(2,4,6-Trimethyl-phenylsulfanyl)-phenyl]-1,2,3,6-tetrahydro-pyridine,
**111.** 4-[5-Methylamino-2-(4-methyl-pheylsulfanyl)-phenyl]-1,2,3,6-tetrahydro-pyridine,
**112.** 4-[5-Fluoro-2-(2-methoxy-phenylsulfanyl)-phenyl]-1,2,3,6-tetrahydro-pyridine,
**113.** 4-(5-Fluoro-2-o-tolylsulfanyl-phenyl)-1,2,3,6-tetrahydro-pyridine,
**114.** 4-(5-Fluoro-2-p-tolylsulfanyl-phenyl)-1,2,3,6-tetrahydro-pyridine,
**115.** 4-[2-(Benzo[1,3]dioxol-5-ylsulfanyl)-5-methyl-phenyl]-1,2,3,6-tetrahydro-pyridine,
**116.** 4-[2-(2-Chloro-4-methoxy-phenylsulfanyl)-5-methyl-phenyl]-1,2,3,6-tetrahydro-pyridine,
**117.** 4-[2-(2,3-Dichloro-phenylsulfanyl)-5-methyl-phenyl]-1,2,3,6-tetrahydro-pyridine,
**118.** 4-[2-(3-Chloro-2-methyl-phenylsulfanyl)-5-methyl-phenyl]-1,2,3,6-tetrahydro-pyridine,
**119.** 4-[2-(Benzo[1,3]dioxol-5-ylsulfanyl)-phenyl]-1,2,3,6-tetrahydro-pyridine,
**120.** 4-[5-Fluoro-2-(3-fluoro-phenylsulfanyl)-phenyl]-1,2,3,6-tetrahydro-pyridine,
**121.** 4-(5-Fluoro-2-m-tolylsulfanyl-phenyl)-1,2,3,6-tetrahydro-pyridine,
**122.** 4-[5-Fluoro-2-(3-methoxy-phenylsulfanyl)-phenyl]-1,2,3,6-tetrahydro-pyridine,
**123.** 4-[2-(3-Chloro-phenylsulfanyl)-5-fluoro-phenyl]-1,2,3,6-tetrahydro-pyridine,
**124.** 4-[2-(2-Chloro-phenylsulfanyl)-5-fluoro-phenyl]-1,2,3, 6-tetrahydro-pyridine,
**125.** 4-[4-Fluoro-2-(4-methyl-pheylsulfanyl)-phenyl]-1,2,3,6-tetrahydro-pyridine,
**126.** 4-[2-(4-Bromo-2-fluoro-phenylsulfanyl)-5-fluoro-phenyl]-1,2,3,6-tetrahydro-pyridine,
**127.** 4-[2-(4-Fluoro-3-methoxy-phenylsulfanyl)-5-methyl-phenyl]-1,2,3,6-tetrahydro-pyridine,
**128.** 4-[2-(3-Fluoro-4-methyl-phenylsulfanyl)-5-methyl-phenyl]-1,2,3,6-tetrahydro-pyridine.
**129.** 4-[5-Fluoro-2-(4-methoxy-phenylsulfanyl)-phenyl]-1,2,3,6-tetrahydro-pyridine

**Table 1. Measured molecular mass, measured HPLC-retention time (RT, min) and UV- and ELSD-purities (%) and synthesis method.**

| **compound** | **M+H⁺** | **RT min.** | **UV-purity** | **ELSDpurity** | **Synthesis** |
|---|---|---|---|---|---|
| | | | **(%)** | **(%)** | **method** |
| **1** | | 2.66 | 99.0 | 100 | A |
| **2** | 302.1 | 2.29 | 100 | 100 | A |
| **3** | 312.1 | 2.12 | 96.7 | 99.5 | A |
| **4** | 296.1 | 2.24 | 98.1 | 99.4 | A |
| **5** | 300.2 | 2.14 | 85.0 | 100 | A |
| **6** | 315.9 | 2.29 | 99.9 | 99.4 | A |
| **7** | 296.0 | 2.33 | 86.1 | 99.4 | A |
| **8** | 370.0 | 2.43 | 98.1 | 99.8 | A |
| **9** | 300.2 | 2.20 | 91.3 | 99.1 | A |
| **10** | 319.9 | 2.20 | 89.3 | 97.2 | A |
| **11** | 300.2 | 2.20 | 98.6 | 99.2 | A |
| **12** | 315.9 | 2.08 | 90.9 | 98.4 | A |
| **13** | 310.3 | 2.37 | 98.7 | 99.2 | A |
| **14** | 364.3 | 2.45 | 93.0 | 99.6 | A |
| **15** | 350.1 | 2.41 | 98.6 | 99.8 | A |
| **16** | 313.8 | 2.33 | 96.9 | 96.1 | A |
| **17** | 373.9 | 2.45 | 94.6 | 99.5 | A |
| **18** | 319.9 | 2.29 | 95.7 | 99.1 | A |
| **19** | 315.8 | 2.12 | 91.4 | 98.0 | A |
| **20** | 336.1 | 2.34 | 96.3 | 96.3 | A |
| **21** | 320.0 | 2.18 | 95.0 | 97.7 | A |
| **22** | 298.2 | 2.03 | 96.8 | 98.7 | B |
| **23** | 315.0 | 2.08 | 92.1 | 99.4 | A |
| **24** | 302.1 | 2.14 | 95.3 | 98.4 | B |
| **25** | 331.9 | 2.10 | 97.0 | 95.7 | B |
| **26** | 285.9 | 2.07 | 99.5 | 97.4 | B |
| **27** | 345.9 | 2.10 | 98.2 | 98.2 | B |
| **28** | 345.8 | 2.22 | 99.5 | 96.4 | B |
| **29** | 282.1 | 2.12 | 97.6 | 96.9 | B |
| **30** | 316.0 | 2.33 | 99.6 | 96.6 | B |
| **31** | 336.2 | 2.27 | 99.9 | 98.6 | B |
| **32** | 336.1 | 2.28 | 98.7 | 96.4 | B |
| **33** | 296.1 | 2.25 | 97.1 | 98.4 | B |
| **34** | 296.0 | 2.24 | 98.8 | 99.4 | B |
| **35** | 312.2 | 2.17 | 98.9 | 98.7 | B |
| **36** | 319.7 | 2.14 | 98.7 | 97.8 | B |
| **37** | 312.2 | 2.12 | 99.2 | 96.1 | B |
| **38** | 316.0 | 2.05 | 98.8 | 98.5 | B |
| **39** | | 2.33 | 96.9 | 100 | B |
| **40** | | 2.08 | 89.7 | 100 | B |
| **41** | 304.0 | 2.04 | 93.9 | 100 | B |
| **42** | 282.2 | 2.15 | 97.1 | 100 | B |
| **43** | 316.1 | 2.10 | 99.0 | 99.5 | B |
| **44** | 364.0 | 2.19 | 99.3 | 100 | B |
| **45** | 338.1 | 2.14 | 95.9 | 100 | B |
| **46** | 354.1 | 2.30 | 95.3 | 100 | B |
| **47** | 302.1 | 2.15 | 98.8 | 100 | B |
| **48** | 298.2 | 2.01 | 98.9 | 100 | B |
| **49** | 350.0 | 2.45 | 99.0 | 100 | B |
| **50** | 334.1 | 2.33 | 98.9 | 100 | B |
| **51** | 330.1 | 2.68 | 98.8 | 100 | B |
| **52** | 314.1 | 2.26 | 96.1 | 100 | B |
| **53** | 318.1 | 2.18 | 99.1 | 100 | B. |
| **54** | 334.1 | 2.33 | 98.0 | 99.3 | B |
| **55** | 378.0 | 2.31 | 97.0 | 100 | B |
| **56** | 374.1 | 2.38 | 97.5 | 100 | B |
| **57** | 360.1 | 2.29 | 97.5 | 100 | B |
| **58** | 360.1 | 2.36 | 99.1 | 100 | B |
| **59** | 296.0 | 2.25 | 98.3 | 100 | B |
| **60** | 326.0 | 2.29 | 96.8 | 99.4 | B |
| **61** | 330.0 | 2.2 | 97.2 | 100 | B |
| **62** | 349.9 | 2.47 | 99.0 | 99.3 | B |
| **63** | 330.0 | 2.42 | 96.3 | 97.4 | B |
| **64** | 341.0 | 2.09 | 96.6 | 98.7 | B |
| **65** | 336.0 | 2.70 | 98.5 | 99.5 | B |
| **66** | 364.0 | 2.28 | 97.6 | 99.7 | B |
| **67** | 360.0 | 2.38 | 98.4 | 99.9 | B |
| **68** | 304.0 | 2.40 | 97.3 | 100 | B |
| **69** | 359.9 | 2.24 | 98.0 | 96.6 | B |
| **70** | 332.0 | 2.53 | 97.8 | 100 | B |
| **71** | 334.0 | 2.09 | 90.0 | 98.0 | B |
| **72** | 350.0 | 2.22 | 77.0 | 100 | B |
| **73** | 318.0 | 2.22 | 91.0 | 100 | B |
| **74** | 334.0 | 2.68 | 98.0 | 100 | B |
| **75** | 337.9 | 2.19 | 100 | 100 | B |
| **76** | 330.0 | 2.19 | 98.0 | 100 | B |
| **77** | 310.1 | 2.38 | 97.7 | 97.6 | B |
| **78** | 296.0 | 2.32 | 97.7 | 99.3 | B |
| **79** | 300.0 | 2.15 | 98.1 | 98.6 | B |
| **80** | 316.0 | 2.30 | 97.2 | 100 | B |
| **81** | 310.0 | 2.38 | 98.7 | 99.7 | B |
| **82** | 346.0 | 2.34 | 97.2 | 98.2 | B |
| **83** | 304.0 | 2.07 | 96.4 | 99.6 | B |
| **84** | 316.0 | 2.24 | 95.6 | 99.6 | B |
| **85** | 318.0 | 2.27 | 99.0 | 99.8 | B |
| **86** | 334.0 | 2.34 | 98.0 | 99.5 | B |
| **87** | 300.0 | 2.14 | 97.2 | 99.8 | B |
| **88** | 316.0 | 2.29 | 97.9 | 99.4 | B |
| **89** | 314.1 | 2.28 | 97.3 | 99.5 | B |
| **90** | 300.1 | 2.21 | 98.8 | 99.7 | B |
| **91** | 316.0 | 2.28 | 98.6 | 99.7 | B |
| **92** | 360.0 | 2.33 | 99.1 | 98.8 | B |
| **93** | 312.1 | 2.16 | 96.6 | 98.1 | B |
| **94** | 350.0 | 2.40 | 97.3 | 99.5 | B |
| **95** | 312.1 | 2.09 | 97.1 | 99.7 | B |
| **96** | 296.0 | 2.12 | 97.1 | 70.8 | B |
| **97** | 296.1 | 2.19 | 100 | 73.0 | B |
| **98** | 324.1 | 2.34 | 100 | 83.5 | B |
| **99** | 324.2 | 2.38 | 100 | 92.0 | B |
| **100** | 286.1 | 1.94 | 99.6 | 90.5 | B |
| **101** | 336.2 | 2.08 | 99.6 | 80.3 | B |
| **102** | 336.1 | 2.14 | 100 | 89.8 | B |
| **103** | 352.1 | 2.23 | 99.3 | 86.1 | B |
| **104** | 313.9 | 2.07 | 99.0 | 83.3 | B |
| **105** | 296.2 | 2.15 | 99.6 | 86.1 | B |
| **106** | 296.1 | 2.14 | 100 | 83.0 | B |
| **107** | 336.1 | 2.15 | 99.9 | 75.8 | B |
| **108** | 336.0 | 2.23 | 99.4 | 74.3 | B |
| **109** | 319.9 | 2.11 | 99.5 | 91.7 | B |
| **110** | 310.3 | 2.27 | 99.9 | 81.7 | B |
| **111** | 311.3 | 1.72 | 99.5 | 83.6 | B |
| **112** | 316.0 | 2.05 | 99.0 | 94.9 | B |
| **113** | 299.8 | 2.16 | 97.8 | 98.8 | B |
| **114** | 300.0 | 2.16 | 95.1 | 97.8 | B |
| **115** | 326.1 | 2.08 | 95.1 | 95.6 | B |
| **116** | 346.0 | 2.26 | 93.7 | 98.1 | B |
| **117** | 350.0 | 2.39 | 94.7 | 99.0 | B |
| **118** | 330.0 | 2.41 | 98.2 | 99.7 | B |
| **119** | 312.2 | 1.96 | 97.0 | 99.0 | B |
| **120** | 304.0 | 2.01 | 97.0 | 99.0 | B |
| **121** | 300.0 | 2.21 | 83.5 | 93.5 | B |
| **122** | 316.0 | 2.02 | 93.0 | 99.0 | B |
| **123** | 319.9 | 2.20 | 97.3 | 92.3 | B |
| **124** | 320.0 | 3.32 | 80.4 | 94.2 | B |
| **125** | 299.9 | 2.21 | 99.9 | 93.7 | B |
| **126** | 384.0 | 3.03 | 77.4 | 91.7 | B |
| **127** | 330.1 | 2.17 | 93.2 | 100 | B |
| **128** | 313.9 | 2.31 | 84.4 | 100 | B |
| **129** | 316.0 | 2.12 | 96.4 | 97.1 | B |

### Measurements of [³H]-5-HT uptake into rat cortical synaptosomes.

Whole brains from male Wistar rats (125-225 g), excluding cerebellum, are homogenized in 0.32 M sucrose supplemented with 1mM nialamid with a glass/teflon homogenizer. The homogenate is centrifuged at 600 x g for 10 min at 4 °C. The pellet is discarded and the supernatant is centrifuged at 20.000 x g for 55 min. The final pellet is homogenized (20 sec) in this assay buffer (0.5 mg original tissue/well). Test compounds (or buffer) and 10 nM [³H]-5-HT are added to 96 well plates and shaken briefly. Composition of assay buffer: 123 mM NaCl, 4.82 mM KCl, 0.973 mM CaCl₂, 1.12 mM MgSO₄, 12.66 mM Na₂HPO₄, 2.97 mM NaH₂PO₄, 0.162 mM EDTA, 10 mM glucose and 1 mM ascorbic acid. Buffer is oxygenated with 95% 0₂/5% CO₂ for 10 min at 37 °C and pH is adjusted 7.4. The incubation is started by adding tissue to a final assay volume of 0.2 mL. After 15 min incubation with radioligand at 37 °C, samples are filtered directly on Unifilter GF/C glass fiber filters (soaked for 1 hour in 0.1% polyethylenimine) under vacuum and immediately washed with 3 x 0.2 ml assay buffer. Non-specific uptake is determined using citalopram (10 µM final concentration). Citalopram is included as reference in all experiments as dose-response curve.

Preferred compounds of the present invention exhibit serotonin reuptake inhibition below 200 nM (IC₅₀) in the assay above. More preferred are the compounds which exhibit inhibition below 100 nM and most preferably below 50 nM.

### [³H]Mesulergine binding to 5-HT_{2C} receptors.

Cell lines expressing 10-20 pmol/mg protein human 5-HT_{2C}-vsv receptors (Euroscreen) were harvested in ice-cold 50 mM Tris pH 7.7buffer containing 125 mM NaCl and stored at -80 °C. On the day of the experiment cells were quickly thawed and homogenized in 50 mM Tris pH 7.7 using an Ultra-Thurax. Aliqouts consisting of 6-30 µg protein, [³H]Mesulergine (1 nM) and testsubstance were incubated for 30 min at 37° C. Total binding was determined using assay buffer (50 mM Tris pH 7.7) and non-specific binding was defined in the presence of 100 µM 5-HT. Bound and free [³H]Mesulergine was separated by vacuum filtration on GF/B filters (pre-soaked in 0.1 % PEI for ½ hour) and counted in a scintillation counter.

### 5-HT_{2C} receptor efficacy as determined by fluorometry.

This assay was carried out as described by Porter et al. British Journal of Pharmacology 1999, 128, 13 with the modifications described below. 2 days before the experiment CHO cells expressing 10-20 pmol/mg protein human 5-HT_{2C-VSV} receptors (Euroscreen) were plated at a density sufficient to yield a mono-confluent layer on the day of the experiment. The cells were dye loaded (Ca²⁺-kit from Molecular Devices, and according to their instructions) at 37 °C in a 5% CO₂ incubator at 95% humidity. Lazer intensity was set to a suitable level to obtain basal values of approximately 8000 RFUs. The variation in basal fluorescence was less than 10%. EC₅₀ values were assessed using increasing concentrations of test compound covering 3 decades. IC₅₀ values were assessed challenging the EC₈₅ of 5-HT with concentrations covering 3 decades of test substances. Ki values were calculated using Cheng-Prusoff equation.

### Measurements of [³H]noradrenaline uptake into rat cortical synaptosomes.

Fresh cortex from male Wistar rats (125-225 g) are homogenized in 0.4M sucrose with a glass/teflon homogenizer. The homogenate is centrifuged at 600 x g for 10 min at 4 °C. The pellet is discarded and the supernatant is centrifuged at 20.000 x g for 55 min. The final pellet is homogenized (20 sec) in this assay buffer (6 mg original tissue/mL = 4 mg/well). Test compounds (or buffer) and 10 nM [³H]-noradrenaline are added to deep 96 well plates and shaken briefly. Composition of assay buffer: 123 mM NaCl, 4.82 mM KCl, 0.973 mM CaCl₂, 1.12 mM MgSO₄, 12.66 mM Na₂HPO₄, 2.97 mM NaH₂PO₄, 0.162 mM EDTA, 10 mM glucose and 1 mM ascorbic acid. Buffer is oxygenated with 95% 0₂/5% CO₂ for 10 min at 37 °C and pH is adjusted 7.4. The incubation is started by adding tissue to a final assay volume of 1 ml. After 15 min incubation with radioligand at 37 °C, samples are filtered directly on Unifilter GF/C glass fiber filters (soaked for 1 hour in 0.1% polyethylenimine) under vacuum and immediately washed with 3 x 1 mL assay buffer. Non-specific uptake is determined using talsupram (10 µM final concentration). Duloxetine is included as reference in all experiments as dose-response curve.

## Claims

1. A compound represented by the general formula I 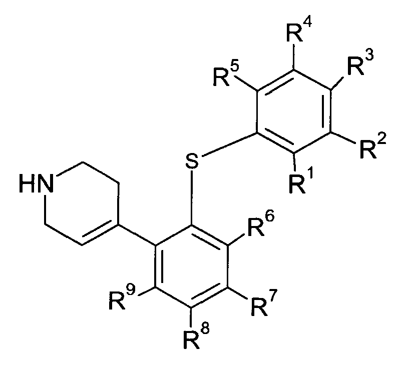 wherein
R¹, R², R³, R⁴, R⁵ are independently selected from hydrogen, halogen, cyano, C₁₋₆-alk(en/yn)yl, C₁₋₆-alk(en/yn)yloxy, C₁₋₆-alk(en/yn)ylsulfanyl, hydroxy, hydroxy-C₁₋₆-alk(en/yn)yl, halo-C₁₋₆-alk(en/yn)yl, halo-C₁₋₆-alk(en/yn)yloxy, or NR^{x}R^{y} wherein R^{x} and R^{y} are independently selected from hydrogen, C₁₋₆-alk(en/yn)yl, C₃₋₈-cycloalk(en)yl, C₃₋₈-cycloalk(en)yl-C₁₋₆-alk(en/yn)yl, or NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl; or R^{x} and R^{y} together with the nitrogen to which they are attached form a 3-7-membered ring which optionally contains one further heteroatom; or
R² and R³ together form a heterocycle fused to the phenyl ring selected from 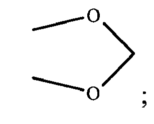 and R¹, R⁴, R⁵ are as defined above;
R⁶, R⁷, R⁸, R⁹ are independently selected from hydrogen, halogen, C₁₋₆-alk(en/yn)yl, C₁₋₆-alk(en/yn)yloxy, C₁₋₆-alk(en/yn)ylsulfanyl, hydroxy, hydroxy-C₁₋₆-alk(en/yn)yl, halo-C₁₋₆-alk(en/yn)yl, halo-C₁₋₆-alk(en/yn)yloxy, or NR^{x}R^{y} wherein R^{x} and R^{y} are independently selected from hydrogen, C₁₋₆-alk(en/yn)yl, C₃₋₈-cycloalk(en)yl, C₃₋₈-cycloalk(en)yl-C₁₋₆-alk(en/yn)yl, or NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl; or R^{x} and R^{y} together with the nitrogen to which they are attached form a 3-7-membered ring which optionally contains one further heteroatom;
provided that at least one of R¹, R², R³, R⁴, R⁵, R⁶, R⁷, R⁸, and R⁹ is different from hydrogen; also provided that when R³ is methyl or methoxy, then at least one of R¹, R², R⁴, R⁵, R⁶, R⁷, R⁸, R⁹ is different from hydrogen;
or a salt thereof.

2. The compound of claim 1, wherein R¹ is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl, or NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃₋₈-cycloalk(en)yl-C₁₋₆-alk(en/yn)yl, or NR^{z}R^{w}-C₁₋₆-alk(enlyn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl, provided that if one of R^{x} and R^{y} is NR^{z}R^{w}-C₁₋₆-alk(en/yn)yl then the other is selected from hydrogen, C₁₋₆-alk(en/yn)yl, cyano-C₁₋₆-alk(en/yn)yl, C₃₋₈-cycloalk(en)yl, or C₃₋₈-cycloalk(en)yl-C₁₋₆-alk(en/yn)yl; or R^{x} and R^{y} together with the nitrogen to which they are attached form a 3-7-membered ring which optionally contains one further heteroatom; typically, R¹ is selected from hydrogen, C₁₋₆-alkyl, halogen, C₁₋₆-alkyloxy, or halo-C₁₋₆-alkyl; more typically, R¹ is selected from hydrogen, C₁₋₆-alkyl, or halogen.

3. The compound of any one of claims 1-2, wherein R² is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl; typically, R² is selected from hydrogen, C₁₋₆-alkoxy, halogen, C₁₋₆-alkyl, or halo-C₁₋₆-alkyl; more typically, R² is selected from hydrogen, or C₁₋₆-alkoxy.

4. The compound of any one of claims 1-3, wherein R³ is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl; typically, R³ is selected from hydrogen, C₁₋₆-alkyl, C₁₋₆-alkoxy, halogen, C₁₋₆-alkylsulfanyl, halo-C₁₋₆-alkyl, or halo-C₁₋₆-alkyloxy; more typically, R³ is selected from hydrogen, C₁₋₆-alkyl, C₁₋₆-alkoxy, or halogen.

5. The compound of any one of claims 1-2, wherein R² and R³ together form a heterocycle fused to the phenyl ring selected from

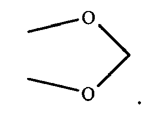

6. The compound of any one of claims 1-5 wherein R⁴ is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl; typically, R⁴ is selected from hydrogen, C₁₋₆-alkoxy, halogen, C₁₋₆-alkyl, or halo-C₁₋₆-alkyl; more typically, R⁴ is selected from hydrogen, or C₁₋₆-alkoxy.

7. The compound of any one of claims 1-6 wherein R⁵ is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl, or NR^{x}R^{y} wherein R^{x} and R^{y} are independently selected from hydrogen, C₁₋₆-alk(enlyn)yl, cyano-C₁₋₆-alk(en/yn)yl, C₃₋₈-cycloalk(en)yl, C₃₋₈-cycloalk(en)yl-C₁₋₆-alk(en/yn)yl, or NR^{z}R^{w}-C₁₋₆-alk(enlyn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl, provided that if one of R^{x} and R^{y} is NR^{z}R^{w}-C₁₋₆-alk(en/yn)yl then the other is selected from hydrogen, C₁₋₆-alk(en/yn)yl, cyano-C₁₋₆-alk(en/yn)yl, C₃₋₈-cycloalk(en)yl, or C₃₋₈-cycloalk(en)yl-C₁₋₆-alk(en/yn)yl; or R^{x} and R^{y} together with the nitrogen to which they are attached form a 3-7-membered ring which optionally contains one further heteroatom; typically, R⁵ is selected from hydrogen, C₁₋₆-alkyl, halogen, C₁₋₆-alkyloxy, or halo-C₁₋₆-alkyl; more typically, R⁵ is selected from hydrogen, C₁₋₆-alkyl, or halogen.

8. The compound of any one of claims 1-7 wherein R⁶ is selected from hydrogen, halogen, C₁₋₆-alk(en/yn)yl, halo-C₁₋₆-alk(enlyn)yl; typically, R⁶ is selected from hydrogen, or halogen.

9. The compound of any one of claims 1-8 wherein R⁷ is selected from hydrogen, halogen, C₁₋₆-alk(en/yn)yl, halo-C₁₋₆-alk(en/yn)yl; typically, R⁷ is selected from hydrogen, or halogen.

10. The compound of any one of claims 1-9 wherein R⁸ is selected from hydrogen, halogen, C₁₋₆-alk(en/yn)yl, halo-C₁₋₆-alk(en/yn)yl, or NR^{x}R^{y} wherein R^{x} and R^{y} are independently selected from hydrogen, C₁₋₆-alk(en/yn)yl, cyano-C₁₋₆-alk(en/yn)yl, C₃-8-cycloalk(en)yl, C₃₋₈-cycloalk(en)yl-C₁₋₆-alk(en/yn)yl, or NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl, provided that if one of R^{x} and R^{y} is NR^{z}R^{w}-C₁₋₆-alk(en/yn)yl then the other is selected from hydrogen, C₁₋₆-alk(en/yn)yl, cyano-C₁₋₆-alk(en/yn)yl, C₃₋₈-cycloalk(en)yl, or C₃₋₈-cycloalk(en)yl-C₁₋₆-alk(en/yn)yl; or R^{x} and R^{y} together with the nitrogen to which they are attached form a 3-7-membered ring which optionally contains one further heteroatom; typically, R⁸ is selected from hydrogen, C₁₋₆-alkyl, halogen, halo-C₁₋₆-alkyl, or NR^{x}R^{y} wherein R^{x} is hydrogen and R^{y} is C₁₋₆-alkyl; more typically, R⁸ is selected from hydrogen, C₁₋₆-alkyl, halogen, or halo-C₁₋₆-alkyl.

11. The compound of any one of claims 1-10 wherein R⁹ is selected from hydrogen, halogen, C₁₋₆-alk(en/yn)yl, halo-C₁₋₆-alk(en/yn)yl; typically, R⁹ is selected from hydrogen.

12. The compound of any one of claims 1-11 wherein the compound of formula I has 1-4 substituents in the phenyl ring(s), selected from any one of R¹-R⁹, which are different from hydrogen, and the remaining substituents are hydrogen.

13. The compound of claim 1, said compound being selected from
4-[2-(4-Fluorophenylsulfanyl)-phenyl]-1,2,3,6-tetrahydropyridine,
4-[2-(4-Chlorophenylsulfanyl)-phenyl]-1,2,3,6-tetrahydropyridine,
4-[2-(4-Methoxyphenylsulfanyl)-5-methyl-phenyl]-1,2,3,6-tetrahydropyridine,
4-(5-Methyl-2-p-tolylsulfanylphenyl)-1,2,3,6-tetrahydropyridine,
4-[2-(4-Fluorophenylsulfanyl)-5-methyl-phenyl]-1,2,3,6-tetrahydropyridine,
4-[2-(4-Chlorophenylsulfanyl)-5-methyl-phenyl]-1,2,3,6-tetrahydropyridine,
4-[2-(2,4-Dimethylphenylsulfanyl)-phenyl]-1,2,3,6-tetrahydropyridine,
4-[2-(4-Chlorophenylsulfanyl)-5-trifluoromethyl-phenyl]-1,2,3,6-tetrahydropyridine,
4-[2-(4-Fluoro-2-methylphenylsulfanyl)-phenyl]-1,2,3,6-tetrahydropyridine,
4-[2-(4-Chlorophenylsulfanyl)-4-fluoro-phenyl]-1,2,3,6-tetrahydropyridine,
4-[4-Fluoro-2-(p-tolylsulfanyl)-phenyl]-1,2,3,6-tetrahydropyridine,
4-[4-Fluoro-2-(4-methoxyphenylsulfanyl)-phenyl] -1,2,3,6-tetrahydropyridine,
4-[2-(2,4-Dimethylphenylsulfanyl)-5-methyl-phenyl]-1,2,3,6-tetrahydropyridine,
4-[2-(2,4-Dimethylphenylsulfanyl)-5-trifluoromethyl-phenyl]-1,2,3,6-tetrahydropyridine,
4-(2-p-Tolylsulfanyl-5-trifluoromethylphenyl)-1,2,3,6-tetrahydropyridine,
4-[2-(4-Fluoro-2-methylphenylsulfanyl)-5-methyl-phenyl]-1,2,3,6-tetrahydropyridine,
4-[5-Bromo-2-(2,4-dimethylphenylsulfanyl)-phenyl]-1,2,3,6-tetrahydropyridine,
4-[2-(4-Chlorophenylsulfanyl)-5-fluoro-phenyl]-1,2,3,6-tetrahydropyridine,
4-[5-Fluoro-2-(4-methoxyphenylsulfanyl)-phenyl]-1,2,3,6-tetrahydropyridine,
4-[2-(2,4-Dichlorophenylsulfanyl)-phenyl]-1,2,3,6-tetrahydropyridine,
4-[2-(4-Chloro-2-fluoro-phenylsulfanyl)-phenyl]-1,2,3,6-tetrahydropyridine,
4-[2-(3-Methoxyphenylsulfanyl)-phenyl]-1,2,3,6-tetrahydropyridine,
4-[3-Fluoro-2-(4-methoxyphenylsulfanyl)-phenyl]-1,2,3,6-tetrahydropyridine,
4-[2-(2-Chlorophenylsulfanyl)-phenyl]-1,2,3,6-tetrahydropyridine,
4-[2-(2-Chloro-4-methoxyphenylsulfanyl)-phenyl]-1,2,3,6-tetrahydropyridine,
4-[2-(2-Fluorophenylsulfanyl)-phenyl]-1,2,3,6-tetrahydropyridine,
4-[2-(2-Bromophenylsulfanyl)-phenyl]-1,2,3,6-tetrahydropyridine,
4-[2-(4-Bromophenylsulfanyl)-phenyl]-1,2, 3, 6-tetrahydropyridine,
4-(2-o-Tolylsulfanylphenyl)-1,2, 3,6-tetrahydropyridine,
4-[2-(4-Chloro-2-methylphenylsulfanyl)-phenyl]-1,2,3, 6-tetrahydropyridine,
4-[2-(4-Trifluoromethyl-phenylsulfanyl)-phenyl]-1,2, 3,6-tetrahydro-pyridine,
4-[2-(2,3-Dichloro-phenylsulfanyl)-phenyl]-1,2,3,6-tetrahydro-pyridine,
4-[2-(2,3-Dimethyl-phenylsulfanyl)-phenyl]-1,2,3,6-tetrahydro-pyridine,
4-[2-(3,4-Dimethyl-phenylsulfanyl)-phenyl]-1,2,3,6-tetrahydro-pyridine,
4-[2-(2-Methoxy-5-methyl-phenylsulfanyl)-phenyl]-1,2,3,6-tetrahydro-pyridine,
4-[2-(2-Chloro-4-fluoro-phenylsulfanyl)-phenyl]-1,2,3,6-tetrahydro-pyridine,
4-[2-(4-Methoxy-2-methyl-phenylsulfanyl)-phenyl]-1,2,3,6-tetrahydro-pyridine,
4-[2-(2-Fluoro-4-methoxy-phenylsulfanyl)-phenyl]-1,2,3,6-tetrahydro-pyridine,
4-[2-(2,4-Dimethyl-phenylsulfanyl)-5-fluoro-phenyl]-1,2,3,6-tetrahydro-pyridine,
4-(5-Fluoro-2-phenylsulfanyl-phenyl)-1,2,3,6-tetrahydro-pyridine,
4-[5-Fluoro-2-(4-fluoro-phenylsulfanyl)-phenyl]-1,2,3,6-tetrahydro-pyridine,
4-(2-m-Tolylsulfanyl-phenyl)-1,2,3,6-tetrahydro-pyridine,
4-[2-(4-Fluoro-3-methoxy-phenylsulfanyl)-phenyl]-1,2,3,6-tetrahydro-pyridine,
4-[2-(2-Bromo-4-fluoro-phenylsulfanyl)-phenyl]-1,2,3,6-tetrahydro-pyridine,
4-[2-(2-Chloro-4-fluoro-phenylsulfanyl)-5-fluoro-phenyl]-1,2,3,6-tetrahydro-pyridine,
4-[2-(2,4-Dichloro-phenylsulfanyl)-5-fluoro-phenyl]-1,2,3,6-tetrahydro-pyridine,
4-[2-(3-Chloro-phenylsulfanyl)-phenyl]-1,2,3,6-tetrahydro-pyridine,
4-[2-(2-Methoxy-phenylsulfanyl)-phenyl]-1,2,3,6-tetrahydro-pyridine,
4-[2-(2,4-Dichloro-phenylsulfanyl)-5-methyl-phenyl]-1,2,3,6-tetrahydro-pyridine,
4-[2-(4-Chloro-2-fluoro-phenylsulfanyl)-5-methyl-phenyl]-1,2,3,6-tetrahydro-pyridine,
4-[2-(4-Chloro-2-methyl-phenylsulfanyl)-5-methyl-phenyl]-1,2,3,6-tetrahydro-pyridine,
4-[2-(2-Fluoro-4-methyl-phenylsulfanyl)-5-methyl-phenyl]-1,2,3,6-tetrahydro-pyridine,
4-[2-(2,4-Difluoro-phenylsulfanyl)-5-methyl-phenyl]-1,2,3,6-tetrahydro-pyridine,
4-[2-(2-Chloro-4-fluoro-phenylsulfanyl)-5-methyl-phenyl]-1,2,3,6-tetrahydro-pyridine,
4-[2-(2-Bromo-4-fluoro-phenylsulfanyl)-5-methyl-phenyl]-1,2,3,6-tetrahydro-pyridine,
4-[2-(2-Bromo-4-methyl-phenylsulfanyl)-5-methyl-phenyl]-1,2,3,6-tetrahydro-pyridine,
4-[2-(2-Bromo-phenylsulfanyl)-5-methyl-phenyl]-1,2,3,6-tetrahydro-pyridine,
4-[2-(4-Bromo-phenylsulfanyl)-5-methyl-phenyl]-1,2,3,6-tetrahydro-pyridine,
4-(5-Methyl-2-o-tolylsulfanyl-phenyl)-1,2,3,6-tetrahydro-pyridine,
4-[2-(4-Methoxy-2-methyl-phenylsulfanyl)-5-methyl-phenyl]-1,2,3,6-tetrahydro-pyridine,
4-[2-(2-Fluoro-4-methoxy-phenylsulfanyl)-5-methyl-phenyl]-1,2,3,6-tetrahydro-pyridine,
4-[2-(3,4-Dichloro-phenylsulfanyl)-5-methyl-phenyl]-1,2,3,6-tetrahydro-pyridine,
4-[2-(2-Chloro-4-methyl-phenylsulfanyl)-5-methyl-phenyl]-1,2,3,6-tetrahydro-pyridine,
4-[2-(2-Fluoro-4-methyl-phenylsulfanyl)-phenyl]-1,2,3,6-tetrahydro-pyridine,
4-[2-(3,4-Dichloro-phenylsulfanyl)-phenyl]-1,2,3,6-tetrahydro-pyridine,
4-[2-(4-Bromo-2-fluoro-phenylsulfanyl)-phenyl]-1,2,3,6-tetrahydro-pyridine,
4-[2-(4-Bromo-2-methyl-phenylsulfanyl)-phenyl]-1,2,3,6-tetrahydro-pyridine,
4-[2-(2,4-Difluoro-phenylsulfanyl)-phenyl]-1,2,3,6-tetrahydro-pyridine,
4-[2-(2-Bromo-4-methyl-phenylsulfanyl)-phenyl]-1,2,3,6-tetrahydro-pyridine,
4-[2-(4-Chloro-2-methoxy-phenylsulfanyl)-phenyl]-1,2,3,6-tetrahydro-pyridine,
4-[5-Fluoro-2-(2-fluoro-4-methoxy-phenylsulfanyl)-phenyl]-1,2,3,6-tetrahydro-pyridine,
4-[2-(2-Chloro-4-methoxy-phenylsulfanyl)-5-fluoro-phenyl]-1,2,3,6-tetrahydro-pyridine,
4-[5-Fluoro-2-(2-fluoro-4-methyl-phenylsulfanyl)-phenyl]-1,2,3,6-tetrahydro-pyridine,
4-[2-(2-Chloro-4-methyl-phenylsulfanyl)-5-fluoro-phenyl]-1,2,3,6-tetrahydro-pyridine,
4-[2-(4-Chloro-2-fluoro-phenylsulfanyl)-5-fluoro-phenyl]-1,2,3,6-tetrahydro-pyridine,
4-[5-Fluoro-2-(4-methoxy-2-methyl-phenylsulfanyl)-phenyl]-1,2,3,6-tetrahydro-pyridine,
4-[2-(2,3-Dimethyl-phenylsulfanyl)-5-methyl-phenyl]-1,2,3,6-tetrahydro-pyridine,
4-[5-Methyl-2-(3-methyl-phenylsulfanyl)-phenyl]-1,2,3,6-tetrahydro-pyridine,
4-[2-(2-Fluoro-phenylsulfanyl)-5-methyl-phenyl]-1,2,3,6-tetrahydro-pyridine,
4-[2-(2-Chloro-phenylsulfanyl)-5-methyl-phenyl]-1,2,3,6-tetrahydro-pyridine,
4-[2-(3,4-Dimethyl-phenylsulfanyl)-5-methyl-phenyl]-1,2,3,6-tetrahydro-pyridine,
4-[2-(4-Chloro-2-methoxy-phenylsulfanyl)-5-methyl-phenyl]-1,2,3,6-tetrahydro-pyridine,
4-[2-(2,3-Difluoro-phenylsulfanyl)-phenyl]-1,2,3,6-tetrahydro-pyridine,
4-[2-(2-Chloro-4-methyl-phenylsulfanyl)-phenyl]-1,2,3,6-tetrahydro-pyridine,
4-[2-(2,3-Difluoro-phenylsulfanyl)-5-methyl-phenyl]-1,2,3,6-tetrahydro-pyridine,
4-[2-(3-Chloro-2-fluoro-phenylsulfanyl)-5-methyl-phenyl]-1,2,3,6-tetrahydro-pyridine,
4-[2-(3-Fluoro-2-methyl-phenylsulfanyl)-phenyl]-1,2,3,6-tetrahydro-pyridine,
4-[2-(3-Chloro-2-methyl-phenylsulfanyl)-phenyl]-1,2,3,6-tetrahydro-pyridine,
4-[2-(3-Fluoro-2-methyl-phenylsulfanyl)-5-methyl-phenyl]-1,2,3,6-tetrahydro-pyridine,
4-[2-(3-Fluoro-phenylsulfanyl)-5-methyl-phenyl]-1,2,3,6-tetrahydro-pyridine,
4-[2-(3-Chloro-phenylsulfanyl)-5-methyl-phenyl]-1,2,3,6-tetrahydro-pyridine,
4-[2-(3-Bromo-phenylsulfanyl)-5-methyl-phenyl]-1,2,3,6-tetrahydro-pyridine,
4-[2-(3-Methoxy-phenylsulfanyl)-5-methyl-phenyl]-1,2,3,6-tetrahydro-pyridine,
4-[5-Methyl-2-(3-trifluoromethyl-phenylsulfanyl)-phenyl]-1,2,3,6-tetrahydro-pyridine,
4-[2-(2-Methoxy-phenylsulfanyl)-5-methyl-phenyl]-1,2,3,6-tetrahydro-pyridine,
4-[2-(2-Ethyl-phenylsulfanyl)-phenyl]-1,2,3,6-tetrahydro-pyridine,
4-[2-(4-Ethyl-phenylsulfanyl)-phenyl]-1,2,3, 6-tetrahydro-pyridine,
4-[2-(2-tert-Butyl-phenylsulfanyl)-phenyl]-1,2,3,6-tetrahydro-pyridine,
4-[2-(4-tert-Butyl-phenylsulfanyl)-phenyl]-1, 2, 3, 6-tetrahydro-pyridine,
4-[2-(3-Fluoro-phenylsulfanyl)-phenyl]-1,2,3,6-tetrahydro-pyridine,
4-[2-(2-Trifluoromethyl-phenylsulfanyl)-phenyl]-1,2,3,6-tetrahydro-pyridine,
4-[2-(3-Trifluoromethyl-phenylsulfanyl)-phenyl]-1,2,3,6-tetrahydro-pyridine,
4-[2-(4-Trifluoromethoxy-phenylsulfanyl)-phenyl]-1,2,3,6-tetrahydro-pyridine,
4-[2-(4-Methylsulfanyl-phenylsulfanyl)-phenyl]- 1,2,3,6-tetrahydro-pyridine,
4-[2-(3,5-Dimethyl-phenylsulfanyl)-phenyl]-1,2,3,6-tetrahydro-pyridine,
4-[2-(2,5-Dimethyl-phenylsulfanyl)-phenyl]-1,2,3,6-tetrahydro-pyridine,
4-[2-(2,5-Dichloro-phenylsulfanyl)-phenyl]-1,2,3,6-tetrahydro-pyridine,
4-[2-(3,5-Dichloro-phenylsulfanyl)-phenyl]-1,2,3,6-tetrahydro-pyridine,
4-[2-(3-Chloro-4-fluoro-phenylsulfanyl)-phenyl]-1,2,3,6-tetrahydro-pyridine,
4-[2-(2,4,6-Trimethyl-phenylsulfanyl)-phenyl]-1,2,3,6-tetrahydro-pyridine,
4-[5-Methylamino-2-(4-methyl-pheylsulfanyl)-phenyl]-1,2, 3,6-tetrahydro-pyridine,
4-[5-Fluoro-2-(2-methoxy-phenylsulfanyl)-phenyl]-1,2,3,6-tetrahydro-pyridine,
4-(5-Fluoro-2-o-tolylsulfanyl-phenyl)-1,2,3,6-tetrahydro-pyridine,
4-(5-Fluoro-2-p-tolylsulfanyl-phenyl)-1,2,3,6-tetrahydro-pyridine,
4-[2-(Benzo[1,3]dioxol-5-ylsulfanyl)-5-methyl-phenyl]-1,2,3,6-tetrahydro-pyridine,
4-[2-(2-Chloro-4-methoxy-phenylsulfanyl)-5-methyl-phenyl]-1,2,3,6-tetrahydro-pyridine,
4-[2-(2,3-Dichloro-phenylsulfanyl)-5-methyl-phenyl]-1,2,3,6-tetrahydro-pyridine,
4-[2-(3-Chloro-2-methyl-phenylsulfanyl)-5-methyl-phenyl]-1,2,3,6-tetrahydro-pyridine,
4-[2-(Benzo[1,3]dioxol-5-ylsulfanyl)-phenyl]-1,2,3,6-tetrahydro-pyridine,
4-[5-Fluoro-2-(3-fluoro-phenylsulfanyl)-phenyl]-1,2,3,6-tetrahydro-pyridine,
4-(5-Fluoro-2-m-tolylsulfanyl-phenyl)-1,2,3,6-tetrahydro-pyridine,
4-[5-Fluoro-2-(3-methoxy-phenylsulfanyl)-phenyl]-1,2,3,6-tetrahydro-pyridine,
4-[2-(3-Chloro-phenylsulfanyl)-5-fluoro-phenyl]-1,2,3,6-tetrahydro-pyridine,
4-[2-(2-Chloro-phenylsulfanyl)-5-fluoro-phenyl]-1,2,3,6-tetrahydro-pyridine,
4-[4-Fluoro-2-(4-methyl-pheylsulfanyl)-phenyl]- 1,2,3,6-tetrahydro-pyridine,
4-[2-(4-Bromo-2-fluoro-phenylsulfanyl)-5-fluoro-phenyl]-1,2,3,6-tetrahydro-pyridine,
4-[2-(4-Fluoro-3-methoxy-phenylsulfanyl)-5-methyl-phenyl]-1,2,3,6-tetrahydro-pyridine,
4-[2-(3-Fluoro-4-methyl-phenylsulfanyl)-5-methyl-phenyl]-1,2,3,6-tetrahydro-pyridine,
4-[5-Fluoro-2-(4-methoxy-phenylsulfanyl)-phenyl]-1,2,3,6-tetrahydro-pyridine,
or a pharmaceutically acceptable salt thereof.

14. A pharmaceutical composition comprising a compound of any one of claims 1-13 or a pharmaceutically acceptable acid addition salt thereof and at least one pharmaceutically acceptable carrier or diluent.

15. The use of a compound of any one of claims 1 to 13 or a pharmaceutically acceptable acid addition salt thereof for the preparation of a medicament for the treatment of an affective disorder, such as depression, anxiety disorders including general anxiety disorder, social anxiety disorder, post traumatic stress disorder, obsessive compulsive disorder, panic disorder, panic attacks, specific phobias, social phobia and agoraphobia.

16. A compound of any one of claims 1-13 for use as a medicament.

## Patentansprüche

1. Verbindung, dargestellt durch die allgemeine Formel (I): 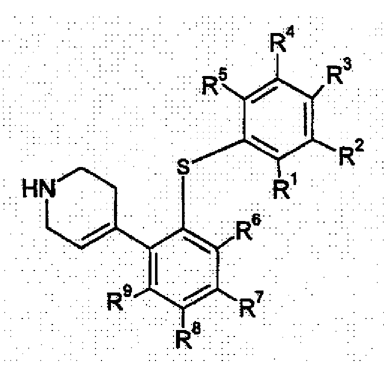 worin
R¹, R², R³, R⁴, R⁵ unabhängig ausgewählt sind aus Wasserstoff, Halogen, Cyano, C₁₋₆-Alk(en/in)yl, C₁₋₆-Alk(en/in)yloxy, C₁₋₆-Alk(en/in)ylsulfanyl, Hydroxy, Hydroxy-C₁₋₆-alk(en/in)yl, Halogen-C₁₋₆-alk(en/in)yl, Halogen-C₁₋₆-alk(en/in)yloxy oder NR^{x}R^{y}, worin R^{x} und R^{y} unabhängig ausgewählt sind aus Wasserstoff, C₁₋₆-Alk(en/in)yl, C₃₋₈-Cycloalk(en/in)yl, C₃₋₈-Cycloalk-(en/in)yl-C₁₋₆-alk(en/in)yl oder NR^{z}R^{w}-C₁₋₆-Alk(en/in)yl, worin R^{z}und R^{w} unabhängig ausgewählt sind aus Wasserstoff, C₁₋₆-Alk(en/in)yl, C₃-₈-Cycloalk(en/in)yl oder C₃₋₈-Cycloalk(enlin)yl-C₁₋₆-alk(en/in)yl; oder R^{x} und RY bilden zusammen mit dem Stickstoffatom, an das sie gebunden sind, einen 3- bis 7-gliedrigen Ring, der gegebenenfalls ein weiteres Heteroatom enthält; oder
R² und R³ bilden zusammen einen Heterocyclus, der an den Phenyl-Ring kondensiert ist, ausgewählt aus 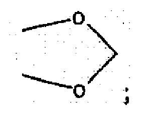 und R¹, R⁴, R⁵ sind wie oben definiert;
R⁶, R⁷, R⁸, R⁹ unabhängig ausgewählt sind aus Wasserstoff, Halogen, C₁₋₆-Alk(en/in)yl, C₁₋₆-Alk(en/in)yloxy, C₁₋₆-Alk(en/in)ylsulfanyl, Hydroxy, Hydroxy-C₁₋₆-alk(en/in)yl, Halogen-C₁₋₆-alk(en/in)yl, Halogen-C₁₋₆-alk(en/in)yloxy oder NR^{x}R^{y}, worin R^{x} und RY unabhängig ausgewählt sind aus Wasserstoff, C₁₋₆-Alk(en/in)yl, Cyano-C₁₋₆-alk(en/in)yl, C₃₋₈-Cycloalk-(en/in)yl, C₃₋₈-Cycloalk(en/in)yl-C₁₋₆-alk(en/in)yl oder NR^{z}R^{w}-C₁₋₆-Alk(en/in)yl, worin R^{z} und R^{w} unabhängig ausgewählt sind aus Wasserstoff, C₁₋₆-Alk(en/in)yl, C₃₋₈-Cycloalk(en/in)yl oder C₃₋₈-Cycloalk(en/in)yl-C₁₋₆-alk(en/in)yl; oder R^{x} und RY bilden zusammen mit dem Stickstoffatom, an das sie gebunden sind, einen 3- bis 7-gliedrigen Ring, der gegebenenfalls ein weiteres Heteroatom enthält;
vorausgesetzt, daß mindestens eines von R¹, R², R³, R⁴, R⁵, R⁶, R⁷, R⁸ und R⁹ kein Wasserstoff ist; ebenfalls vorausgesetzt, daß, wenn R³ Methyl oder Methoxy ist, dann ist mindestens eines von R¹, R², R⁴, R⁵, R⁶, R⁷, R⁸, R⁹ kein Wasserstoff;
oder ein Salz davon.

2. Verbindung gemäß Anspruch 1, worin R¹ ausgewählt ist aus Wasserstoff, Halogen, Cyano, C₁₋₆-Alk(en/in)yl, C₁₋₆-Alk(en/in)yloxy, C₁₋₆-Alk(en/in)ylsulfanyl, Halogen-C₁₋₆-alk(en/in)yl oder NR^{x}R^{y}, worin R^{x} und R^{y} unabhängig ausgewählt sind aus Wasserstoff, C₁₋₆-Alk(en/in)yl, Cyano-C₁₋₆-alk(en/in)yl, C₃₋₈-Cycloalk(en/in)yl, C₃₋₈-Cycloalk(en)yl, C₃₋₈-Cycloalk-(en)yl-C₁₋₆-alk(en/in)yl oder NR^{z}R^{w}-C₁₋₆-alk(enlin)yl, worin R^{z} und R^{w} unabhängig ausgewählt sind aus Wasserstoff, C₁₋₆-Alk(en/in)yl, C₃₋₈-Cycloalk(en)yl oder C₃₋₈-Cycloalk(en)yl-C₁₋₆-alk(en/in)yl, vorausgesetzt, daß wenn eines von R^{x} und RY NR^{z}R^{w}-C₁₋₆-Alk(en/in)yl ist, dann ist das andere ausgewählt aus Wasserstoff, C₁₋₆-Alk(en/in)yl, Cyano-C₁₋₆-alk(en/in)yl, C₃₋₈-Cycloalk(en)yl oder C₃₋₈-Cycloalk(en)yl-C₁₋₆-alk(en/in)yl; oder R^{x} und RY bilden zusammen mit Stickstoffatom, an das sie gebunden sind, einen 3- bis 7-gliedrigen Ring, der gegebenenfalls ein weiteres Heteroatom enthält; typisch ist R¹ ausgewählt aus Wasserstoff, C₁₋₆-Alkyl, Halogen, C₁₋₆-Alkoxy oder Halogen-C₁₋₆-alkyl; typischerweise ist R¹ ausgewählt aus Wasserstoff, C₁₋₆-Alkyl oder Halogen.

3. Verbindung gemäß einem der Ansprüche 1 bis 2, worin R² ausgewählt aus Wasserstoff, Halogen, Cyano, C₁₋₆-Alk(en/in)yl, C₁₋₆-Alk(en/in)yloxy, C₁₋₆-Alk(en/in)ylsulfanyl, Halogen-C₁₋₆-alk(en/in)yl; typisch ist R² ausgewählt aus Wasserstoff, C₁₋₆-Alkoxy, Halogen, C₁₋₆-Alkyl oder Halogen-C₁₋₆-alkyl; typischerweise ist R² ausgewählt aus Wasserstoff, oder C₁₋₆-Alkoxy.

4. Verbindung gemäß einem der Ansprüche 1 bis 3, worin R³ ausgewählt aus Wasserstoff, Halogen, Cyano, C₁₋₆-Alk(en/in)yl, C₁₋₆-Alk(en/in)yloxy, C₁₋₆-Alk(en/in)ylsulfanyl, Halogen-C₁₋₆-alk(en/in)yl, typischer ist R³ ausgewählt aus Wasserstoff, C₁₋₆-Alkyl, C₁₋₆-Alkoxy, Halogen, C₁₋₆-Alkylsulfanyl, Halogen-C₁₋₆-alkyl oder Halogen-C₁₋₆-alkyloxy; typischerweise ist R³ ausgewählt aus Wasserstoff, C₁₋₆-Alkyl, C₁₋₆-Alkoxy oder Halogen.

5. Verbindung gemäß einem der Ansprüche 1 bis 2, worin R² und R³ zusammen einen Heterocyclus bilden, der an den Phenyl-Ring kondensiert ist, ausgewählt aus

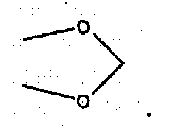

6. Verbindung gemäß einem der Ansprüche 1 bis 5, worin R⁴ ausgewählt ist aus Wasserstoff, Halogen, Cyano, C₁₋₆-Alk(en/in)yl, C₁₋₆-Alk(en/in)yloxy, C₁₋₆-Alk(en/in)ylsulanyl, Halogen-C₁₋₆-alk(en/in)yl; typisch ist R⁴ ausgewählt aus Wasserstoff, C₁₋₆-Alkoxy, Halogen, C₁₋₆-Alkyl oder Halogen-C₁₋₆-alkyl; typischerweise ist R⁴ ausgewählt aus Wasserstoff oder C₁₋₆-Alkoxy.

7. Verbindung gemäß einem der Ansprüche 1 bis 6, worin R⁵ ausgewählt aus Wasserstoff, Halogen, Cyano, C₁₋₆-Alk(en/in)yl, C₁₋₆-Alk(en/in)yloxy, C₁₋₆-Alk(en/in)ylsulfanyl, Halogen-C₁₋₆-alk(en/in)yl oder NR^{x}R^{y}, worin R^{x} und R^{y} unabhängig ausgewählt sind aus Wasserstoff, C₁₋₆-Alk(en/in)yl, Cyano-C₁₋₆-alk(en/in)yl, C₃₋₈-Cycloalkyl(en)yl, C₃₋₈-Cycloalk-(en)yl-C₁₋₆-alk(en/in)yl oder NR^{z}R^{w}-C₁₋₆-Alk(en/in)yl, worin R^{z} und R^{w} unabhängig ausgewählt sind aus Wasserstoff, C₁₋₆-Alk(en/in)yl, C₃₋₈-Cycloalk(en)yl oder C₃₋₈-Cycloalkyl(en)yl-C₁₋₆-alk(en/in)yl, vorausgesetzt, daß wenn eines von R^{x} und RY NR^{z}R^{w}-C₁₋₆-Alk(en/in)yl ist, dann ist das andere ausgewählt aus Wasserstoff, C₁₋₆-Alk(en/in)yl, Cyano-C₁₋₆-alk(en/in)yl, C₃₋₈-Cycloalk(en/in)yl oder C₃₋₈-Cycloalkyl(en)yl-C₁₋₆-alk(en/in)yl; oder R^{x} und R^{y} bilden zusammen mit dem Stickstoffatom, an das sie gebunden sind, einen 3- bis 7-gliedrigen Ring der gegebenenfalls ein weiteres Heteroatom enthält; typischerweise ist R⁵ ausgewählt aus Wasserstoff, C₁₋₆-Alkyl, Halogen, C₁₋₆-Alkyloxy oder Halogen-C₁₋₆-alkyl, bevorzugter ist R⁵ ausgewählt aus Wasserstoff, C₁₋₆-Alkyl oder Halogen.

8. Verbindung gemäß einem der Ansprüche 1 bis 7, worin R⁶ ausgewählt ist aus Wasserstoff, Halogen, C₁₋₆-Alk(en/in)yl, Halogen-C₁₋₆-alk(en/in)yl; typischerweise ist R⁶ ausgewählt aus Wasserstoff oder Halogen.

9. Verbindung gemäß einem der Ansprüche 1 bis 8, worin R⁷ ausgewählt ist aus Wasserstoff, Halogen, C₁₋₆-Alk(en/in)yl, Halogen-C₁₋₆-alk(en/in)yl; typischerweise ist R⁷ ausgewählt aus Wasserstoff oder Halogen.

10. Verbindung gemäß einem der Ansprüche 1 bis 9, worin R⁸ ausgewählt ist aus Wasserstoff, Halogen, C₁₋₆-Alk(en/in)yl, Halogen-C₁₋₆-alk(en/in)yl oder NR^{x}R^{y}, worin R^{x} und RY unabhängig ausgewählt sind aus Wasserstoff, C₁₋₆-Alk(en/in)yl, Cyano-C₁₋₆-alk(en/in)yl, C₃₋₈-Cycloalk(en)yl, C₃₋₈-Cycloalk(en)yl-C₁₋₆-alk(en/in)yl oder NR^{z}R^{w}-C₁₋₆-Alk(en/in)yl, worin R^{z} und R^{w} unabhängig ausgewählt sind aus Wasserstoff, C₁₋₆-Alk(en/in)yl, C₃₋₈-Cycloalk(en)yl oder C₃₋₈-Cycloalk(en)yl-C₁₋₆-alk(en/in)yl, vorausgesetzt daß wenn eines von R^{x} und R^{y}NP^{z}R^{w}-C₁₋₆-Alk(en/in)yl ist, dann ist das andere ausgewählt aus Wasserstoff, C₁₋₆-Alk(en/in)yl, Cyano-C₁₋₆-alk(en/in)yl, C₃₋₈-Cycloalk(en/in)yl oder C₃₋₈-Cycloalk(en)yl-C₁₋₆-alk-(en/in)yl; oder R^{x} und RY bilden zusammen mit dem Stickstoffatom, an das sie gebunden sind einen 3- bis 7-gliedrigen Ring, der gegebenenfalls ein weiteres Heteroatom enthält; typisch ist R⁸ ausgewählt aus Wasserstoff, C₁₋₆-Alkyl, Halogen, Halogen-C₁₋₆-alkyl oder NR^{x}R^{y}, worin R^{x} Wasserstoff ist und R^{y} C₁₋₆-Alkyl ist; typisch ist R⁸ ausgewählt aus Wasserstoff, C₁₋₆-Alkyl, Halogen oder Halogen-C₁₋₆-alkyl.

11. Verbindung gemäß einem der Ansprüche 1 bis 10, worin R⁹ ausgewählt ist aus Wasserstoff, Halogen, C₁₋₆-Alk(en/in)yl, Halogen-C₁₋₆-alk(en/in)yl; typisch ist R⁹ ausgewählt aus Wasserstoff.

12. Verbindung gemäß einem der Ansprüche 1 bis 11, worin die Verbindung der Formel (I) 1 bis 4 Substituenten in dem/den Phenyl-Ring(en) aufweist, ausgewählt aus irgendeinem von R¹-R⁹, die kein Wasserstoff sind, und die verbleibenden Substituenten Wasserstoff sind.

13. Verbindung gemäß Anspruch 1, worin die Verbindung ausgewählt ist aus:
4-[2-(4-Fluorphenylsulfanyl)-phenyl]-1,2,3,6-tetrahydropyridin,
4-[2-(4-Chlorphenylsulfanyl)-phenyl]-1,2,3,6-tetrahydropyridin,
4-[2-(4-Methoxyphenylsulfanyl)-5-methyl-phenyl]-1,2,3,6-tetrahydropyridin,
4-(5-Methyl-2-p-tolylsulfanylphenyl)-1,2,3,6-tetrahydropyridin,
4-[2-(4-Fluoryphenylsulfanyl)-5-methyl-phenyl]-1,2,3,6-tetrahydropyridin,
4-[2-(4-Chlorphenylsulfanyl)-5-methyl-phenyl]-1,2,3,6-tetrahydropyridin,
4-[2-(2,4-Dimethylphenylsulfanyl)-phenyl]-1,2,3,6-tetrahydropyridin,
4-[2-(4-Chlorphenylsulfanyl)-5-trifluormethyl-phenyl]-1,2,3,6-tetrahydropyridin,
4-[2-(4-Fluor-2-methylphenylsulfanyl)-phenyl]-1,2,3,6-tetrahydropyridin,
4-[2-(4-Chlorphenylsulfanyl)-4-fluor-phenyl]-1,2,3,6-tetrahydropyridin,
4-[4-Fluor-2-(p-tolylsulfanyl)-phenyl]-1,2,3,6-tetrahydropyridin,
4-[4-Fluor-2-(4-methoxyphenylsulfanyl)-phenyl]-1,2,3,6-tetrahydropyridin,
4-[2-(2,4-Dimethylphenylsulfanyl)-5-methyl-phenyl]-1,2,3,6-tetrahydropyridin,
4-[2-(2,4-Dimethylphenylsulfanyl)-5-trifluormethylphenyl]-1,2,3,6-tetrahydropyridin,
4-(2-p-Tolylsulfanyl-5-trifluormethylphenyl)-1,2,3,6-tetrahydropyridin,
4-[2-(4-Fluor-2-methylphenylsulfanyl)-5-methyl-phenyl]-1,2,3,6-tetrahydropyridin,
4-[5-Brom-2-(2,4-dimethylphenylsulfanyl)-phenyl]-1,2,3,6-tetrahydropyridin,
4-[2-(4-Chlorphenylsulfanyl)-5-fluor-phenyl]-1,2,3,6-tetrahydropyridin,
4-[5-Fluor-2-(4-methoxyphenylsulfanyl)-phenyl]-1,2,3,6-tetrahydropyridin,
4-[2-(2,4-Dichlorphenylsulfanyl)-phenyl]-1,2,3,6-tetrahydropyridin,
4-[2-(4-Chlor-2-fluor-phenylsulfanyl)-phenyl}-1,2,3,6-tetrahydropyridin,
4-[2-(3-Methoxyphenylsulfanyl)-phenyl]-1,2,3,6-tetrahydropyridin,
4-[3-Fluor-2-(4-methoxyphenylsulfanyl)-phenyl]-1,2,3,5-tetrahydropyridin,
4-[2-(2-Chlorphenylsulfanyl)-phenyl]-1,2,3,6-tetrahydropyridin,
4-[2-(2-Chlor-4-methoxyphenylsulfanyl)-phenyl]-1,2,3,6-tetrahydropyridin,
4-[2-(2Fluorphenylsulfanyl)-phenyl]-1,2,3,6-tetrahydropyridin,
4-[2-(2-Bromphenylsulfanyl)-phenyl]-1,2,3,6-tetrahydropyridin,
4-[2-(4-Bromphenylsulfanyl)-phenyl]-1,2,3,6-tetrahydropyridin,
4-(2-o-Tolylsulfanylphenyl)-1,2,3,6-tetrahydropyridin,
4-[2-(4-Chlor-2-methylphenylsulfanyl)-phenyl]-1,2,3,6-tetrahydropyridin,
4-[2-(4-Trifluormethyl-phenylsulfanyl)-phenyl]-1,2,3,6-tetrahydropyridin,
4-[2-(2,3-Dichlor-phenylsulfanyl)-phenyl]-1,2,3,6-tetrahydropyridin,
4-[2-(2,3-Dimethyl-phenylsulfanyl)-phenyl]-1,2,3,6-tetrahydropyridin,
4-[2-(3,4-Dimethyl-phenylsulfanyl)-phenyl]-1,2,3,6-tetrahydropyridin,
4-[2-(2Methoxy-5-methyl-phenylsulfanyl)-phenyl]-1,2,3,6-tetrahydropyridin,
4-[2-(2-Chlor-4-fluor-phenylsulfanyl)-phenyl]-1,2,3,6-tetrahydropyridin,
4-[2-(4-Methoxy-2-methyl-phenylsulfanyl)-phenyl]-1,2,3,6-tetrahydropyridin,
4-[2-(2-Fluor-4-methoxy-phenylsulfanyl)-phenyl]-1,2,3,6-tetrahydropyridin,
4-[2-(2,4-Dimethyl-phenylsulfanyl)-5-fluor-phenyl]-1,2,3,6-tetrahydropyridin,
4-(5-Fluor-2-phenylsulfanyl-phenyl)-1,2,3,6-tetrahydropyridin,
4-[5-Fluor-2-(4-fluor-phenylsulfanyl)-phenyl]-1,2,3,6-tetrahydropyridin,
4-(2-m-Tolylsulfanyl-phenyl)-1,2,3,6-tetrahydropyridin,
4-[2-(4-Fluor-3-methoxy-phenylsulfanyl)-phenyl]-1,2,3,6-tetrahydropyridin,
4-[2-(2-Brom-4-fluor-phenylsulfanyl)-phenyl]-1,2,3,6-tetrahydropyridin,
4-[2-(2-Chlor-4-fluor-phenylsulfanyl)-5-fluor-phenyl]-1,2,3,6-tetrahydropyridin,
4-[2-(2,4-Dichlor-phenylsulfanyl)-5-fluor-phenyl]-1,2,3,6-tetrahydropyridin,
4-[2-(3-Chlor-phenylsulfanyl)-phenyl]-1,2,3,6-tetrahydropyridin,
4-[2-(2-Methoxy-phenylsulfanyl)-phenyl]-1,2,3,6-tetrahydropyridin,
4-[2-(2,4-Dichlor-phenylsulfanyl)-5-methyl-phenyl]-1,2,3,6-tetrahydropyridin,
4-[2-(4-Chlor-2-fluor-phenylsulfanyl)-5-methyl-phenyl]-1,2,3,6-tetrahydropyridin,
4-[2-(4-Chlor-2-methyl-phenylsulfanyl)-5-methylphenyl]-1,2,3,6-tetrahydropyridin,
4-[2-(2-Fluor-4-methyl-phenylsulfanyl)-5-methylphenyl]-1,2,3,6-tetrahydropyridin,
4-[2-(2,4-Difluor-phenylsulfanyl)-5-methyl-phenyl]-1,2,3,6-tetrahydropyridin,
4-[2-(2-Chlor-4-fluor-phenylsulfanyl)-5-methyl-phenyl]-1,2,3,6-tetrahydropyridin,
4-[2-(2-Brom-4-fluor-phenylsulfanyl)-5-methyl-phenyl]-1,2,3,6-tetrahydropyridin,
4-[2-(2-Brom-4-methyl-phenylsulfanyl)-5-methyl-phenyl]-1,2,3,6-tetrahydropyridin,
4-[2-(2-Brom-phenylsulfanyl)-5-methyl-phenyl]-1,2,3,6-tetrahydropyridin,
4-[2-(4-Brom-phenylsulfanyl)-5-methyl-phenyl]-1,2,3,6-tetrahydropyridin,
4-(5-Methyl-2-o-tolylsulfanyl-phenyl)-1,2,3,6-tetrahydropyridin,
4-[2-(4-Methoxy-2-methyl-phenylsulfanyl)-5-methylphenyl]-1,2,3,6-tetrahydropyridin,
4-[2-(2-Fluor-4-methoxy-phenylsulfanyl)-5-methylphenyl]-1,2,3,6-tetrahydropyridin,
4-[2-(3,4-Dichlor-phenylsulfanyl)-5-methyl-phenyl]-1,2,3,6-tetrahydropyridin,
4-[2-(2-Chlor-4-methyl-phenylsulfanyl)-5-methylphenyl]-1,2,3,6-tetrahydropyridin,
4-[2-(2-Fluor-4-methyl-phenylsulfanyl)-phenyl]-1,2,3,6-tetrahydropyridin,
4-[2-(3,4-Dichlor-phenylsulfanyl)-phenyl]-1,2,3,6-tetrahydropyridin,
4-[2-(4-Brom-2-fluor-phenylsulfanyl)-phenyl]-1,2,3,6-tetrahydropyridin,
4-[2-(4-Brom-2-methyl-phenylsulfanyl)-phenyl]-1,2,3,6-tetrahydropyridin,
4-[2-(2,4-Difluor-phenylsulfanyl)-phenyl]-1,2,3,6-tetrahydropyridin,
4-[2-(2-Brom-4-methyl-phenylsulfanyl)-phenyl]-1,2,3,6-tetrahydropyridin,
4-[2-(4-Chlor-2-methoxy-phenylsulfanyl)-phenyl]-1,2,3,6-tetrahydropyridin,
4-[5-Fluor-2-(2-fluor-4-methoxy-phenylsulfanyl)-phenyl]-1,2,3,5-tetrahydropyridin,
4-[2-(2-Chlor-4-methoxy-phenylsulfanyl)-5-fluorphenyl]-1,2,3,6-tetrahydropyridin,
4-[5-Fluor-2-(2fluor-4-methyl-phenylsulfanyl)-phenyl]-1,2,3,6-tetrahydropyridin,
4-[2-(2-Chlor-4-methyl-phenylsulfanyl)-5-fluor-phenyl]-1,2,3,6-tetrahydropyridin,
4-[2-(4-Chlor-2-fluor-phenylsulfanyl)-5-fluor-phenyl]-1,2,3,6-tetrahydropyridin,
4-[5-Fluor-2-(4-methoxy-2-methyl-phenylsulfanyl)-phenyl]-1,2,3,6-tetrahydropyridin,
4-[2-(2,3-Dimethyl-phenylsulfanyl)-5-methyl-phenyl]-1,2,3,6-tetrahydropyridin,
4-[5-Methyl-2-(3-methyl-phenylsulfanyl)-phenyl]-1,2,3,6-tetrahydropyridin,
4-[2-(2-Fluor-phenylsulfanyl)-5-methyl-phenyl]-1,2,3,6-tetrahydropyridin,
4-[2-(2-Chlor-phenylsulfanyl)-5-methyl-phenyl]-1,2,3,6-tetrahydropyridin,
4-[2-(3,4-Dimethyl-phenylsulfanyl)-5-methyl-phenyl]-1,2,3,6-tetrahydropyridin,
4-[2-(4-Chlor-2-methoxy-phenylsulfanyl)-5-methylphenyl]-1,2,3,6-tetrahydropyridin,
4-[2-(2,3-Difluor-phenylsulfanyl)-phenyl]-1,2,3,6-tetrahydropyridin,
4-[2-(2-Chlor-4-methyl-phenylsulfanyl)-phenyl]-1,2,3,6-tetrahydropyridin,
4-[2-(2,3-Difluor-phenylsulfanyl)-5-methyl-phenyl]-1,2,3,6-tetrahydropyridin,
4-[2-(3-Chlor-2-fluor-phenylsulfanyl)-5-methyl-phenyl]-1,2,3,6-tetrahydropyridin,
4-[2-(3-Chlor-2-methyl-phenylsulfanyl)-phenyl]-1,2,3,6-tetrahydropyridin,
4-[2-(3-Chlor-2-methyl-phenylsulfanyl)-phenyl]-1,2,3,6-tetrahydropyridin,
4-[2-(3-Fluor-2-methyl-phenylsulfanyl)-5-methylphenyl]-1,2,3,5-tetrahydropyridin,
4-[2-(3-Fluor-phenylsulfanyl)-5-methyl-phenyl]-1,2,3,6-tetrahydropyridin,
4-[2-(3-Chlor-phenylsulfanyl)-5-methyl-phenyl]-1,2,3,6-tetrahydropyridin,
4-[2-(3-Brom-phenylsulfanyl)-5-methyl-phenyl]-1,2,3,6-tetrahydropyridin,
4-[2-(3-Methoxy-phenylsulfanyl)-5-methyl-phenyl]-1,2,3,6-tetrahydropyridin,
4-[5-Methyl-2-(3-trifluormethyl-phenylsulfanyl)-phenyl]-1,2,3,6-tetrahydropyridin,
4-[2-(2-Methoxy-phenylsulfanyl)-5-methyl-phenyl]-1,2,3,6-tetrahydropyridin,
4-[2-(2-Ethyl-phenylsulfanyl)-phenyl]-1,2,3,6-tetrahydropyridin,
4-[2-(4-Ethyl-phenylsulfanyl)-phenyl]-1,2,3,6-tetrahydropyridin,
4-[2-(2-tert-Butyl-phenylsulfanyl)-phenyl]-1,2,3,6-tetrahydropyridin,
4-[2-(9-tert-Butyl-phenylsulfanyl)-phenyl]-1,2,3,6-tetrahydropyridin,
4-[2-(3-Fluor-phenylsulfanyl)-phenyl]-1,2,3,6-tetrahydropyridin,
4-[2-(2-Trifluormethyl-phenylsulfanyl)-phenyl]-1,2,3,6-tetrahydropyridin,
4-[2-(3-Trifluormethyl-phenylsulfanyl)-phenyl]-1,2,3,6-tetrahydropyridin,
4-[2-(4-Trifluormethoxy-phenylsulfanyl)-phenyl]-1,2,3,6-tetrahydropyridin,
4-[2-(4-Methylsulfanyl-phenylsulfanyl)-phenyl]-1,2,3,6-tetrahydropyridin,
4-[2-(3,5-Dimethyl-phenylsulfanyl)-phenyl]-1,2,3,6-tetrahydropyridin,
4-[2-(2,5-Dimethyl-phenylsulfanyl)-phenyl]-1,2,3,6-tetrahydropyridin,
4-[2-(2,5-Dichlor-phenylsulfanyl)-phenyl]-1,2,3,6-tetrahydropyridin,
4-[2-(3,5-Dichlor-phenylsulfanyl)-phenyl]-1,2,3,6-tetrahydropyridin,
4-[2-(3-Chlor-4-fluor-phenylsulfanyl)-phenyl]-1,2,3,6-tetrahydropyridin,
4-[2-(2,4,6-Trimethyl-phenylsulfanyl)-phenyl]-1,2,3,6-tetrahydropyridin,
4-[5-Methylamino-2-(4-methyl-phenylsulfanyl)-phenyl]-1,2,3,6-tetrahydropyridin,
4-[5-Fluor-2-(2-methoxy-phenylsulfanyl)-phenyl]-1,2,3,6-tetrahydropyridin,
4-(5-Fluor-2-o-tolylsulfanyl-phenyl)-1,2,3,6-tetrahydro-pyridin,
4-(5-Fluor-2-p-tolylsulfanyl-phenyl)-1,2,3,6-tetrahydro-pyridin,
4-[2-(Benzol[1,3]dioxol-5-ylsulfanyl)-5-methyl-phenyl]-1,2,3,6-tetrahydropyridin,
4-[2-(2-Chlor-4-methoxy-phenylsulfanyl)-5-methylphenyl]-1,2,3,6-tetrahydropyridin,
4-[2-(2,3-Dichlor-phenylsulfanyl)-5-methyl-phenyl]-1,2,3,6-tetrahydropyridin,
4-[2-(3-Chlor-2-methyl-phenylsulfanyl)-5-methyl-phenyl]-1,2,3,6-tetrahydropyridin,
4-[2-(Benzo[1,3]dioxol-5-ylsulfanyl)-phenyl]-1,2,3,6-tetrahydropyridin,
4-[5-Fluor-2-(3-fluor-phenylsulfanyl)-phenyl]-1,2,3,6-tetrahydropyridin,
4-(5-Fluor-2-m-tolylsulfanyl-phenyl)-1,2,3,6-tetrahydropyridin,
4-[5-Fluar-2-(3-methoxy-phenylsulfanyl)-phenyl]-1,2,3,6-tetrahydropyridin,
4-[2-(3-Chlor-phenylsulfanyl)-5-fluor-phenyl]-1,2,3,6-tetrahydropyridin,
4-[2-(2-Chlor-phenylsulfanyl)-5-fluor-phenyl]-1,2,3,6-tetrahydropyridin,
4-[4-Fluor-2-(4-methyl-phenylsulfanyl)-phenyl]-1,2,3,6-tetrahydro-pyridin,
4-[2-(4-Brom-2-fluor-phenylsulfanyl)-5-fluor-phenyl]-1,2,3,6-tetrahydropyridin,
4-[2-(4-Fluor-3-methoxy-phenylsulfanyl)-phenyl]-1,2,3,6-tetrahydropyridin,
4-[2-3-Fluor-4-methyl-phenylsulfanyl)-5-phenyl]-1,2,3,6-tetrahydropyridin,
4-[5-Fluor-2-(4-methoxy-phenylsulfanyl)-phenyl]-1,2,3,6-tetrahydropyridin,
oder ein pharmazeutisch akzeptables Salz davon.

14. Pharmazeutische Zusammensetzung umfassend eine Verbindung gemäß einem der Ansprüche 1 bis 13 oder ein pharmazeutisch annehmbares Säureadditionssalz davon und mindestens einen pharmazeutisch annehmbaren Träger oder Verdünner.

15. Verwendung einer Verbindung gemäß einem der Ansprüche 1 bis 13 oder ein pharmazeutisch annehmbares Säureadditionssalz davon zur Herstellung eines Arzneimittels zur Behandlung einer affektiven Psychose, wie Depression, Angststörung, einschließlich allgemeine Angststörung, soziale Angststörung, posttraumatische Belastungsstörung, obsessiver Zwangsstörung, Panikstörung, Panikattacken, spezifische Phobien, soziale Phobien und Platzangst.

16. Verbindung gemäß einem der Ansprüche 1 bis 13 zur Verwendung als Arzneimittel.

## Revendications

1. Composé représenté par la formule générale I : 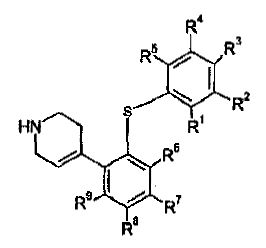 dans lequel R¹, R², R³, R⁴, R⁵ sont indépendamment choisis parmi hydrogène, halogène, cyano, C₁₋₆-alk(én/yn)yle, C₁₋₆-alk(én/yn)yloxy, C₁₋₆-alk(én/yn)ylsulfanyle, hydroxyle, hydroxy-C₁₋₆-alk(én/yn)yle, halo-C₁₋₆-alk(én/yn)yle, halo-C₁₋₆-alk(én/yn)yloxy, ou NR^{x}R^{y} dans lequel R^{x} et R^{y} sont indépendamment choisis parmi hydrogène, C₁₋₆-alk(én/yn)yle, C₃₋₈-cycloalk(én)yle, C₃₋₈-cycloalk(én)yl-C₁₋₆-alk(én/yn)yle, ou NR^{z}R^{w}-C₁₋₆-alk(én/yn)yle, dans lequel R^{z} et R^{w} sont indépendamment choisis parmi hydrogène, C₁₋₆-alk(én/yn)yle, C₃₋₈-cycloalk(én)yle, ou C₃₋₈-cycloalk(én)yl-C₁₋₆-alk(én/yn)yle; ou R^{x} et R^{y} conjointement avec l'azote auquel ils sont attachés forment un cycle de 3 à 7 éléments qui contient facultativement un hétéroatome supplémentaire ; ou
R² et R³ ensemble forment un hétérocycle condensé au cycle phényle choisi parmi 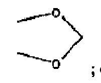 et R¹, R⁴, R⁵ sont tels que définis ci-dessus ;
R⁶, R⁷, R⁸, R⁹ sont indépendamment choisis parmi hydrogène, halogène, C₁₋₆-alk(én/yn)yle, C₁₋₆-alk(én/yn)yloxy, C₁₋₆-alk(én/yn)ylsulfanyle, hydroxyle, hydroxy-C₁₋₆-alk(én/yn)yle, halo-C₁₋₆-alk(én/yn)yle, halo-C₁₋₆-alk(én/yn)yloxy, ou NR^{x}R^{y} dans lequel R^{x} et R^{y} sont indépendamment choisis parmi hydrogène, C₁₋₆-alk(én/yn)yle. C₃₋₈-cycloalk(én)yle, C₃₋₈-cycloalk(én)yl-C₁₋₆-alk(én/yn)yle, ou NR^{z}R^{w}-C₁₋₆-alk(énlyn)yle, dans lequel R^{z} et R^{w} sont indépendamment choisis parmi hydrogène, C₁₋₆-alk(én/yn)yle, C₃₋₈-cycloalk(én)yle, ou C₃₋₈-cycloalk(én)yl-C₁₋₆-alk(én/yn)yle ; ou R^{x} et R^{y} conjointement avec l'azote auquel ils sont attachés forment un cycle de 3 à 7 éléments qui contient facultativement un hétéroatome supplémentaire ;
à condition qu'au moins un de R¹, R², R³, R⁴, R⁵, R⁶, R⁷, R⁸, et R⁹ soit différent de l'hydrogène; aussi à condition que quand R³ est méthyle ou méthoxy, alors au moins un de R¹, R², R⁴, R⁵, R⁶, R⁷, R⁸, et R⁹ soit différent de l'hydrogène ;
ou un sel de celui-ci.

2. Composé selon la revendication 1, dans lequel R¹ est choisi parmi hydrogène, halogène, cyano, C₁₋₆-alk(én/yn)yle, C₁₋₆-alk(én/yn)yloxy, C₁₋₆-alk(én/yn)ylsulfanyle, halo-C₁₋₆-alk(én/yn)yle, ou NR^{x}R^{y} dans lequel R^{x} et R^{y} sont indépendamment choisis parmi hydrogène, C₁₋₆-alk(én/yn)yle, cyano-C₁₋₆-alk(én/yn)yle, C₃₋₈-cycloalk(én)yle, C₃₋₈-cycloalk(én)yl-C₁₋₆-alk(én/yn)yle, ou NR^{z}R^{w}-C₁₋₆-alk(én/yn)yle, dans lequel R^{z} et R^{w} sont indépendamment choisis parmi hydrogène, C₁₋₆-alk(én/yn)yle, C₃₋₈-cycloalk(én)yle, ou C₃₋₈-cycloalk(én)yl-C₁₋₆-alk(én/yn)yle, à condition que si un de R^{x} et R^{y} est NR^{z}R^{w}-C₁₋₆-alk(én/yn)yle alors l'autre est choisi parmi hydrogène, C₁₋₆-alk(énlyn)yle, cyano-C₁₋₆-alk(én/yn)yle, C₃₋₈-cycloalk(én)yle, ou C₃₋₈-cycloalk(én)yl-C₁₋₆-alk(én/yn)yle; ou R^{x} et R^{y} conjointement avec l'azote auquel ils sont attachés forment un cycle de 3 à 7 éléments qui contient facultativement un hétéroatome supplémentaire. ; typiquement, R¹ est choisi parmi hydrogène, C₁₋₆-alkyle, halogène, C₁₋₆-alkyloxy, ou halo-C₁₋₆-alkyle ; plus typiquement, R¹ est choisi parmi hydrogène, C₁₋₆-alkyle, ou halogène.

3. Composé selon l'une quelconque des revendications 1 ou 2, dans lequel R² est choisi parmi hydrogène, halogène, cyano, C₁₋₆-alk(en/yn)yle, C₁₋₆-alk(én/yn)yloxy, C₁₋₆-alk(én/yn)ylsulfanyle, halo-C₁₋₆-alk(én/yn)yle; typiquement, R² est choisi parmi hydrogène, C₁₋₆-alcoxy, halogène, C₁₋₆-alkyle, ou halo-C₁₋₆-alkyle; plus typiquement, R² est choisi parmi hydrogène, ou C₁₋₆-alcoxy.

4. Composé selon l'une quelconque des revendications 1 à 3, dans lequel R³ est choisi parmi hydrogène, halogène, cyano, C₁₋₆-alk(én/yn)yle, C₁₋₆-alk(én/yn)yloxy, C₁₋₆alk(énlyn)ylsulfanyle, halo-C₁₋₆-alk(én/yn)yle; typiquement, R³ est choisi parmi hydrogène, C₁₋₆-alkyle, C₁₋₆-alcoxy, halogène, C₁₋₆-alkylsulfanyle, halo-C₁₋₆-alkyle, ou halo-C₁₋₆-alkyloxy; plus typiquement, R³ est choisi parmi hydrogène, C₁₋₆-alkyle, C₁₋₆-alcoxy, ou halogène.

5. Composé de l'une quelconque des revendications 1 à 2, dans lequel R² et R³ ensemble forment un hétérocycle condensé avec le cycle phényle choisi parmi

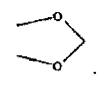

6. Composé selon l'une quelconque des revendications 1 à 5, dans lequel R⁴ est choisi parmi hydrogène, halogène, cyano, C₁₋₆-alk(én/yn)yle, C₁₋₆-alk(én/yn)yloxy, C₁₋₆-alk(én/yn)ylsulfanyle, halo-C₁₋₆-alk(én/yn)yle ; typiquement, R⁴ est choisi parmi hydrogène, C₁₋₆-alcoxy, halogène, C₁₋₆-alkyle, ou halo-C₁₋₆-alkyle ; plus typiquement, R⁴ est choisi parmi hydrogène, ou C₁₋₆-alcoxy.

7. Composé selon l'une quelconque des revendications 1 à 6, dans lequel R⁵ est choisi parmi hydrogène, halogène, cyano, C₁₋₆-alk(én/yn)yle, C₁₋₆-alk(én/yn)yloxy, C₁₋₆-alk(én/yn)ylsulfanyle, halo-C₁₋₆-alk(én/yn)yle, ou NR^{x}R^{y} dans lequel R^{x} et R^{y} sont indépendamment choisis parmi hydrogène, C₁₋₆-alk(én/yn)yle, cyano-C₁₋₆-alk(én/yn)yle, C₃₋₈-cycloalk(én)yle, C₃₋₈-cycloalk(én)yl-C₁₋₆-alk(én/yn)yle, ou NR^{z}R^{w}-C₁₋₆-alk(én/yn)yle, dans lequel R^{z} et R^{w} sont indépendamment choisis parmi hydrogène, C₁₋₆-alk(én/yn)yle, C₃₋₈-cycloalk(én)yle, ou C₃₋₈-cycloalk(én)yl-C₁₋₆-alk(én/yn)yle, à condition que si un de R^{x} et R^{y} est NR^{z}R^{w}-C₁₋₆-alk(én/yn)yle alors l'autre est choisi parmi hydrogène, C₁₋₆-alk(én/yn)yle, cyano-C₁₋₆-alk(én/yn)yle, C₃₋₈-cycloalk(én)yle, ou C₃₋₈-cycloalk(én)yl-C₁₋₆alk(én/yn)yle; ou R^{x} et R^{y} conjointement avec l'azote auquel ils sont attachés forment un cycle de 3 à 7 éléments qui contient facultativement un hétéroatome supplémentaire ; typiquement, R⁵ est choisi parmi hydrogène, C₁₋₆-alkyle, halogène, C₁₋₆-alkyloxy, ou halo-C₁₋₆-alkyle ; plus typiquement, R⁵ est choisi parmi hydrogène, C₁₋₆-alkyle, ou halogène.

8. Composé selon l'une quelconque des revendications 1 à 7, dans lequel R⁶ est choisi parmi hydrogène, halogène, C₁₋₆-alk(én/yn)yle, halo-C₁₋₆-alk(én/yn)yle; typiquement, R⁶ est choisi parmi hydrogène, ou halogène.

9. Composé selon l'une quelconque des revendications 1 à 8, dans lequel R⁷ est choisi parmi hydrogène, halogène, C₁₋₆-alk(én/yn)yle, halo-C₁₋₆-alk(én/yn)yle; typiquement, R⁷ est choisi parmi hydrogène, ou halogène.

10. Composé selon l'une quelconque des revendications 1 à 9, dans lequel R⁸ est choisi parmi hydrogène, halogène, C₁₋₆-alk(én/yn)yle, halo-C₁₋₆-alk(én/yn)yle, ou NR^{x}R^{y} dans lequel R^{x} et R^{y} sont indépendamment choisis parmi hydrogène, C₁₋₆-alk(én/yn)yle, cyano-C₁₋₆-alk(én/yn)yle, C₃₋₈-cycloalk(én)yle, C₃₋₈-cycloalk(én)yl-C₁₋₆-alk(én/yn)yle, ou NR^{z}R^{w}-C₁₋₆-alk(én/yn)yle, dans lequel R^{z} et R^{w} sont indépendamment choisis parmi hydrogène, C₁₋₆-alk(én/yn)yle, C₃₋₈-cycloalk(én)yle, ou C₃₋₈-cycloalk(én)yl-C₁₋₆-alk(én/yn)yle, à condition que si un de R^{x} et R^{y} est NR^{z}R^{w}-C1-6-alk(én/yn)yle alors l'autre est choisi parmi hydrogène, C₁₋₆-alk(én/yn)yle, cyano-C₁₋₆-alk(én/yn)yle, C₃₋₈-cycloalk(én)yle, ou C₃₋₈-cycloalk(én)yl-C₁₋₆-alk(én/yn)yle ; ou R^{x} et R^{y} conjointement avec l'azote auquel ils sont attachés forment un cycle de 3 à 7 éléments qui contient facultativement un hétéroatome supplémentaire ; typiquement, R⁸ est choisi parmi hydrogène, C₁₋₆-alkyle, halogène, halo-C₁₋₆-alkyle, ou NR^{x}R^{y} dans lequel R^{x} est hydrogène et R^{y} est C₁₋₆-alkyle ; plus typiquement, R⁸ est choisi parmi hydrogène, C₁₋₆-alkyle, halogène, ou halo-C₁₋₆-alkyle.

11. Composé selon l'une quelconque des revendications 1 à 10, dans lequel R⁹ est choisi parmi hydrogène, halogène, C₁₋₆-alk(én/yn)yle, halo-C₁₋₆-alk(én/yn)yle ; typiquement, R⁹ est choisi parmi hydrogène.

12. Composé selon l'une quelconque des revendications 1 à 11, dans lequel le composé de formule I a 1 à 4 substituants dans le ou les cycles phényles, choisi dans le groupe comprenant l'un quelconque de R¹-R⁹, qui sont différents de l'hydrogène, et les substituants restants sont hydrogène.

13. Composé selon la revendication 1, ledit composé étant choisi dans le groupe comprenant
4-[2-(4-fluorophénylsulfanyl)-phényl]-1,2,3,6-tétrahydropyridine,
4-[2-(4-chlorophénylsulfanyl)-phényl]-1,2,3,6-tétrahydropyridine,
4-[2-(4-méthoxyphénylsulfanyl)-5-méthyl-phényl]-1,2,3,6-tétrahydropyridine,
4-(5-méthyl-2-p-tolylsulfanylphényl)-1,2,3,6-tétrahydropyridine,
4-[2-(4-fluorophénylsulfanyl)-5-méthyl-phényl]-1,2,3,6-tétrahydropyridine,
4-[2-(4-chlorophénylsulfanyl)-5-méthyl-phényl]-1,2,3,6-tétrahydropyridine,
4-[2-(2,4-diméthylphénylsulfanyl)-phényl]-1,2,3,6-tétrahydropyridine,
4-[2-(4-chlorophénylsulfanyl)-5-trifluorométhyl-phényl]-1,2,3,6-tétrahydropyridine,
4-[2-(4-fluoro-2-méthylphénylsulfanyl)-phényl]-1,2,3,6-tétrahydropyridine,
4-[2-(4-chlorophénylsulfanyl)-4-fluoro-phényl]-1,2,3,6-tétrahydropyridine,
4-[4-tluoro-2-(p-tolylsulfanyl)-phényl]-1,2,3,6-tétrahydropyridine,
4-[4-fluoro-2-(4-méthoxyphénylsulfanyl)-phényl]-1,2,3,6-tétrahydropyridine,
4-[2-(2,4-diméthylphénylsulfanyl)-5-méthyl-phényl]-1,2,3,6-tétrahydropyridine,
4-[2-(2,4-diméthylphénylsulfanyl)-5-trifluorométhyl-phényl]-1,2,3,6-tétrahydropyridine,
4-(2-p-tolylsulfanyl-5-trifluotométhylphényl)-1,2,3,6-tétrahydropyridine,
4-[2-(4-fluoro-2-méthylphénylsulfanyl)-5-méthyl-phényl]-1,2,3,6-tétrahydropyridine,
4-[5-bromo-2-(2,4-diméthylphénylsulfanyl)-phényl]-1,2,3,6-tétrahydropyridine,
4-[2-(4-chlorophénylsulfanyl),5-fluoro-phényl]-1,2,3,6-tétrahyropyridine,
4-[5-fluoro-2-(4-méthoxyphénylsulfanyl)-phényl]-1,2,3,6-tétrahydropyridine,
4-[2-(2,4-dichlorophénylsulfanyl)-phényl]-1,2,3,6-tétrahydropyridine,
4-[2-(4-chloro-2-fluoro-phénylsulfanyl)-phényl]-1,2,3,6-tétrahydropyridine,
4-[2-(3-méthoxyphénylsulfanyl)-phényl]-1,2,3,6-tétrahydropyridine,
4-[3-fluoro-2-(4-méthoxyphénylsulfanyl)-phényl]-1,2,3,6-tétrahydropyridine,
4-[2-(2-chlorophénylsulfanyl)-phényl]-1,2,3,6-tétrahydropyridine,
4-[2-(2-chloro-4-méthoxyphénylsulfanyl)-phényl]-1,2,3,6-tétrahydropyridine,
4-[2-(2-fluorophénylsulfanyl)-phényl]-1,2,3,6-tétrahydropyridine,
4-[2-(2-bromophénylsulfanyl)-phényl]-1,2,3,6-tétrahydropyridine,
4-[2-(4-bromophénylsulfanyl)-phényl]-1,2,3,6-tétrahydropyridine,
4-(2-o-tolylsulfanylphényl)-1,2,3,6-tétrahydropyridine,
4-[2-(4-chloro-2-méthylphénylsulfanyl)-phényl]-1,2,3,6-tétrahydropyridine,
4-[2-(4-trifluorométhyl-phénylsulfanyl)-phényl]-1,2,3,6-tétrahydro-pyridine,
4-[2-(2,3-dichloro-phénylsulfanyl)-phényl]-1,2,3,6-tétrahydro-pyridine,
4-[2-(2,3-diméthyl-phénylsulfanyl)-phényl]-1,2,3,6-tétahydro-pyridine,
4-[2-(3,4-diméthyl-phénylsulfanyl)-phényl]-1,2,3,6-tétrahydro-pyridine,
4-[2-(2-méthoxy-5-méthyl-phénylsulfanyl)-phényl]-1,2,3,6-tétrahydro-pyridine,
4-[2-(2-chloro-4-fluoro-phénylsulfanyl)-phényl]-1,2,3,6-tétrahydro-pyridine,
4-[2-(4,méthoxy-2-méthyl-phénylsulfanyl)-phényl]-1,2,3,6-tétrahydro, pyridine,
4-[2-(2-fluoro-4-méthoxy-phénylsulfanyl)-phényl]-1,2,3,6-tétrahydro-pyridine,
4-[2-(2,4-diméthyl-phénylsulfanyl)-5-fluoro-phényl]-1,2,3,6-tétrahydro-pyridine,
4-(5- fluoro-2-phénylsulfanyl-phényl)-1,2,3,6-tétrahydro-pyridine,
4-[5-fluoro-2-(4-fluoro-phénylsulfanyl)-phényl]-1,2,3,6-tétrahydro-pyridine,
4-(2-m-tolylsulfanyl-phényl)-1,2,3,6-tétrahydro-pyridine,
4-[2-(4-fluoro-3-méthoxy-phénylsulfanyl)-phényl]-1,2,3,6-tétrahydro-pyridine,
4-[2-(2-bromo-4-fluoro-phénylsulfanyl)-phényl]-1,2,3,6-tétrahydro-pyridine,
4-[2-(2-chloro-4-fluoro-phénylsulfanyl)-5-fluoro-phényl]-1,2,3,6-tétrahydro-pyridine,
4-[2-(2,4-dichloro-phénylsulfanyl)-5-fluoro-phényl]-1,2,3,6-tébahydro-pyridine,
4-[2-(3-ehloro-phénylsulfanyl)-phényl]-1,2,3,6-tétrahydro-pyridine,
4-[2-(2-méthoxy-phényisulfanyl)-phényl]-1,2,3,6-tétrahydre-pyridine,
4-[2-(2,4-dichloro-phénylsulfanyl)-5-méthyl-phényl]-1,2,3,6-tétrahydro-pyridine,
4-[2-(4-chloro-2-fluoro-phénylsulfanyl)-5-méthyl-phényl]-1,2,3,6-tétrahydro-pyridine,
4-[2-(4-chloro-2-méthyl-phénylsulfanyl)-5-méthyl-phényl]-1,2,3,6-tétrahydro-pyridine,
4-[2-(2-fluoro-4-méthyl-phénylsulfanyl)-5-méthyl-phényl]-1,2,3,6-tétrahydro-pyridine,
4-[2-(2,4-difluoro-phénylsulfanyl)-5-méthyl-phényl]- 1,2,3,6-tétrahydro-pyridine,
4-[2-(2-chlore-4-fluoro-phénylsulfanyl)-5-méthyl-phényl]-1,2,3,6-tétrahydro-pyridine,
4-[2-(2-bromo-4-fluoro-phénylsulfanyl)-5-méthyl-phényl]-1,2,3,6-tétrahydro-pyridine,
4-[2-(2-bromo-4-méthyl-phénylsulfanyl)-5-méthyl-phényl]-1,2,3,6-tétrahydro-pyridine,
4-[2-(2-bromo-phénylsulfanyl)-5-méthyl-phényl]-1,2,3,6-tétrahydro-pyridine,
4-[2-(4-bromo-phénylsulfanyl)-5-méthyl-phényl]-1,2,3,6-tétrahydro-pyridine,
4-(5-méthyl-2-o-tolylsulfanyl-phényl)-1,2,3,6-tétrahydro-pyridine,
4-[2-(4-méthoxy-2-méthyl-phénylsulfanyl)-5-méthyl-phényl]-1,2,3,6-tétrahydro-pyridine,
4-[2-(2-fluoro-4-méthoxy-phénylsulfanyl)-5-méthyl-phényl]-1,2,3,6-tétrahydro-pyridine,
4-[2-(3,4-dichloro-phénylsulfanyl)-5-méthyl-phényl]-1,2,3,6-tétrahydro-pyridine,
4-[2-(2-chloro-4-méthyl-phénylsulfanyl)-5-méthyl-phényl]-1,2,3,6-tétrahydro-pyridine,
4-[2-(2-fluoro-4-méthyl-phénylsulfanyl)-phényl]-1,2,3,6-tétrahydro-pyridine,
4-[2-(3,4-dichloro-phénylsulfanyl)-phényl]-1,2,3,6-tétrahydropyridine,
4-[2-(4-bromo-2-fluoro-phénylsulfanyl)-phényl]-1,2,3,6-tétrahydro-pyridine,
4-[2-(4-bromo-2-méthyl-phénylsulfanyl)-phényl]-1,2,3,6-tétrahydro-pyridine,
4-[2-(2,4-difluoro-phénylsulfanyl)-phényl]-1,2,3,6-tétrahydropyrldine,
4-[2-(2-bromo-4-méthyl,phénylsulfanyl)-phényl]-1,2,3,6-tétrahydro-pyridine,
4-[2-(4-chloro-2-méthoxy-phénylsulfanyl)-phényl]-1,2,3,6-tétrahydro-pyridine,
4-[5-fluoro-2-(2-fluoro-4-méthoxy-phénylsulfanyl)-phényl]-1,2,3,6-tétrahydro-pyridine,
4-[2-(2-chloro-4-méthoxy-phénylsulfanyl)-5-fluoro-phényl]-1,2,3,6-tétrahydro-pyridine,
4-[5-fluoro-2-(2-fluoro-4-méthyl-phénylsulfanyl)-phényl]-1,2,3,6-tétrahydro-pyridine,
4-[2-(2-chloro-4-méthyl-phénylsulfanyl)-5-fluoro-phényl]-1,2,3,6-tétrahydro-pyridine,
4-[2-(4-chloro-2-fluoro-phénylsulfanyl)-5-fluoro-phényl]-1,2,3,6-tétrahydro-pyridine,
4-[5-fluoro-2-(4-méthoxy-2-méthyl-phénylsulfanyl)-phényl]-1,2,3,6-tétrahydro-pyridine,
4-[2-(2,3-diméthyl-phénylsulfanyl)-5-méthyl-phényl]-1,2,3,6-tétrahydro-pyridine,
4-[5-méthyl-2-(3,méthyl-phénylsulfanyl)-phényl]-1,2,3,6.tétrahydro-pyridine,
4-[2-(2-fluoro-phénylsulfanyl)-5-méthyl-phényl]-1,2,3,6-tétrahydro-pyridine,
4-[2-(2-chloro-phénylsulfattyl)-5-méthyl-phényl]-1,2,3,6-tétrahydro-pyridine,
4-[2-(3,4-diméthyl-phénylsulfanyl)-5-méthyl-phényl]-1,2,3,6-tétrahydro-pyridine,
4-[2-(4-chloro-2-méthoxy-phénylsulfanyl)-5-méthyl-phényl]-1,2,3,6-tétrahydro-pyridine,
4-[2-(2,3-fluoro-phénylsulfanyl)-phényl]-1,2,3,6-tétrahydropyridine,
4-[2-(2-chloro-4-méthyl-ghénylsulfanyl)-phényl]-1,2,3,6-tétrahydro-pyridine,
4-[2-(2,3-difluoro-phénylsulfanyl)-5-méthyl-phényl]-1,2,3,6-tétrahydro-pyridine,
4-[2-(3-chloro-2-fluoro-phénylsulfanyl)-S-méthyl-phényl]-1,2,3,6-tétrahydro-pyridine,
4-[2-(3-fluoro-2-méthyl-phénylsulfattyl)-phényl]-1,2,3,b-tétrahydro-pyridine,
4-[2-(3-chloro-2-méthyl-phénylsulfanyl)-phényl]-1,2,3,6-tétrahydro-pyridine,
4-[2-(3-fluoro-2-méthyl-phénylsulfanyl)-5-méthyl-phényl]-1,2,3,6-tétrahydro-pyridine,
4-[2-(3-fluoro-phénylsulfanyl)-5-méthyl-phényl]-1,2,3,6-tétrahydro-pyridine,
4-[2-(3-chloro-phénylsulfanyl)-5-méthyl-phényl]-1,2,3,6-tétrahydro-pyridine,
4-[2-(3-bromo-phénylsulfanyl)-5-méthyl-phényl]-1,2,3,6-tétrahydro,pyridine,
4-[2-(3-méthoxy-phénylsulfanyl)-5-méthyl-phényl]-1,2,3,6-tétrahydro-pyridine,
4-[5-méthyl-2-(3-trifluorométhyl-phénylsulfanyl)-phényl]-1,2,3,6-tétrahydro-pyridine,
4-[2-(2-méthoxy-phénylsulfanyl)-5-méthyl-phényl]-1,2,3,6-tétrahydro-pyridine,
4-[2-(2-éthyl-phénylsulfanyl)-phényl]-1,2,3,6-tétrahydro-pyridine,
4-[2-(4-éthyl-phénylsulfanyl)-phényl]-1,2,3,6-tétrahydro-pyridine,
4-[2-(2-tert-butyl-phénylsulfanyl)-phényl]-1,2,3,6-tétrahydro-pyridine,
4-[2-(4-tert-butyl-phénylsulfanyl)-phényl]-1,2,3,6-tétrahydro-pyridine,
4-[2-(3-fluoro-phénylsulfanyl)-phényl]-1,2,3,6-tétrahydro-pyridine,
4-[2-(2-trifluorométhyl-phénylsulfanyl)-phényl]-1,2,3,6-tétrahydro-pyridine,
4-[2-(3-trifluvrométhyl-phénylsulfanyl)-phényl]-1,2,3,6-tétrahydro-pyridine,
4-[2-(4-trifluorométhoxy-phénylsufanyl)-phényl]-1,2,3,6-tétrahydro-pyridine,
4-[2-(4-méthylsulfanyl-phénylsulfanyl)-phényl]-1,2,3,6-tétrahydro-pyridine,
4-[2-(3,5-diméthyl-phénylsulfanyl)-phényl]-1,2,3,6-tétrahydro-pyridine,
4-[2-(2,5-diméthyl-phénylsulfanyl)-phényl]-1,2,3,6-tétrahydro-pyridine,
4-[2-(2,5-diclùoro-phénylsulfanyl)-phényl]-1,2,3,6-tétrahydro-pyridine,
4-[2-(3,5-dichlaro-phênylsulfanyl)-phényl]-1,2,3,6-tétrahydro-pyridine,
4-[2-(3-chloro-4-fluoro-phénylsulfanyl)-phényl]-1,2,3,6-tétrahydro-pyridine,
4-[2-(2,4,6-triméthyl-phénylsulfanyl)-phényl]-1,2,3,6-tétrahydro-pyridine,
4-[5-méthylamino-2-(4-méthyl-phénylsulfanyl)-phényl]-1,2,3,6-tétrahydro-pyridine,
4-[5-fluoro-2-(2-methoxy-phénylsulfanyl)-phényl]-1,2,3,6-tétrahydro-pyridine,
4-(5-fluoro-2-o-tolylsulfanyl-phényl)-1,2,3,6-tétrahydro-pyridine,
4-(5-fluoro-2-p-tolylsulfanyl-phényl)-1,2,3,6-tétrahydro-pyridine,
4-[2-(benzo[1,3]dioxol-5-ylsulfanyl)-5-méthyl-phényl]-1,2,3,6-tétrahydro-pyridine,
4-[2-(2-chloro-4-méthoxy-phénylsulfanyl)-5-méthyl-phényl]-1,2,3,6, tétrahydro-pyridine,
4-[2-(2,3-dichloro-phénylsulfanyl)-5-méthyl-phényl]-1,2,3,6-tétrahydro-pyridine,
4-[2-(3-chloro-2-méthyl-phénylsulfanyl)-5-méthyl-phényl]-1,2,3,6-tétrahydro-pyridine,
4-[2-(benzo[1,3]dioxol-5-ylsulfanyl)-phényl]-1,2,3,6-tétrahydro-pyridine,
4-[5-2luoro-2-(3-fluoro-phénylsulfanyl)-phényl]-1,2,3,6-tétrahydro-pyridine,
4-(5-fluoro-2-m-tolylsulfanyl-phényl)-1,2,3,6-tétrahydro-pyridine,
4-[5-fluoro-2-(3-méthoxy-phénylsulfanyl)-phényl]-1,2,3,6-tétrahydro-pyridine,
4-[2-(3-chloro-phénylsulfanyl)-5-fluoro-phényl]-1,2,3,6-tétrahydro-pyridine,
4-[2-(2-chloro-phénylsulfanyl)-5-fluoro-phényl]-1,2,3,6-tétrahydro-pyridine,
4-[4-fluoro-2-(4-méthyl-phénylsulfanyl)-phényl]-1,2,3,6-tétrahydro-pyridine,
4-[2-(4-bromo-2-fluoro-phénylsulfanyl)-5,fluoro-phényl]-1,2,3,6-tetrahydro-pyridine,
4-[2-(4-fluoro-3-méthoxy-phénylsulfanyl)-5-méthyl-phényl]-1,2,3,6-tétrahydro-pyridine,
4-[2-(3-fluoro-4-méthyl-phénylsulfanyl)-5-méthyl-phényl]-1,2,3,6-tétrahydro-pyridine,
4-[5-fluoro-2-(4-méthoxy-phénylsulfanyl)-phényl]-1,2,3,6-tétrahydro-pyridine,
ou un sel pharmaceutiquement acceptable de celui-ci,

14. Composition pharmaceutique comprenant un composé selon l'une quelconque des revendications 1 à 13 ou un sel d'addition d'acide pharmaceutiquement acceptable de celui-ci et au moins un support ou diluant pharmaceutiquement acceptable.

15. Utilisation d'un composé selon l'une quelconque des revendications 1 à 13 ou un sel d'addition d'acide pharmaceutiquement acceptable de celui-ci pour la préparation d'un médicament pour le traitement d'un trouble affectif, tel que la dépression, de troubles d'anxiété incluant le trouble d'anxiété général, trouble d'anxiété social, trouble de stress post-traumatique, trouble obsessif compulsif, trouble de panique, attaques de panique, phobies spécifiques, phobie sociale et agoraphobie.

16. Composé selon l'une quelconque des revendications 1 à 13 destiné à être utilisé en tant que médicament.
